# EUROPEAN PATENT APPLICATION

(11) **EP 4 744 659 A1**
(43) Date of publication of application: **20.05.2026**
(21) Application number: 24838842.3
(22) Date of filing: 10.07.2024
(51) Int. Cl.: A61K 31/433, A61K 47/68, C07K 16/18, C07D 417/14, A61P 35/00, A61P 17/04

(54) **ANTIBODY DERIVATIVE AND USE THEREOF**

(30) Priority: 11.07.2023 CN 202310846345
(71) Applicant: Tsinghua University, Haidian District, Beijing 100084 (CN)
(72) Inventor: DU, Juanjuan, Beijing 100084 (CN); WU, Mengling, Beijing 100084 (CN); DING, Yanchao, Beijing 100084 (CN); ZHU, Rui, Beijing 100084 (CN)
(74) Representative: Potter Clarkson
(86) International application number: PCT/CN2024/104793
(87) International publication number: WO 2025/011590

(57) **Abstract**

Provided are an antibody derivative and a use thereof. The antibody derivative is a ligand-anchored antibody smacAb. Also provided are a method for constructing a smacAb library and a method for screening smacAb. The prepared smacAb has higher affinity, inhibitory activity and specificity, and the constructed smacAb library has diversity and a capacity of about 10⁹.

## Description

### FIELD

The present disclosure relates to the technical field of biopharmaceuticals, specifically to an antibody derivative and uses thereof in the construction of a smacAb library and in the screening of an antibody or smacAb.

### BACKGROUND

Phage display technology is a biological technique in which a DNA sequence encoding an exogenous protein or polypeptide is inserted into an appropriate position of a structural gene of a phage coat protein, such that the exogenous gene is expressed together with the coat protein, and the exogenous protein is displayed on the phage surface during phage reassembly.

By means of phage display technology, fully human antibodies for the treatment of various diseases can be screened. For example, Chinese Patent Application CN112851814A discloses the screening of BCMA-specific antibodies with strong binding ability and high affinity through multiple rounds of panning and ELISA.

However, for antibodies with relatively weak binding ability, it is difficult to screen such antibodies using phage display technology. Therefore, in the present application, a small molecule compound is covalently conjugated to a specific site of an antibody to further enhance the binding ability of the antibody to a target antigen, thereby enabling the screening of functional antibodies.

### SUMMARY

In a first aspect, the present disclosure provides an antibody derivative. The antibody derivative is a ligand-anchored antibody smacAb, and includes an antibody or antigen-binding fragment thereof and a ligand modified on the antibody or antigen-binding fragment thereof. The ligand is a small molecule compound, and the antibody or antigen-binding fragment thereof is conjugated to the small molecule compound to form a covalent conjugate. The small molecule compound and the antibody or antigen-binding fragment thereof jointly participate in binding to a target antigen.

The small molecule compound is capable of binding to a target antigen.

The antibody or antigen-binding fragment thereof and the small molecule compound may bind to the same target antigen or to the same class of target antigens.

The antibody or antigen-binding fragment thereof may be conjugated to one or more (e.g., 2, 3, 4, 5, 6, 7, 8, 9, 10, or more) small molecule compounds. When two small molecule compounds are conjugated, they may be identical or different. When three or more small molecule compounds are conjugated, they may be all identical, all different, or partially identical.

The small molecule compound is conjugated to a complementarity-determining region (CDR) of the antibody or antigen-binding fragment thereof. Preferably, the small molecule compound is conjugated to a heavy chain CDR or a light chain CDR of the antibody or antigen-binding fragment thereof, such as at least one of heavy chain CDR1, heavy chain CDR2, heavy chain CDR3, light chain CDR1, light chain CDR2, or light chain CDR3.

Preferably, the small molecule compound is conjugated to heavy chain CDR3 or light chain CDR3 of the antibody or antigen-binding fragment thereof. More preferably, the small molecule compound is conjugated to light chain CDR3 of the antibody or antigen-binding fragment thereof.

The CDR may have a length of 3 to 20 amino acids, e.g., 3, 4, 5, 6, 7, 8, 9, 10, 11, 12, 13, 14, 15, 16, 17, 18, 19, or 20 amino acids.

The conjugating may be at amino acid position 1, 2, 3, 4, 5, 6, 7, 8, 9, 10, 11, 12, 13, 14, 15, 16, 17, 18, 19, or 20 of the CDR.

In a specific embodiment of the present disclosure, the small molecule compound is conjugated to light chain CDR3 of the antibody or antigen-binding fragment thereof.

The light chain CDR3 has a length of 3 to 20 amino acids, e.g., 3, 4, 5, 6, 7, 8, 9, 10, 11, 12, 13, 14, 15, 16, 17, 18, 19, or 20 amino acids.

The small molecule compound is conjugated to one amino acid residue in the light chain CDR3 of the antibody or antigen-binding fragment thereof, preferably at position 1, 2, 3, 4, 5, 6, 7, 8, 9, 10, 11, 12, 13, 14, 15, 16, 17, 18, 19, or 20.

The light chain CDR3 includes QQ*YTTPPT (SEQ ID NO: 5), or a sequence extended by 1-5 amino acids (for example, 1, 2, 3, 4, or 5 amino acids) at the N-terminus and/or the C-terminus thereof. The extension refers to an extension in the N-terminal and/or C-terminal direction relative to the position of the light chain CDR3 within the light chain variable region of the antibody or antigen-binding fragment thereof in which the light chain CDR3 is present.

In a specific embodiment of the present disclosure, the light chain CDR3 includes QQ*YTTPPT (SEQ ID NO: 5), CQQ*YTTPPT (SEQ ID NO: 6), YCQQ*YTTPPT (SEQ ID NO: 7), YYCQQ*YTTPPT (SEQ ID NO: 8), TYYCQQ*YTTPPT (SEQ ID NO: 9), QQ*YTTPPTF (SEQ ID NO: 10), QQ*YTTPPTFG (SEQ ID NO: 11), QQ*YTTPPTFGQ (SEQ ID NO: 12), QQ*YTTPPTFGQG (SEQ ID NO: 13), CQQ*YTTPPTF (SEQ ID NO: 14), CQQ*YTTPPTFG (SEQ ID NO: 15), CQQ*YTTPPTFGQ (SEQ ID NO: 16), CQQ*YTTPPTFGQG (SEQ ID NO: 17), YCQQ*YTTPPTF (SEQ ID NO: 18), YCQQ*YTTPPTFG (SEQ ID NO: 19), YCQQ*YTTPPTFGQ (SEQ ID NO: 20), YCQQ*YTTPPTFGQG (SEQ ID NO: 21), YYCQQ*YTTPPTF (SEQ ID NO: 22), YYCQQ*YTTPPTFG (SEQ ID NO: 23), YYCQQ*YTTPPTFGQ (SEQ ID NO: 24), YYCQQ*YTTPPTFGQG (SEQ ID NO: 25), TYYCQQ*YTTPPTF (SEQ ID NO: 26), TYYCQQ*YTTPPTFG (SEQ ID NO: 27), TYYCQQ*YTTPPTFGQ (SEQ ID NO: 28), or TYYCQQ*YTTPPTFGQG (SEQ ID NO: 29), or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 5 to SEQ ID NO: 29, wherein * represents a natural amino acid or an unnatural amino acid and denotes a site where the small molecule compound is conjugated to the antibody or antigen-binding fragment thereof.

The conjugation site on the antibody or antigen-binding fragment thereof may be mutated or may remain unmutated. For example, the site may be mutated to cysteine (Cys), or an azide group may be introduced by an unnatural amino acid, or another natural or unnatural amino acid suitable for conjugation to a small molecule compound or a linker may be introduced.

The small molecule compound may include a modification or may be unmodified. For example, the small molecule compound may include a modification that enhances binding to or inhibition of the target antigen, or a modification that substantially does not affect binding to or inhibition of the target antigen, or a modification that has no significant effect on binding to or inhibition of the target antigen. Specifically, the small molecule compound may include a modification suitable for conjugation to an amino acid or a linker, such as a modified maleimide terminal group, a modified cyclooctyne terminal group, or a derivative thereof.

The antibody or antigen-binding fragment thereof is site-specifically conjugated to the small molecule compound. Preferably, the site-specific conjugation is performed preferably by means of a Michael addition reaction or a Huisgen reaction.

Preferably, the Michael addition reaction includes: introducing a cysteine (Cys) at the conjugation site of the antibody or antigen-binding fragment thereof, modifying the small molecule compound with a maleimide terminal group, and performing a Michael addition reaction. For example, an amino acid residue at the conjugation site may be mutated to cysteine (Cys), followed by modification of the small molecule compound with a maleimide terminal group and performance of the Michael addition reaction. Alternatively, when the CDR region itself contains a cysteine residue, the cysteine residue may be directly subjected to a Michael addition reaction with a small molecule compound modified with a maleimide terminal group.

Preferably, the Huisgen reaction includes: introducing UAA-N₃ (an azide group introduced via an unnatural amino acid) at the conjugation site of the antibody or antigen-binding fragment thereof, modifying the small molecule compound with a cyclooctyne terminal group or a derivative thereof, and performing a Huisgen reaction. The cyclooctyne terminal group or the derivative thereof is selected from DIFO, BCN, DIBAC, DIBO, ADIBO, or DBCO.

The antibody or antigen-binding fragment thereof is conjugated to the small molecule compound via a linker group. Preferably, the linker group may be a linker peptide, a PEG moiety,

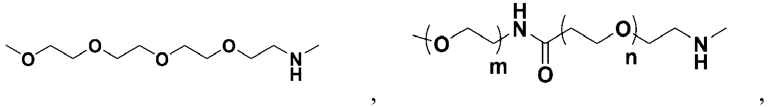

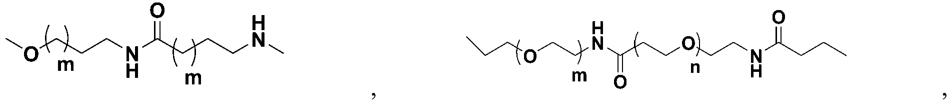

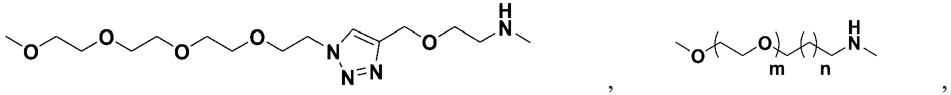

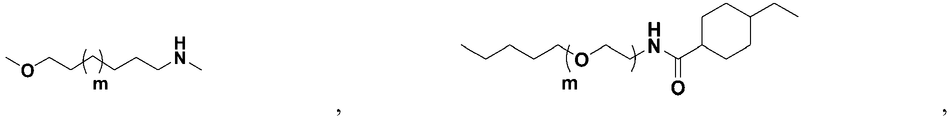

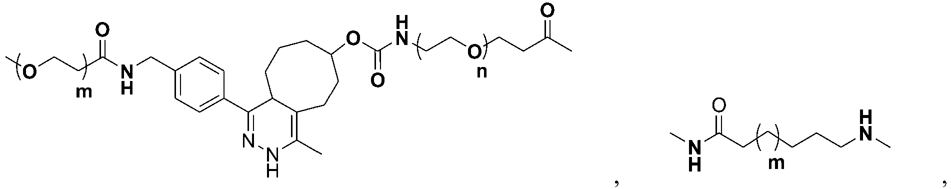

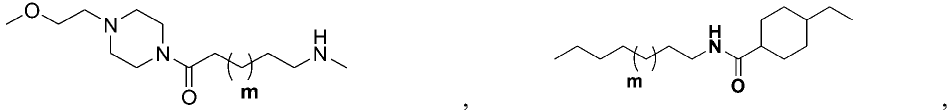

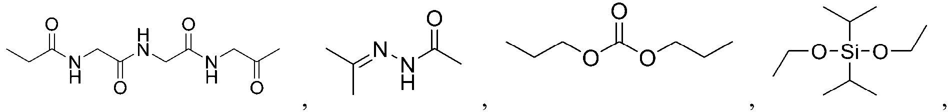

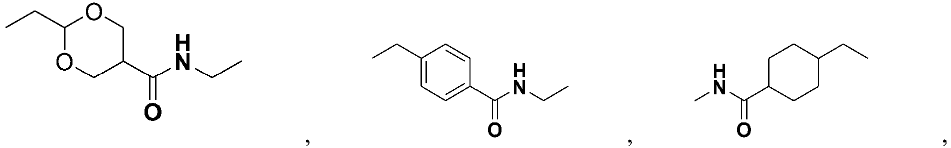

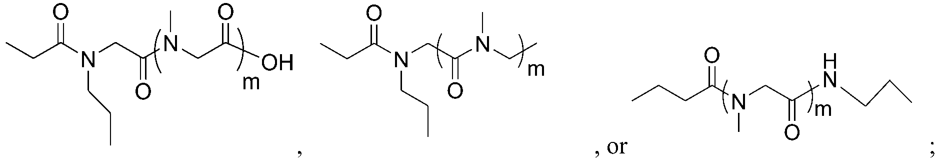

where m and n are each independently an integer selected from 1 to 20, for example, 1, 2, 3, 4, 5, 6, 7, 8, 9, 10, 11, 12, 13, 14, 15, 16, 17, 18, 19, or 20.

Preferably, the linker peptide may include LLTPPPPPLFPPPFF (SEQ ID NO: 240, J Mol Biol. 2013; 425:2100-32), (EAAAK)ₙ (n = 2-5, wherein n = 2, 3, 4, and 5 correspond to SEQ ID NO: 241, 242, 243, and 244, respectively, Proteins. 2004; 57:829-38), E₄K₄ (SEQ ID NO: 245, J Biol Chem. 2014; 289:25460-7), (E₄R₄)₂ (SEQ ID NO: 246, J Biol Chem. 2014; 289:25460-7*),* (E₄K₄)₂ (SEQ ID NO: 148, J Biol Chem. 2014; 289:25460-7), KLYPYDVPDYA (SEQ ID NO: 139, J Mol Biol. 2005; 354:546-55), PKPSTPPGSS (SEQ ID NO: 130, J Biotechnol. 2015; 199:90-7), GSPAG (SEQ ID NO: 121, Nucleic Acids Res. 2020;48(4):e24*),* GGASPAGG (SEQ ID NO: 113, Nucleic Acids Res. 2020;48(4):e24)*,* GGASPAAPAPAG (SEQ ID NO: 106, Nucleic Acids Res. 2020;48(4):e24), or AMGPSSGAPGGGGS (SEQ ID NO: 98, Enzyme Microb Technol. 2016;82:105-109), or a sequence having at least 70%, 75%, 80%, 85%, 90%, 91%, 92%, 93%, 94%, 95%, 96%, 97%, 98%, 99%, 99.5%, or 99.9% identity to any of SEQ ID NO: 98, 106, 113, 121, 130, 139, 148, and 240 to 246.

Preferably, 2-10 (e.g., 2, 3, 4, 5, 6, 7, 8, 9, or 10) PEG moieties are further present between the small molecule compound and the antibody or antigen-binding fragment thereof. More preferably, 3-5 PEG moieties are further present between the small molecule compound and the antibody or antigen-binding fragment thereof.

For example, the structure of PEG3-DBCO is as follows:

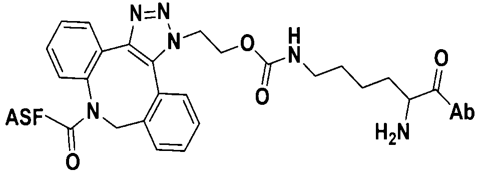

As another example, the structure of PEG3-Mal is as follows:

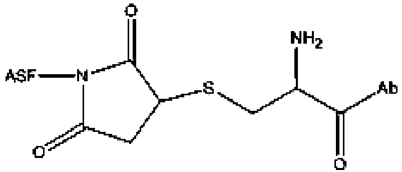

Preferably, the small molecule compound is an inhibitor of the target antigen. Such small molecule compound includes, for example, PSB12379, ASF, or ZM241385.

The target antigen may be a target protein, peptide, lipid, polysaccharide, or polynucleotide. Preferably, the target protein is selected from a membrane protein, a membrane transport protein, a receptor protein, a complex formed by a membrane protein and a membrane protein, or a complex formed by a membrane protein and another protein.

More preferably, the membrane protein is a peripheral membrane protein, an integral membrane protein, or a lipid-anchored protein.

More preferably, the membrane transport protein is a carrier protein or a channel protein. Preferably, the channel protein is a sodium ion channel protein, a calcium ion channel protein, a chloride ion channel protein, a potassium ion channel protein, an Ach receptor channel, or an amino acid receptor channel.

More preferably, the receptor protein is an intracellular receptor, a cell surface receptor, an ion channel receptor, a G-protein-coupled receptor (GPCR), or an enzyme-linked receptor.

In a specific embodiment of the present disclosure, the target antigen may be Naᵥ1.1, Naᵥ1.2, Naᵥ1.3, Naᵥ1.4, Naᵥ1.5, Naᵥ1.6, Naᵥ1.7, Naᵥ1.8, Naᵥ1.9, CD73, VEGFA, VEGFB, VEGFC, VEGFD, VEGFR, FGF, FGFR, PIGF, PDGF, ANG2, Endoglin (CD105), TGF, Integrin, Integrin receptor, IL-1B, IL-2, IL-3, IL-4, IL-6, IL-10, IL-12, IL-15, IL-17, IL-23, IL1R1, IL2Rα, IL3R, IL4Rα, IL6R, IL10R, IL12R, IL15Rα, IL17R, IL23R, PCSK9, TNF-α, TNFR, RANKL, complement protein C3, complement protein C5, GPCR, GLP1R, CD3, CD19, CD20, CD22, CD25, CD27, CD28, CD30, CD33, CD38, CD40, CD47, CD80, CD86, CD96, CD99, CD111, CD112, CD123, CD133, CD138, CD155, CD171, Claudin 18.2, OX40, ICOS, CTLA4, 4-1BB, TCR, B7-1, B7-2, BTLA, TIM-3, LAG3, Galectin-9, PD-L1, PD-L2, PD-1, TIGIT, EGFR, Her2, PSCA, CEA, FAP, EGFRVIII, BCMA, PSMA, CA125, EphA2, C-met, L1CAM, CS1, ROR1, EC, NY-ESO-1, MUC1, MUC16, mesothelin, LewisY, GPC3, GD2, EPG, DLL 3, A2aR, or 5T4.

The antibody derivative may be: 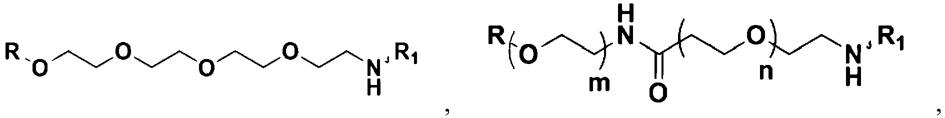 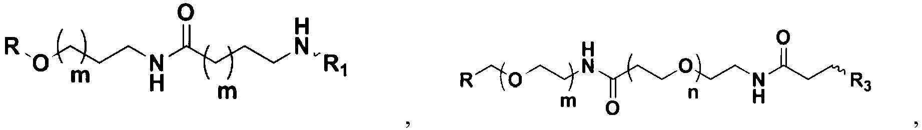 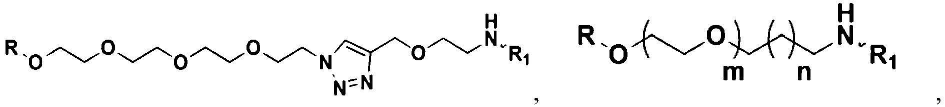 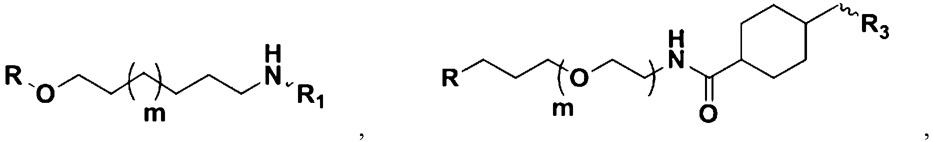 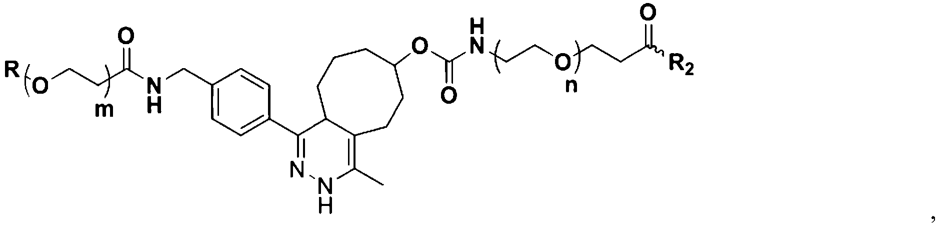 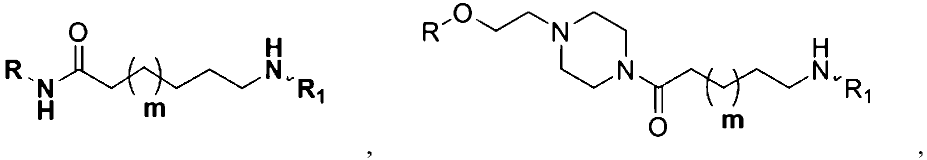 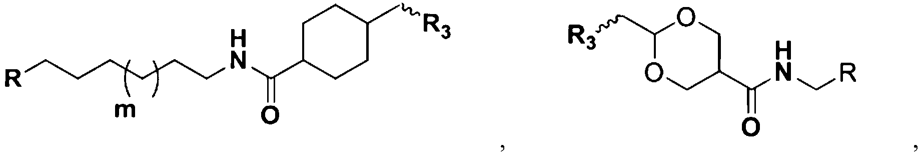 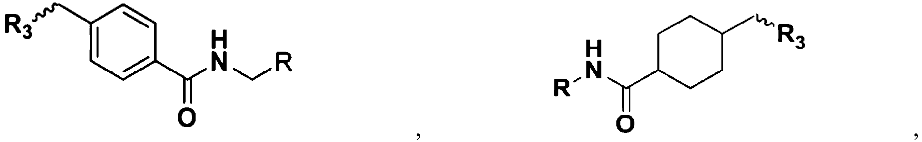 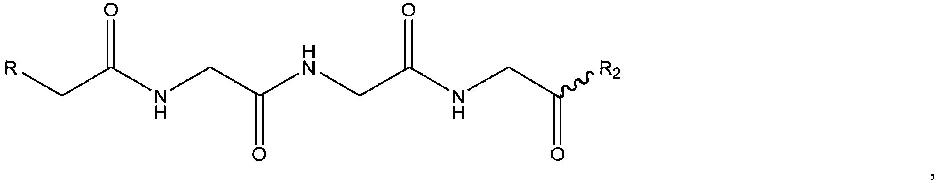 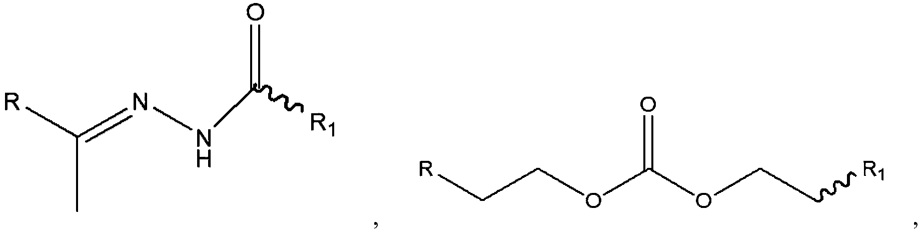 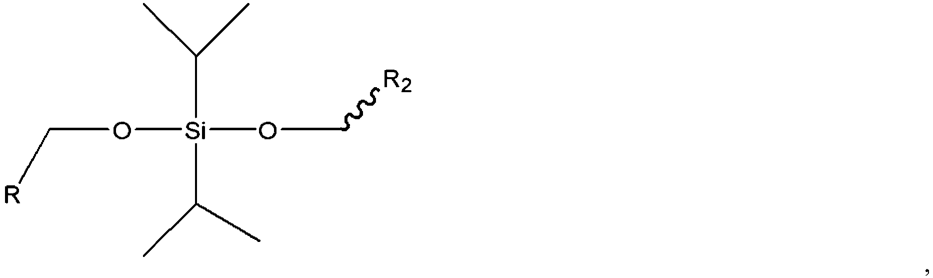 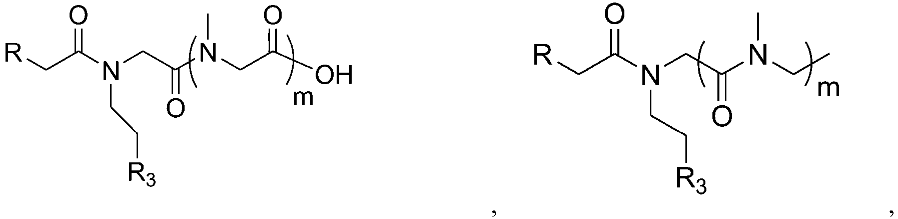 or

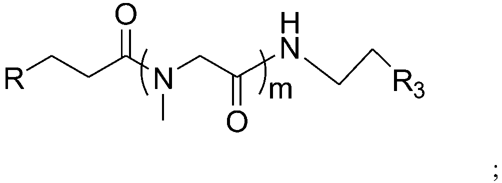

where m and n are each independently an integer selected from 1 to 20, for example, 1, 2, 3, 4, 5, 6, 7, 8, 9, 10, 11, 12, 13, 14, 15, 16, 17, 18, 19, or 20.

Wherein R is a small molecule compound, R1 is 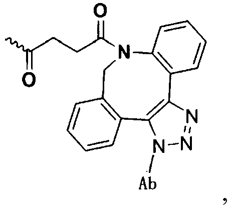 R2 is 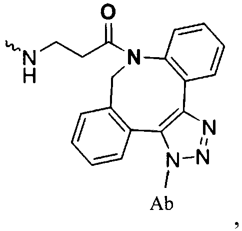 and R3 is 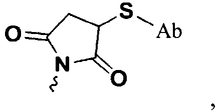 where Ab is an antibody.

The antibody or antigen-binding fragment thereof may be Fab, Fv, Fd, Fab', Fab'-SH, F(ab')2, a nanobody, a single-domain antibody, or a single-chain antibody.

The antibody or antigen-binding fragment thereof is a CD73 antibody or antigen-binding fragment thereof. The CD73 antibody or antigen-binding fragment thereof includes a heavy chain variable region and/or a light chain variable region. The light chain variable region includes VLCDR1, VLCDR2, and VLCDR3, and the heavy chain variable region includes VHCDR1, VHCDR2, and VHCDR3.

The VLCDR1 includes the amino acid sequence as set forth in any one of SEQ ID NO: 30 to SEQ ID NO: 39, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 30 to SEQ ID NO: 39.

The VLCDR2 includes the amino acid sequence as set forth in any one of SEQ ID NO: 40 to SEQ ID NO: 45, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 40 to SEQ ID NO: 45.

The VLCDR3 includes the amino acid sequence as set forth in any one of SEQ ID NO: 5 to SEQ ID NO: 29, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 5 to SEQ ID NO: 29.

The VHCDR1 includes the amino acid sequence as set forth in any one of SEQ ID NO: 46 to SEQ ID NO: 55, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 46 to SEQ ID NO: 55.

The VHCDR2 includes the amino acid sequence as set forth in any one of SEQ ID NO: 56 to SEQ ID NO: 64, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 56 to SEQ ID NO: 64.

The VHCDR3 includes the amino acid sequence as set forth in any one of SEQ ID NO: 65 to SEQ ID NO: 72, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 65 to SEQ ID NO: 72.

VLCDR1 and VLCDR2, respectively, include:

| Antibody No. | VL CDR1 | VL CDR2 |
|---|---|---|
| 10D8 | RASQSISSWLA (SEQ ID NO: 30) | KLLIYDVSNVQTGVPS (SEQ ID NO: 40) |
| 10A7 | RASQGISRWLA (SEQ ID NO: 31) | NLLIYGASTLQSGVPP (SEQ ID NO: 41) |
| 4C10 | RATQAISNYLA (SEQ ID NO: 32) | KLLIYDVSNVQTGVPS (SEQ ID NO: 40) |
| 5E12 | RTSQGITDPLN (SEQ ID NO: 33) | KLLIYKTSSLESGVPS (SEQ ID NO: 42) |
| 5H12 | RASQGLSSWLA (SEQ ID NO: 34) | KLLIYDASNLETGVPS (SEQ ID NO: 43) |
| 6D11 | RASQNIGTYLA (SEQ ID NO: 35) | KLLIYDASNLETGVPS (SEQ ID NO: 43) |
| ABN3-2B | RASQGINNYLA (SEQ ID NO: 36) | KLLIYDASNLETGVPS (SEQ ID NO: 43) |
| ABN3-2G | QASHDISKYLN (SEQ ID NO: 37) | KLLIYDASNLETGVPS (SEQ ID NO: 43) |
| ABN3-2A | RASETIGSWLA (SEQ ID NO: 38) | NLLIYAASTLQSGVSS (SEQ ID NO: 44) |
| 10F8 | QASQDISNYLN (SEQ ID NO: 39) | NLLIYAASTLQSGVPS (SEQ ID NO: 45) |

VHCDR1, VHCDR2, and VHCDR3, respectively, include:

| Antibody No. | VH CDR1 | VH CDR2 | VH CDR3 |
|---|---|---|---|
| ABN3-2A | FTLSTYAMH (SEQ ID NO: 46) | WLARIDWDDDKYYSTS LKTRL (SEQ ID NO: 56) | ARGRDFGEPPYVRAFDI (SEQ ID NO: 65) |
| ABN3-2B | FTFSSYWMH (SEQ ID NO: 47) | WVSAVSGSGDSTYYADS VKGRF (SEQ ID NO: 57) | AREGDWGLDY (SEQ ID NO: 66) |
| ABN3-2G | ITFGSYTMS (SEQ ID NO: 48) | WIGTVHYTGKTFYSPSL KSRV (SEQ ID NO: 58) | ARGRDFGEPPYVRAFDI (SEQ ID NO: 65) |
| 4C10 | FTFSSYWMN (SEQ ID NO: 49) | WVANIKQDGSEKYYVD SVKGRF (SEQ ID NO: 59) | VTGSYRYAY (SEQ ID NO: 67) |
| 5E12 | FTFSNYAMH (SEQ ID NO: 50) | WVSAISGSGGSTYYADS VKGRF (SEQ ID NO: 60) | ARGGGNYYDDY (SEQ ID NO: 68) |
| 5H12 | FTFSHYAMH (SEQ ID NO: 51) | WIGNIYYSGSTYYNPSL KSRV (SEQ ID NO: 61) | AKGHGSGSYFPYYFDY (SEQ ID NO: 69) |
| 6D11 | FTFSGHWMH (SEQ ID NO: 52) | WIGSIYYSGSTYYNPSLK SRV (SEQ ID NO: 62) | ARDRGWLQFDY (SEQ ID NO: 70) |
| 10A7 | FAFSSYSMS (SEQ ID NO: 53) | WIGSIYYSGSTYYNPSLK SRV (SEQ ID NO: 62) | ARNSGFETLDY (SEQ ID NO: 71) |
| 10D8 | FTFSTYAIH (SEQ ID NO: 54) | WVSSISGSGGGGGTYYA DSVKGRF (SEQ ID NO: 63) | ARDRGFLQFDY (SEQ ID NO: 72) |
| 10F8 | FTFSSYGMH (SEQ ID NO: 55) | WVAAISYDGSNELYADS VKGRF (SEQ ID NO: 64) | ARDRGWLQFDY (SEQ ID NO: 70) |

The heavy chain variable region includes the sequence as set forth in any one of SEQ ID NO: 73 to SEQ ID NO: 82, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 73 to SEQ ID NO: 82.

The light chain variable region includes the sequence as set forth in any one of SEQ ID NO: 83 to SEQ ID NO: 92, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 83 to SEQ ID NO: 92.

In a specific embodiment of the present disclosure, the antibody or antigen-binding fragment thereof is a CD73 antibody or antigen-binding fragment thereof, and the small molecule compound is PSB12379 having a structure of:

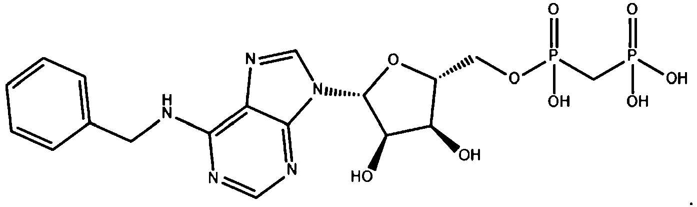

In a specific embodiment of the present disclosure, the antibody derivative is formed by conjugating the light chain CDR3 of a CD73 antibody or antigen-binding fragment thereof to (PEG)₂-ₗo-PSB12379 via a Huisgen reaction.

The antibody or antigen-binding fragment thereof is a Naᵥ1.7 antibody or antigen-binding fragment thereof. The Naᵥ1.7 antibody or antigen-binding fragment thereof includes a heavy chain variable region and/or a light chain variable region. The light chain variable region includes VLCDR1, VLCDR2, and VLCDR3, and the heavy chain variable region includes VHCDR1, VHCDR2, and VHCDR3.

The VLCDR1 includes the sequence as set forth in any one of SEQ ID NO: 93 to 97 and 99 to 100, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 93 to 97 and 99 to 100.

The VLCDR2 includes the sequence as set forth in any one of SEQ ID NO: 101 to 105 and 107 to 108, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 101 to 105 and 107 to 108.

The VLCDR3 includes the amino acid sequence as set forth in any one of SEQ ID NO: 5 to SEQ ID NO: 29, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 5 to SEQ ID NO: 29.

The VHCDR1 includes the sequence as set forth in any one of SEQ ID NO: 109 to 112 and 114, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 109 to 112 and 114.

The VHCDR2 includes the sequence as set forth in any one of SEQ ID NO: 115 to 120 and 122 to 123, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 115 to 120 and 122 to 123.

The VHCDR3 includes the sequence as set forth in any one of SEQ ID NO: 124 to 129 and 131 to 132, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 124 to 129 and 131 to 132.

VLCDR1 and VLCDR2, respectively, include:

| Antibody No. | VL CDR1 | VL CDR2 |
|---|---|---|
| F3 | RASQVIGNDLG (SEQ ID NO: 93) | KLLIYDVSNVQTGVPS (SEQ ID NO: 101) |
| G3 | RASQYISTYLN (SEQ ID NO: 94) | KVLISKTSYLESGVPS (SEQ ID NO: 102) |
| H3 | RASQSISSWLA (SEQ ID NO: 95) | NLLMYAASSLQSGVPS (SEQ ID NO: 103) |
| A4 | QASQDIRNDLN (SEQ ID NO: 96) | KSLIYAASRLQSGVPS (SEQ ID NO: 104) |
| 15F7 | RASQGISSDLG (SEQ ID NO: 97) | KLLIYDVSNVQTGVPS (SEQ ID NO: 101) |
| 15F9 | RASQSISSWLA (SEQ ID NO: 95) | KLLIYDASSLESGVPS (SEQ ID NO: 105) |
| 15A10 | RASQSIGSSLH (SEQ ID NO: 99) | TVLIYAASSLQTGVPS (SEQ ID NO: 107) |
| 15C12 | QASQDISNSLN (SEQ ID NO: 100) | NLLIYATSNLQSGVPS (SEQ ID NO: 108) |

VHCDR1, VHCDR2, and VHCDR3, respectively, include:

| Antibody No. | VH CDR1 | VH CDR2 | VH CDR3 |
|---|---|---|---|
| F3 | FTFSGHWMH (SEQ ID NO: 109) | WVASISDSSSYIYYADSV RGRF (SEQ ID NO: 115) | ARDYEGATRAADY (SEQ ID NO: 124) |
| G3 | FTFSASYMS (SEQ ID NO: 110) | WIGSIYYSGSTYYNPSLK SRV (SEQ ID NO: 116) | ARGAPYYEGAFDI (SEQ ID NO: 125) |
| H3 | FTFSDYWIS (SEQ ID NO: 111) | WIGNVYYSGNAYYNPSL ESRV (SEQ ID NO: 117) | ARNTGFETLDN (SEQ ID NO: 126) |
| A4 | FTFSGHWMH (SEQ ID NO: 109) | WVSGISGSGGSTYYADS VKGRF (SEQ ID NO: 118) | ARELTMATTTDAFDI (SEQ ID NO: 127) |
| 15F7 | FTFSGHWMH (SEQ ID NO: 109) | WVSAISGSGGSTYYADS VKGRF (SEQ ID NO: 119) | AREVTMVRGVTDAFDI (SEQ ID NO: 128) |
| 15F9 | FTFSKFWMH (SEQ ID NO: 112) | WVSSISSSSSYIYYADSV KGRF (SEQ ID NO: 120) | ARDPISTGAFDI (SEQ ID NO: 129) |
| 15A10 | FPFSGHWMH (SEQ ID NO: 114) | WVSGISGTGATSHYADS VKGRF (SEQ ID NO: 122) | AREVITAASTDAFDL (SEQ ID NO: 131) |
| 15C12 | FTFSGHWMH (SEQ ID NO: 109) | WVSAISGSGGSTYYADS VKGRF (SEQ ID NO: 123) | ARELTMASTTDAFDF (SEQ ID NO: 132) |

The heavy chain variable region includes the sequence as set forth in any one of SEQ ID NO: 142 to 147 and 149 to 150, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 142 to 147 and 149 to 150.

The light chain variable region includes the sequence as set forth in any one of SEQ ID NO: 133 to 138 and 140 to 141, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 133 to 138 and 140 to 141.

The antibody or antigen-binding fragment thereof is a Naᵥ1.2 antibody or antigen-binding fragment thereof. The Naᵥ1.2 antibody or antigen-binding fragment thereof includes a heavy chain variable region and/or a light chain variable region. The light chain variable region includes VLCDR1, VLCDR2, and VLCDR3, and the heavy chain variable region includes VHCDR1, VHCDR2, and VHCDR3.

The VLCDR1 includes the sequence as set forth in any one of SEQ ID NO: 94, 95, and 151 to 156, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 94, 95, and 151 to 156.

The VLCDR2 includes the sequence as set forth in any one of SEQ ID NO: 102, 103, and 157 to 163, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 102, 103, and 157 to 163.

The VLCDR3 includes the amino acid sequence as set forth in any one of SEQ ID NO: 5 to SEQ ID NO: 29, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 5 to SEQ ID NO: 29.

The VHCDR1 includes the sequence as set forth in any one of SEQ ID NO: 110, 111, and 164 to 170, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 110, 111, and 164 to 170.

The VHCDR2 includes the sequence as set forth in any one of SEQ ID NO: 116, 117, and 171 to 177, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 116, 117, and 171 to 177.

The VHCDR3 includes the sequence as set forth in any one of SEQ ID NO: 125, 126, and 178 to 183, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 125, 126, and 178 to 183.

VLCDR1 and VLCDR2, respectively, include:

| Antibody No. | VL CDR1 | VL CDR2 |
|---|---|---|
| H3 | RASQSISSWLA (SEQ ID NO: 95) | NLLMYAASSLQSGVPS (SEQ ID NO: 103) |
| G3 | RASQYISTYLN (SEQ ID NO: 94) | KVLISKTSYLESGVPS (SEQ ID NO: 102) |
| 12B5 | QASQGISHALA (SEQ ID NO: 151) | KLLIYAASSLQSGVPS (SEQ ID NO: 157) |
| 14H3 | RASQTISDWLA (SEQ ID NO: 152) | KLLIYDASSLESGVPS (SEQ ID NO: 158) |
| 14H4 | RASQSISSDLN (SEQ ID NO: 153) | KLLIYRATFLESGVPS (SEQ ID NO: 159) |
| 12D4 | QASQDISNYLN (SEQ ID NO: 154) | KLLIYAASRLQSGVPS (SEQ ID NO: 160) |
| 14B8 | RASQSISSWLA (SEQ ID NO: 95) | NLLMYAASSLQSGVPS (SEQ ID NO: 161) |
| 14C7 | RTSQSVNNHLN (SEQ ID NO: 155) | KLLIYRASSLESGVPS (SEQ ID NO: 162) |
| 14E6 | RASQPISDWLA (SEQ ID NO: 156) | KSLIYDAYNLQSGVPP (SEQ ID NO: 163) |

VHCDR1, VHCDR2, and VHCDR3, respectively, include:

| | | | |
|---|---|---|---|
| G3 | FTFSASYMS (SEQ ID NO: 110) | WIGSIYYSGSTYYNPSLKSRV (SEQ ID NO: 116) | ARGAPYYEGAFDI (SEQ ID NO: 125) |
| H3 | FTFSDYWIS (SEQ ID NO: 111) | WIGNVYYSGNAYYNPSLESRV (SEQ ID NO: 117) | ARNTGFETLDN (SEQ ID NO: 126) |
| 12B5 | FTFSTYVMH (SEQ ID NO: 164) | WLANIDTGGGVTNYADSVKGRF (SEQ ID NO: 171) | ARNSGFETLDY (SEQ ID NO: 178) |
| 12D4 | FTFSDYAMT (SEQ ID NO: 165) | VWVSRIKHDGSSTAYADSVKGRF (SEQ ID NO: 172) | AREGNYCLDY (SEQ ID NO: 179) |
| 14H3 | FTFSSYCMN (SEQ ID NO: 166) | WVSSVTGSAQRTNYADSVKGRF (SEQ ID NO: 173) | ARNTGFETLDN (SEQ ID NO: 126) |
| 14H4 | FSFSKFWMH (SEQ ID NO: 167) | WVAFMAHDESNKNYADSVKGRF (SEQ ID NO: 174) | ARDKVCNTFDI (SEQ ID NO: 180) |
| 14E6 | FTFSNFAMH (SEQ ID NO: 168) | YVSAISGGNGGGTYYADSAKGRF (SEQ ID NO: 175) | AREGNYCLDY (SEQ ID NO: 181) |
| 14C7 | FTFSSFSMS (SEQ ID NO: 169) | WVANIKEDGSDKYYVDSVTGRF (SEQ ID NO: 176) | ARDWGAAAVLHH (SEQ ID NO: 182) |
| 14B8 | FTFSGHWMH (SEQ ID NO: 170) | WVAVISSDGSYKSYADSVKGRF (SEQ ID NO: 177) | ASRTVAGTLDY (SEQ ID NO: 183) |

The heavy chain variable region includes the sequence as set forth in any one of SEQ ID NO: 143, 144, and 191 to 197, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 143, 144, and 191 to 197.

The light chain variable region includes the sequence as set forth in any one of SEQ ID NO: 134, 135, and 184 to 190, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 134, 135, and 184 to 190.

The antibody or antigen-binding fragment thereof is a Naᵥ1.6 antibody or antigen-binding fragment thereof. The Naᵥ1.6 antibody or antigen-binding fragment thereof includes a heavy chain variable region and/or a light chain variable region. The light chain variable region includes VLCDR1, VLCDR2, and VLCDR3, and the heavy chain variable region includes VHCDR1, VHCDR2, and VHCDR3.

The VLCDR1 includes the sequence as set forth in any one of SEQ ID NO: 93, 95, 96, and 198 to 203, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 93, 95, 96, and 198 to 203.

The VLCDR2 includes the sequence as set forth in any one of SEQ ID NO: 101, 103, 104, and 204 to 209, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 101, 103, 104, and 204 to 209.

The VLCDR3 includes the amino acid sequence as set forth in any one of SEQ ID NO: 5 to SEQ ID NO: 29, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 5 to SEQ ID NO: 29.

The VHCDR1 includes the sequence as set forth in any one of SEQ ID NO: 109, 111, and 210 to 215, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 109, 111, and 210 to 215.

The VHCDR2 includes the sequence as set forth in any one of SEQ ID NO: 115, 117, 118, and 216 to 221, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 115, 117, 118, and 216 to 221.

The VHCDR3 includes the sequence as set forth in any one of SEQ ID NO: 124, 126, 127, and 222 to 227, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 124, 126, 127, and 222 to 227.

VLCDR1 and VLCDR2, respectively, include:

| Antibody No. | VL CDR1 | VL CDR2 |
|---|---|---|
| F3 | RASQVIGNDLG (SEQ ID NO: 93) | KLLIYDVSNVQTGVPS (SEQ ID NO: 101) |
| H3 | RASQSISSWLA (SEQ ID NO: 95) | NLLMYAASSLQSGVPS (SEQ ID NO: 103) |
| A4 | QASQDIRNDLN (SEQ ID NO: 96) | KSLIYAASRLQSGVPS (SEQ ID NO: 104) |
| 15F6 | QASQDISHYLN (SEQ ID NO: 198) | KLLIHRVSSLESGVPS (SEQ ID NO: 204) |
| 15D3 | RASQGISSDLG (SEQ ID NO: 199) | KLLIYDVSNVQTGVPS (SEQ ID NO: 205) |
| 15C1 | RASETIGSWLA (SEQ ID NO: 200) | KLLIYKSTTVEGGVSS (SEQ ID NO: 206) |
| 15G4 | RASQNINRHLN (SEQ ID NO: 201) | KLLIYDVSNVQTGVPS (SEQ ID NO: 207) |
| 15A2 | QASQDIGKFLN (SEQ ID NO: 202) | KLLISATSNLQWGVPS (SEQ ID NO: 208) |
| 5A5 | RASQSISDWLA (SEQ ID NO: 203) | KLLIYAASSLQSGVPS (SEQ ID NO: 209) |

VHCDR1, VHCDR2, and VHCDR3, respectively, include:

| Antibody No. | VH CDR1 | VH CDR2 | VH CDR3 |
|---|---|---|---|
| F3 | FTFSGHWMH (SEQ ID NO: 109) | WVASISDSSSYIYYADSVR GRF (SEQ ID NO: 115) | ARDYEGATRAADY (SEQ ID NO: 124) |
| H3 | FTFSDYWIS (SEQ ID NO: 111) | WIGNVYYSGNAYYNPSLE SRV (SEQ ID NO: 117) | ARNTGFETLDN (SEQ ID NO: 126) |
| A4 | FTFSGHWMH (SEQ ID NO: 109) | WVSGISGSGGSTYYADSV KGRF (SEQ ID NO: 118) | ARELTMATTTDAFDI (SEQ ID NO: 127) |
| 15F6 | FSFSTYAMH (SEQ ID NO: 210) | WVANIDGDGGVTNYAGS VKGRF (SEQ ID NO: 216) | ARDRGWLQFDY (SEQ ID NO: 222) |
| 15D3 | FTFSGHWMH (SEQ ID NO: 211) | WVSAISGSGGSTYYADSV KGRF (SEQ ID NO: 217) | AREVTMVRGVTDAFDI (SEQ ID NO: 223) |
| 15C1 | FTFTSYAMA (SEQ ID NO: 212) | WVSSISNGGSVIYYANSV KGRF (SEQ ID NO: 218) | ARDRGFLQFDY (SEQ ID NO: 224) |
| 15G4 | FTFDEYIMH (SEQ ID NO: 213) | YVSAISSNGGSTYYANSV KGRF (SEQ ID NO: 219) | ARDRGFLQFDY (SEQ ID NO: 225) |
| 15A2 | FSFSNFAMT (SEQ ID NO: 214) | WVSSISNGGSVIYYANSV KGRF (SEQ ID NO: 220) | ARDRGFLQFDY (SEQ ID NO: 226) |
| 5A5 | FPFSGHWMH (SEQ ID NO: 215) | WVSGISWNGGTTGYADSV KGRF (SEQ ID NO: 221) | ARDDYGGDSFDS (SEQ ID NO: 227) |

The heavy chain variable region includes the sequence as set forth in any one of SEQ ID NO: 142, 144, 145, and 234 to 239, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 142, 144, 145, and 234 to 239.

The light chain variable region includes the sequence as set forth in any one of SEQ ID NO: 133, 135, 136, and 228 to 233, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 133, 135, 136, and 228 to 233.

In a specific embodiment of the present disclosure, the antibody or antigen-binding fragment thereof is a Naᵥ1.2 antibody or antigen-binding fragment thereof, a Naᵥ1.6 antibody or antigen-binding fragment thereof, or a Naᵥ1.7 antibody or antigen-binding fragment thereof, and the small molecule compound is an arylsulfonamide compound, preferably 5-(2-(4-(N-(1,2,4-thiadiazol-5-sulfonamido)-2,5-difluorophenoxy)-5-chlorobenzyl)benzisoxaz ol)-3-amine (ASF), having the structure of:

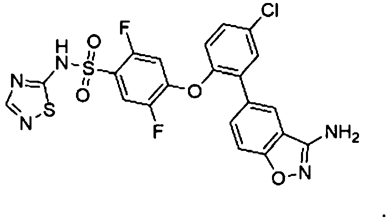

In a specific embodiment of the present disclosure, the antibody derivative is formed by conjugating the light chain CDR3 of a Naᵥ1.2 antibody or antigen-binding fragment thereof, a Naᵥ1.6 antibody or antigen-binding fragment thereof, or a Naᵥ1.7 antibody or antigen-binding fragment thereof to (PEG)₂₋₁₀-ASF via a Huisgen reaction.

The antibody or antigen-binding fragment thereof is an A2aR antibody or antigen-binding fragment thereof. The A2aR antibody or antigen-binding fragment thereof includes a heavy chain variable region and/or a light chain variable region. The light chain variable region includes VLCDR1, VLCDR2, and VLCDR3, and the heavy chain variable region includes VHCDR1, VHCDR2, and VHCDR3.

The VLCDR1 includes the sequence as set forth in any one of SEQ ID NO: 30 and SEQ ID NO: 247 to 250, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 30 and SEQ ID NO: 247 to 250.

The VLCDR2 includes the sequence as set forth in any one of SEQ ID NO: 251 to 255 and SEQ ID NO: 284, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 251 to 255 and SEQ ID NO: 284.

The VLCDR3 includes the amino acid sequence as set forth in any one of SEQ ID NO: 5 to SEQ ID NO: 29, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 5 to SEQ ID NO: 29.

The VHCDR1 includes the amino acid sequence as set forth in any one of SEQ ID NO: 256 to SEQ ID NO: 260, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 256 to SEQ ID NO: 260.

The VHCDR2 includes the amino acid sequence as set forth in any one of SEQ ID NO: 261 to SEQ ID NO: 266, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 261 to SEQ ID NO: 266.

The VHCDR3 includes the amino acid sequence as set forth in any one of SEQ ID NO: 267 to SEQ ID NO: 272, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 267 to SEQ ID NO: 272.

VLCDR1 and VLCDR2, respectively, include:

| Antibody No. | VL CDR1 | VL CDR2 |
|---|---|---|
| 6G5 | QASQDIRNSLN (SEQ ID NO: 247) | KLLIKYASQSISGVPS (SEQ ID NO: 251) |
| 5C9 | RASQSISRALA (SEQ ID NO: 248) | KLLIYKASSLQSGVPS (SEQ ID NO: 252) |
| 6A6 | QASQSISRYLS (SEQ ID NO: 249) | KLLIYKTSRLESGVPS (SEQ ID NO: 253) |
| 5D10 | QASQDIRNSLN (SEQ ID NO: 247) | KLLIYAASTLQSGVPS (SEQ ID NO: 254) |
| 5F9 | QASQDIRNSLN (SEQ ID NO: 247) | KLLIYAASTLQSGVPS (SEQ ID NO: 254) |
| 6D6 | RASQNIGTSLH (SEQ ID NO: 250) | KLLISRASSLQSGVPS (SEQ ID NO: 255) |
| 6C6 | RASQSISSWLA (SEQ ID NO: 30) | KLLIYKTSNLESGVPS (SEQ ID NO: 284) |

VHCDR1, VHCDR2, and VHCDR3, respectively, include:

| Antibody No. | VH CDR1 | VH CDR2 | VH CDR3 |
|---|---|---|---|
| 6G5 | FTFSGHWMH (SEQ ID NO: 256) | WIGTVSYNGRTYYNPSLK SRV (SEQ ID NO: 261) | ARGASGRTYNNFFDP (SEQ ID NO: 267) |
| 5C9 | FTVGSNSMT (SEQ ID NO: 257) | WVSSISGTGGRTYYADSV KGRF (SEQ ID NO: 262) | ARILPVRGASGQWAR KNWFDP (SEQ ID NO: 268) |
| 6A6 | FTFSGHWMH (SEQ ID NO: 256) | WVSSITGDSNYRYYADSV KGRF (SEQ ID NO: 263) | ARDRSGSLRAFDY (SEQ ID NO: 269) |
| 5D10 | FTFGSFWMS (SEQ ID NO: 258) | WVSSISNGGSVIYYANSV KGRF (SEQ ID NO: 264) | ARGLRRASGPFDY (SEQ ID NO: 270) |
| 5F9 | FRFSSYSMN (SEQ ID NO: 259) | WVSGISGSGGSTYYADPV KGRF (SEQ ID NO: 265) | ARGGRGPTTPFDY (SEQ ID NO: 271) |
| 6D6 | FTFSNHAMH (SEQ ID NO: 260) | WVSAITGNGRSKYYADS VKGRF (SEQ ID NO: 266) | ARDRGWLQFDY (SEQ ID NO: 272) |
| 6C6 | FTFSNHAMH (SEQ ID NO: 260) | WVSAITGNGRSKYYADS VKGRF (SEQ ID NO: 266) | ARDRGWLQFDY (SEQ ID NO: 272) |

The heavy chain variable region includes the sequence as set forth in any one of SEQ ID NO: 273 to SEQ ID NO: 278, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 273 to SEQ ID NO: 278.

The light chain variable region includes the sequence as set forth in any one of SEQ ID NO: 279 to 283 and SEQ ID NO: 285, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 279 to 283 and SEQ ID NO: 285.

In a specific embodiment of the present disclosure, the antibody or antigen-binding fragment thereof is an A2aR antibody or antigen-binding fragment thereof, and the small molecule compound is ZM241385 having a structure of:

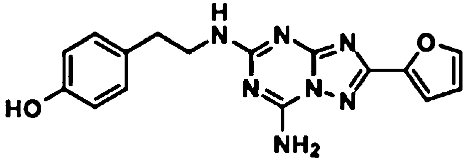

In a specific embodiment of the present disclosure, the antibody derivative is formed by conjugating the light chain CDR3 of an A2aR antibody or antigen-binding fragment thereof to (PEG)₂₋₁₀-ZM241385 (ZP3) via a Huisgen reaction.

In a second aspect, the present disclosure provides a method for preparing the antibody derivative according to the first aspect. The method includes conjugating an antibody or antigen-binding fragment thereof to a small molecule compound. The small molecule compound and the antibody or antigen-binding fragment thereof jointly participate in binding to a target antigen.

The antibody or antigen-binding fragment thereof is site-specifically conjugated to the small molecule compound, for example, via a Michael addition reaction or a Huisgen reaction.

The small molecule compound is capable of binding to a target antigen.

In a third aspect, the present disclosure provides a CD73 antibody or antigen-binding fragment thereof. The CD73 antibody or antigen-binding fragment thereof includes a heavy chain variable region and/or a light chain variable region. The light chain variable region includes VLCDR1, VLCDR2, and VLCDR3, and the heavy chain variable region includes VHCDR1, VHCDR2, and VHCDR3.

The VLCDR1 includes the amino acid sequence as set forth in any one of SEQ ID NO: 30 to SEQ ID NO: 39, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 30 to SEQ ID NO: 39.

The VLCDR2 includes the amino acid sequence as set forth in any one of SEQ ID NO: 40 to SEQ ID NO: 45, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 40 to SEQ ID NO: 45.

The VLCDR3 includes the sequence as set forth in any one of SEQ ID NO: 5 to SEQ ID NO: 29, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 5 to SEQ ID NO: 29, wherein * represents a natural amino acid or an unnatural amino acid.

The VHCDR1 includes the amino acid sequence as set forth in any one of SEQ ID NO: 46 to SEQ ID NO: 55, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 46 to SEQ ID NO: 55.

The VHCDR2 includes the amino acid sequence as set forth in any one of SEQ ID NO: 56 to SEQ ID NO: 64, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 56 to SEQ ID NO: 64.

The VHCDR3 includes the amino acid sequence as set forth in any one of SEQ ID NO: 65 to SEQ ID NO: 72, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 65 to SEQ ID NO: 72.

VLCDR1 and VLCDR2, respectively, include:

| Antibody No. | VL CDR1 | VL CDR2 |
|---|---|---|
| 10D8 | RASQSISSWLA (SEQ ID NO: 30) | KLLIYDVSNVQTGVPS (SEQ ID NO: 40) |
| 10A7 | RASQGISRWLA (SEQ ID NO: 31) | NLLIYGASTLQSGVPP (SEQ ID NO: 41) |
| 4C10 | RATQAISNYLA (SEQ ID NO: 32) | KLLIYDVSNVQTGVPS (SEQ ID NO: 40) |
| 5E12 | RTSQGITDPLN (SEQ ID NO: 33) | KLLIYKTSSLESGVPS (SEQ ID NO: 42) |
| 5H12 | RASQGLSSWLA (SEQ ID NO: 34) | KLLIYDASNLETGVPS (SEQ ID NO: 43) |
| 6D11 | RASQNIGTYLA (SEQ ID NO: 35) | KLLIYDASNLETGVPS (SEQ ID NO: 43) |
| ABN3-2B | RASQGINNYLA (SEQ ID NO: 36) | KLLIYDASNLETGVPS (SEQ ID NO: 43) |
| ABN3-2G | QASHDISKYLN (SEQ ID NO: 37) | KLLIYDASNLETGVPS (SEQ ID NO: 43) |
| ABN3-2A | RASETIGSWLA (SEQ ID NO: 38) | NLLIYAASTLQSGVSS (SEQ ID NO: 44) |
| 10F8 | QASQDISNYLN (SEQ ID NO: 39) | NLLIYAASTLQSGVPS (SEQ ID NO: 45) |

VHCDR1, VHCDR2, and VHCDR3, respectively, include:

| Antibody No. | VH CDR1 | VH CDR2 | VH CDR3 |
|---|---|---|---|
| ABN3-2A | FTLSTYAMH (SEQ ID NO: 46) | WLARIDWDDDKYYSTSLKTRL (SEQ ID NO: 56) | ARGRDFGEPPYVRAF DI (SEQ ID NO: 65) |
| ABN3-2B | FTFSSYWMH (SEQ ID NO: 47) | WVSAVSGSGDSTYYADSVKGRF (SEQ ID NO: 57) | AREGDWGLDY (SEQ ID NO: 66) |
| ABN3-2G | ITFGSYTMS (SEQ ID NO: 48) | WIGTVHYTGKTFYSPSLKSRV (SEQ ID NO: 58) | ARGRDFGEPPYVRAF DI (SEQ ID NO: 65) |
| 4C10 | FTFSSYWMN (SEQ ID NO: 49) | WVANIKQDGSEKYYVDSVKGRF (SEQ ID NO: 59) | VTGSYRYAY (SEQ ID NO: 67) |
| 5E12 | FTFSNYAMH (SEQ ID NO: 50) | WVSAISGSGGSTYYADSVKGRF (SEQ ID NO: 60) | ARGGGNYYDDY (SEQ ID NO: 68) |
| 5H12 | FTFSHYAMH (SEQ ID NO: 51) | WIGNIYYSGSTYYNPSLKSRV (SEQ ID NO: 61) | AKGHGSGSYFPYYFD Y (SEQ ID NO: 69) |
| 6D11 | FTFSGHWMH (SEQ ID NO: 52) | WIGSIYYSGSTYYNPSLKSRV (SEQ ID NO: 62) | ARDRGWLQFDY (SEQ ID NO: 70) |
| 10A7 | FAFSSYSMS (SEQ ID NO: 53) | WIGSIYYSGSTYYNPSLKSRV (SEQ ID NO: 62) | ARNSGFETLDY (SEQ ID NO: 71) |
| 10D8 | FTFSTYAIH (SEQ ID NO: 54) | WVSSISGSGGGGGTYYADSVKG RF (SEQ ID NO: 63) | ARDRGFLQFDY (SEQ ID NO: 72) |
| 10F8 | FTFSSYGMH (SEQ ID NO: 55) | WVAAISYDGSNELYADSVKGRF (SEQ ID NO: 64) | ARDRGWLQFDY (SEQ ID NO: 70) |

The heavy chain variable region includes the sequence as set forth in any one of SEQ ID NO: 73 to SEQ ID NO: 82, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 73 to SEQ ID NO: 82.

The light chain variable region includes the sequence as set forth in any one of SEQ ID NO: 83 to SEQ ID NO: 92, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 83 to SEQ ID NO: 92.

The antibody or antigen-binding fragment thereof is Fab, Fv, Fd, Fab', Fab'-SH, F(ab')2, a nanobody, a single-domain antibody, or a single-chain antibody.

In a fourth aspect, the present disclosure provides a Naᵥ1.2 antibody or antigen-binding fragment thereof. The Naᵥ1.2 antibody or antigen-binding fragment thereof includes a heavy chain variable region and/or a light chain variable region. The light chain variable region includes VLCDR1, VLCDR2, and VLCDR3, and the heavy chain variable region includes VHCDR1, VHCDR2, and VHCDR3.

The VLCDR1 includes the sequence as set forth in any one of SEQ ID NO: 94, 95, and 151 to 156, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 94, 95, and 151 to 156.

The VLCDR2 includes the sequence as set forth in any one of SEQ ID NO: 102, 103, and 157 to 163, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 102, 103, and 157 to 163.

The VLCDR3 includes the sequence as set forth in any one of SEQ ID NO: 5 to SEQ ID NO: 29, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 5 to SEQ ID NO: 29, wherein * represents a natural amino acid or an unnatural amino acid.

The VHCDR1 includes the sequence as set forth in any one of SEQ ID NO: 110, 111, and 164 to 170, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 110, 111, and 164 to 170.

The VHCDR2 includes the sequence as set forth in any one of SEQ ID NO: 116, 117, and 171 to 177, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 116, 117, and 171 to 177.

The VHCDR3 includes the sequence as set forth in any one of SEQ ID NO: 125, 126, and 178 to 183, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 125, 126, and 178 to 183.

VLCDR1 and VLCDR2, respectively, include:

| Antibody No. | VL CDR1 | VL CDR2 |
|---|---|---|
| H3 | RASQSISSWLA (SEQ ID NO: 95) | NLLMYAASSLQSGVPS (SEQ ID NO: 103) |
| G3 | RASQYISTYLN (SEQ ID NO: 94) | KVLISKTSYLESGVPS (SEQ ID NO: 102) |
| 12B5 | QASQGISHALA (SEQ ID NO: 151) | KLLIYAASSLQSGVPS (SEQ ID NO: 157) |
| 14H3 | RASQTISDWLA (SEQ ID NO: 152) | KLLIYDASSLESGVPS (SEQ ID NO: 158) |
| 14H4 | RASQSISSDLN (SEQ ID NO: 153) | KLLIYRATFLESGVPS (SEQ ID NO: 159) |
| 12D4 | QASQDISNYLN (SEQ ID NO: 154) | KLLIYAASRLQSGVPS (SEQ ID NO: 160) |
| 14B8 | RASQSISSWLA (SEQ ID NO: 95) | NLLMYAASSLQSGVPS (SEQ ID NO: 161) |
| 14C7 | RTSQSVNNHLN (SEQ ID NO: 155) | KLLIYRASSLESGVPS (SEQ ID NO: 162) |
| 14E6 | RASQPISDWLA (SEQ ID NO: 156) | KSLIYDAYNLQSGVPP (SEQ ID NO: 163) |

VHCDR1, VHCDR2, and VHCDR3, respectively, include:

| | | | |
|---|---|---|---|
| G3 | FTFSASYMS | WIGSIYYSGSTYYNPSLKSRV | ARGAPYYEGAFDI |
| | (SEQ ID NO: 110) | (SEQ ID NO: 116) | (SEQ ID NO: 125) |
| H3 | FTFSDYWIS (SEQ ID NO: 111) | WIGNVYYSGNAYYNPSLESRV (SEQ ID NO: 117) | ARNTGFETLDN (SEQ ID NO: 126) |
| 12B5 | FTFSTYVMH (SEQ ID NO: 164) | WLANIDTGGGVTNYADSVKGR F (SEQ ID NO: 171) | ARNSGFETLDY (SEQ ID NO: 178) |
| 12D4 | FTFSDYAMT (SEQ ID NO: 165) | VWVSRIKHDGSSTAYADSVKG RF (SEQ ID NO: 172) | AREGNYCLDY (SEQ ID NO: 179) |
| 14H3 | FTFSSYCMN (SEQ ID NO: 166) | WVSSVTGSAQRTNYADSVKGR F (SEQ ID NO: 173) | ARNTGFETLDN (SEQ ID NO: 126) |
| 14H4 | FSFSKFWMH (SEQ ID NO: 167) | WVAFMAHDESNKNYADSVKG RF (SEQ ID NO: 174) | ARDKVCNTFDI (SEQ ID NO: 180) |
| 14E6 | FTFSNFAMH (SEQ ID NO: 168) | YVSAISGGNGGGTYYADSAKG RF (SEQ ID NO: 175) | AREGNYCLDY (SEQ ID NO: 181) |
| 14C7 | FTFSSFSMS (SEQ ID NO: 169) | WVANIKEDGSDKYYVDSVTGR F (SEQ ID NO: 176) | ARDWGAAAVLHH (SEQ ID NO: 182) |
| 14B8 | FTFSGHWMH (SEQ ID NO: 170) | WVAVISSDGSYKSYADSVKGRF (SEQ ID NO: 177) | ASRTVAGTLDY (SEQ ID NO: 183) |

The heavy chain variable region includes the sequence as set forth in any one of SEQ ID NO: 143, 144, and 191 to 197, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 143, 144, and 191 to 197.

The light chain variable region includes the sequence as set forth in any one of SEQ ID NO: 134, 135, and 184 to 190, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 134, 135, and 184 to 190.

The antibody or antigen-binding fragment thereof is Fab, Fv, Fd, Fab', Fab'-SH, F(ab')2, a nanobody, a single-domain antibody, or a single-chain antibody.

In a fifth aspect, the present disclosure provides a Naᵥ1.6 antibody or antigen-binding fragment thereof. The Naᵥ1.6 antibody or antigen-binding fragment thereof includes a heavy chain variable region and/or a light chain variable region. The light chain variable region includes VLCDR1, VLCDR2, and VLCDR3, and the heavy chain variable region includes VHCDR1, VHCDR2, and VHCDR3.

The VLCDR1 includes the sequence as set forth in any one of SEQ ID NO: 93, 95, 96, and 198 to 203, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 93, 95, 96, and 198 to 203.

The VLCDR2 includes the sequence as set forth in any one of SEQ ID NO: 101, 103, 104, and 204 to 209, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 101, 103, 104, and 204 to 209.

The VLCDR3 includes the sequence as set forth in any one of SEQ ID NO: 5 to SEQ ID NO: 29, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 5 to SEQ ID NO: 29, wherein * represents a natural amino acid or an unnatural amino acid.

The VHCDR1 includes the sequence as set forth in any one of SEQ ID NO: 109, 111, and 210 to 215, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 109, 111, and 210 to 215.

The VHCDR2 includes the sequence as set forth in any one of SEQ ID NO: 115, 117, 118, and 216 to 221, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 115, 117, 118, and 216 to 221.

The VHCDR3 includes the sequence as set forth in any one of SEQ ID NO: 124, 126, 127, and 222 to 227, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 124, 126, 127, and 222 to 227.

VLCDR1 and VLCDR2, respectively, include:

| Antibody No. | VL CDR1 | VL CDR2 |
|---|---|---|
| F3 | RASQVIGNDLG (SEQ ID NO: 93) | KLLIYDVSNVQTGVPS (SEQ ID NO: 101) |
| H3 | RASQSISSWLA (SEQ ID NO: 95) | NLLMYAASSLQSGVPS (SEQ ID NO: 103) |
| A4 | QASQDIRNDLN (SEQ ID NO: 96) | KSLIYAASRLQSGVPS (SEQ ID NO: 104) |
| 15F6 | QASQDISHYLN (SEQ ID NO: 198) | KLLIHRVSSLESGVPS (SEQ ID NO: 204) |
| 15D3 | RASQGISSDLG (SEQ ID NO: 199) | KLLIYDVSNVQTGVPS (SEQ ID NO: 205) |
| 15C1 | RASETIGSWLA (SEQ ID NO: 200) | KLLIYKSTTVEGGVSS (SEQ ID NO: 206) |
| 15G4 | RASQNINRHLN (SEQ ID NO: 201) | KLLIYDVSNVQTGVPS (SEQ ID NO: 207) |
| 15A2 | QASQDIGKFLN (SEQ ID NO: 202) | KLLISATSNLQWGVPS (SEQ ID NO: 208) |
| 5A5 | RASQSISDWLA (SEQ ID NO: 203) | KLLIYAASSLQSGVPS (SEQ ID NO: 209) |

VHCDR1, VHCDR2, and VHCDR3, respectively, include:

| Antibody No. | VH CDR1 | VH CDR2 | VH CDR3 |
|---|---|---|---|
| F3 | FTFSGHWMH (SEQ ID NO: 109) | WVASISDSSSYIYYADSVRGR F (SEQ ID NO: 115) | ARDYEGATRAADY (SEQ ID NO: 124) |
| H3 | FTFSDYWIS (SEQ ID NO: 111) | WIGNVYYSGNAYYNPSLESR V (SEQ ID NO: 117) | ARNTGFETLDN (SEQ ID NO: 126) |
| A4 | FTFSGHWMH (SEQ ID NO: 109) | WVSGISGSGGSTYYADSVKG RF (SEQ ID NO: 118) | ARELTMATTTDAFDI (SEQ ID NO: 127) |
| 15F6 | FSFSTYAMH (SEQ ID NO: 210) | WVANIDGDGGVTNYAGSVK GRF (SEQ ID NO: 216) | ARDRGWLQFDY (SEQ ID NO: 222) |
| 15D3 | FTFSGHWMH (SEQ ID NO: 211) | WVSAISGSGGSTYYADSVKG RF (SEQ ID NO: 217) | AREVTMVRGVTDAFDI (SEQ ID NO: 223) |
| 15C1 | FTFTSYAMA (SEQ ID NO: 212) | WVSSISNGGSVIYYANSVKGR F (SEQ ID NO: 218) | ARDRGFLQFDY (SEQ ID NO: 224) |
| 15G4 | FTFDEYIMH (SEQ ID NO: 213) | YVSAISSNGGSTYYANSVKG RF (SEQ ID NO: 219) | ARDRGFLQFDY (SEQ ID NO: 225) |
| 15A2 | FSFSNFAMT (SEQ ID NO: 214) | WVSSISNGGSVIYYANSVKGR F (SEQ ID NO: 220) | ARDRGFLQFDY (SEQ ID NO: 226) |
| 5A5 | FPFSGHWMH (SEQ ID NO: 215) | WVSGISWNGGTTGYADSVK GRF (SEQ ID NO: 221) | ARDDYGGDSFDS (SEQ ID NO: 227) |

The heavy chain variable region includes the sequence as set forth in any one of SEQ ID NO: 142, 144, 145, and 234 to 239, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 142, 144, 145, and 234 to 239.

The light chain variable region includes the sequence as set forth in any one of SEQ ID NO: 133, 135, 136, and 228 to 233, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 133, 135, 136, and 228 to 233.

The antibody or antigen-binding fragment thereof is Fab, Fv, Fd, Fab', Fab'-SH, F(ab')2, a nanobody, a single-domain antibody, or a single-chain antibody.

In a sixth aspect, the present disclosure provides a Naᵥ1.7 antibody or antigen-binding fragment thereof. The Naᵥ1.7 antibody or antigen-binding fragment thereof includes a heavy chain variable region and/or a light chain variable region. The light chain variable region includes VLCDR1, VLCDR2, and VLCDR3, and the heavy chain variable region includes VHCDR1, VHCDR2, and VHCDR3.

The VLCDR1 includes the sequence as set forth in any one of SEQ ID NO: 93 to 97 and 99 to 100, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 93 to 97 and 99 to 100.

The VLCDR2 includes the sequence as set forth in any one of SEQ ID NO: 101 to 105 and 107 to 108, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 101 to 105 and 107 to 108.

The VLCDR3 includes the sequence as set forth in any one of SEQ ID NO: 5 to SEQ ID NO: 29, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 5 to SEQ ID NO: 29, wherein * represents a natural amino acid or an unnatural amino acid.

The VHCDR1 includes the sequence as set forth in any one of SEQ ID NO: 109 to 112 and 114, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 109 to 112 and 114.

The VHCDR2 includes the sequence as set forth in any one of SEQ ID NO: 115 to 120 and 122 to 123, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 115 to 120 and 122 to 123.

The VHCDR3 includes the sequence as set forth in any one of SEQ ID NO: 124 to 129 and 131 to 132, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 124 to 129 and 131 to 132.

VLCDR1 and VLCDR2, respectively, include:

| Antibody No. | VL CDR1 | VL CDR2 |
|---|---|---|
| F3 | RASQVIGNDLG (SEQ ID NO: 93) | KLLIYDVSNVQTGVPS (SEQ ID NO: 101) |
| G3 | RASQYISTYLN (SEQ ID NO: 94) | KVLISKTSYLESGVPS (SEQ ID NO: 102) |
| H3 | RASQSISSWLA (SEQ ID NO: 95) | NLLMYAASSLQSGVPS (SEQ ID NO: 103) |
| A4 | QASQDIRNDLN (SEQ ID NO: 96) | KSLIYAASRLQSGVPS (SEQ ID NO: 104) |
| 15F7 | RASQGISSDLG (SEQ ID NO: 97) | KLLIYDVSNVQTGVPS (SEQ ID NO: 101) |
| 15F9 | RASQSISSWLA (SEQ ID NO: 95) | KLLIYDASSLESGVPS (SEQ ID NO: 105) |
| 15A10 | RASQSIGSSLH (SEQ ID NO: 99) | TVLIYAASSLQTGVPS (SEQ ID NO: 107) |
| 15C12 | QASQDISNSLN (SEQ ID NO: 100) | NLLIYATSNLQSGVPS (SEQ ID NO: 108) |

VHCDR1, VHCDR2, and VHCDR3, respectively, include:

| Antibody No. | VH CDR1 | VH CDR2 | VH CDR3 |
|---|---|---|---|
| F3 | FTFSGHWMH (SEQ ID NO: 109) | WVASISDSSSYIYYADSV RGRF (SEQ ID NO: 115) | ARDYEGATRAADY (SEQ ID NO: 124) |
| G3 | FTFSASYMS (SEQ ID NO: 110) | WIGSIYYSGSTYYNPSLK SRV (SEQ ID NO: 116) | ARGAPYYEGAFDI (SEQ ID NO: 125) |
| H3 | FTFSDYWIS (SEQ ID NO: 111) | WIGNVYYSGNAYYNPSL ESRV (SEQ ID NO: 117) | ARNTGFETLDN (SEQ ID NO: 126) |
| A4 | FTFSGHWMH (SEQ ID NO: 109) | WVSGISGSGGSTYYADS VKGRF (SEQ ID NO: 118) | ARELTMATTTDAFDI (SEQ ID NO: 127) |
| 15F7 | FTFSGHWMH (SEQ ID NO: 109) | WVSAISGSGGSTYYADS VKGRF (SEQ ID NO: 119) | AREVTMVRGVTDAFDI (SEQ ID NO: 128) |
| 15F9 | FTFSKFWMH (SEQ ID NO: 112) | WVSSISSSSSYIYYADSV KGRF (SEQ ID NO: 120) | ARDPISTGAFDI (SEQ ID NO: 129) |
| 15A10 | FPFSGHWMH (SEQ ID NO: 114) | WVSGISGTGATSHYADS VKGRF (SEQ ID NO: 122) | AREVITAASTDAFDL (SEQ ID NO: 131) |
| 15C12 | FTFSGHWMH (SEQ ID NO: 109) | WVSAISGSGGSTYYADS VKGRF (SEQ ID NO: 123) | ARELTMASTTDAFDF (SEQ ID NO: 132) |

The heavy chain variable region includes the sequence as set forth in any one of SEQ ID NO: 142 to 147 and 149 to 150, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 142 to 147 and 149 to 150.

The light chain variable region includes the sequence as set forth in any one of SEQ ID NO: 133 to 138 and 140 to 141, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 133 to 138 and 140 to 141.

The antibody or antigen-binding fragment thereof is Fab, Fv, Fd, Fab', Fab'-SH, F(ab')2, a nanobody, a single-domain antibody, or a single-chain antibody.

In a seventh aspect, the present disclosure provides an A2aR antibody or antigen-binding fragment thereof. The A2aR antibody or antigen-binding fragment thereof includes a heavy chain variable region and/or a light chain variable region. The light chain variable region includes VLCDR1, VLCDR2, and VLCDR3, and the heavy chain variable region includes VHCDR1, VHCDR2, and VHCDR3.

The VLCDR1 includes the sequence as set forth in any one of SEQ ID NO: 30 and SEQ ID NO: 247 to 250, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 30 and SEQ ID NO: 247 to 250.

The VLCDR2 includes the sequence as set forth in any one of SEQ ID NO: 251 to 255 and SEQ ID NO: 284, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 251 to 255 and SEQ ID NO: 284.

The VLCDR3 includes the amino acid sequence as set forth in any one of SEQ ID NO: 5 to SEQ ID NO: 29, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 5 to SEQ ID NO: 29.

The VHCDR1 includes the amino acid sequence as set forth in any one of SEQ ID NO: 256 to SEQ ID NO: 260, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 256 to SEQ ID NO: 260.

The VHCDR2 includes the amino acid sequence as set forth in any one of SEQ ID NO: 261 to SEQ ID NO: 266, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 261 to SEQ ID NO: 266.

The VHCDR3 includes the amino acid sequence as set forth in any one of SEQ ID NO: 267 to SEQ ID NO: 272, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 267 to SEQ ID NO: 272.

VLCDR1 and VLCDR2, respectively, include:

| Antibody No. | VL CDR1 | VL CDR2 |
|---|---|---|
| 6G5 | QASQDIRNSLN (SEQ ID NO: 247) | KLLIKYASQSISGVPS (SEQ ID NO: 251) |
| 5C9 | RASQSISRALA (SEQ ID NO: 248) | KLLIYKASSLQSGVPS (SEQ ID NO: 252) |
| 6A6 | QASQSISRYLS (SEQ ID NO: 249) | KLLIYKTSRLESGVPS (SEQ ID NO: 253) |
| 5D10 | QASQDIRNSLN (SEQ ID NO: 247) | KLLIYAASTLQSGVPS (SEQ ID NO: 254) |
| 5F9 | QASQDIRNSLN (SEQ ID NO: 247) | KLLIYAASTLQSGVPS (SEQ ID NO: 254) |
| 6D6 | RASQNIGTSLH (SEQ ID NO: 250) | KLLISRASSLQSGVPS (SEQ ID NO: 255) |
| 6C6 | RASQSISSWLA (SEQ ID NO: 30) | KLLIYKTSNLESGVPS (SEQ ID NO: 284) |

VHCDR1, VHCDR2, and VHCDR3, respectively, include:

| Antibody No. | VH CDR1 | VH CDR2 | VH CDR3 |
|---|---|---|---|
| 6G5 | FTFSGHWMH (SEQ ID NO: 256) | WIGTVSYNGRTYYNPSLK SRV (SEQ ID NO: 261) | ARGASGRTYNNFFDP (SEQ ID NO: 267) |
| 5C9 | FTVGSNSMT (SEQ ID NO: 257) | WVSSISGTGGRTYYADSV KGRF (SEQ ID NO: 262) | ARILPVRGASGQWARKN WFDP (SEQ ID NO: 268) |
| 6A6 | FTFSGHWMH (SEQ ID NO: 256) | WVSSITGDSNYRYYADSV KGRF (SEQ ID NO: 263) | ARDRSGSLRAFDY (SEQ ID NO: 269) |
| 5D10 | FTFGSFWMS (SEQ ID NO: 258) | WVSSISNGGSVIYYANSV KGRF (SEQ ID NO: 264) | ARGLRRASGPFDY (SEQ ID NO: 270) |
| 5F9 | FRFSSYSMN (SEQ ID NO: 259) | WVSGISGSGGSTYYADPV KGRF (SEQ ID NO: 265) | ARGGRGPTTPFDY (SEQ ID NO: 271) |
| 6D6 | FTFSNHAMH (SEQ ID NO: 260) | WVSAITGNGRSKYYADS VKGRF (SEQ ID NO: 266) | ARDRGWLQFDY (SEQ ID NO: 272) |
| 6C6 | FTFSNHAMH (SEQ ID NO: 260) | WVSAITGNGRSKYYADS VKGRF (SEQ ID NO: 266) | ARDRGWLQFDY (SEQ ID NO: 272) |

The heavy chain variable region includes the sequence as set forth in any one of SEQ ID NO: 273 to SEQ ID NO: 278, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 273 to SEQ ID NO: 278.

The light chain variable region includes the sequence as set forth in any one of SEQ ID NO: 279 to 283 and SEQ ID NO: 285, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 279 to 283 and SEQ ID NO: 285.

The antibody or antigen-binding fragment thereof is Fab, Fv, Fd, Fab', Fab'-SH, F(ab')2, a nanobody, a single-domain antibody, or a single-chain antibody.

In an eighth aspect, the present disclosure provides a nucleic acid molecule encoding the above-mentioned CD73 antibody or antigen-binding fragment thereof, the above-mentioned Naᵥ1.2 antibody or antigen-binding fragment thereof, the above-mentioned Naᵥ1.6 antibody or antigen-binding fragment thereof, the above-mentioned Naᵥ1.7 antibody or antigen-binding fragment thereof, or the above-mentioned A2aR antibody or antigen-binding fragment thereof.

In a ninth aspect, the present disclosure provides a vector expressing the above-mentioned antibody or antigen-binding fragment thereof, or including the above-mentioned nucleic acid molecule.

In a tenth aspect, the present disclosure provides a host cell or immune cell including the above-mentioned antibody or antigen-binding fragment thereof, or the above-mentioned nucleic acid molecule, or the above-mentioned vector.

The host cell or immune cell expresses the above-mentioned antibody or antigen-binding fragment thereof.

Preferably, the immune cell includes, but is not limited to, lymphocytes (e.g., T cells, B cells, NK cells), dendritic cells, monocytes/macrophages, granulocytes, or mast cells.

In an eleventh aspect, the present disclosure provides a chimeric antigen receptor. The extracellular domain of the chimeric antigen receptor includes the above-mentioned antibody or antigen-binding fragment thereof.

In a twelfth aspect, the present disclosure provides a cell expressing the above-mentioned chimeric antigen receptor.

In a thirteenth aspect, the present disclosure provides a method for preparing the above-mentioned antibody or antigen-binding fragment thereof. The method includes introducing the above-mentioned nucleic acid molecule into a cell and inducing expression in the cell.

In a fourteenth aspect, the present disclosure provides use of a small molecule compound in:
A) the construction of a smacAb library,
   preferably, conjugating (preferably site-specifically conjugating) the small molecule compound to an antibody or antigen-binding fragment thereof in an antibody or an antigen-binding fragment library to obtain a smacAb library;
B) the screening of smacAb,
   preferably, conjugating (preferably site-specifically conjugating) the small molecule compound to an antibody or antigen-binding fragment thereof in an antibody or an antigen-binding fragment library to obtain a smacAb library, and screening with a target antigen to obtain the smacAb;
C) the improvement of binding strength or affinity between an antibody and a target antigen,
   preferably, conjugating (preferably site-specifically conjugating) the small molecule compound to an antibody or antigen-binding fragment thereof, wherein the small molecule compound and the antibody jointly participate in binding to the target antigen;
D) the diagnose and/or treatment of a disease;
E) the manufacture of a medicament for diagnosing and/or treating a disease.

Preferably, the disease may be a tumor, an infectious disease, or inflammation.

Preferably, the medicament includes the antibody derivative according to the first aspect.

The small molecule compound is selected from PSB12379, 5-(2-(4-(N-(1,2,4-thiadiazol-5-sulfonamido)-2,5-difluorophenoxy)-5-chlorophenyl)benzisoxa zol-3-amine, or ZM241385.

The target antigen may be a target protein, peptide, lipid, polysaccharide, or polynucleotide. Preferably, the target protein is selected from a membrane protein, membrane a transport protein, a receptor protein, a complex formed by a membrane protein and a membrane protein, or a complex formed by a membrane protein and another protein.

More preferably, the membrane protein is a peripheral membrane protein, an integral membrane protein, or a lipid-anchored protein.

More preferably, the membrane transport protein is a carrier protein or a channel protein. Preferably, the channel protein is a sodium ion channel protein, a calcium ion channel protein, a chloride ion channel protein, a potassium ion channel protein, an Ach receptor channel, or an amino acid receptor channel.

More preferably, the receptor protein is an intracellular receptor, a cell surface receptor, an ion channel receptor, a G-protein-coupled receptor (GPCR), or an enzyme-linked receptor.

Preferably, the antibody or antigen-binding fragment thereof is selected from the antibody or antigen-binding fragment thereof according to the third to seventh aspects as described above.

The mode of attachment between the small molecule compound and the antibody or antigen-binding fragment thereof, and the linker group therebetween, are as defined in the first aspect.

In a fifteenth aspect, the present disclosure provides a smacAb library. The smacAb library includes a small molecule compound conjugated to an antibody or antigen-binding fragment thereof in an antibody or antigen-binding fragment thereof library.

The small molecule compound is conjugated to a complementarity-determining region (CDR) of the antibody or antigen-binding fragment thereof. Preferably, the small molecule compound is conjugated to heavy chain CDR3 or light chain CDR3 of the antibody or antigen-binding fragment thereof. More preferably, the small molecule compound is conjugated to light chain CDR3 of the antibody or antigen-binding fragment thereof.

The light chain CDR3 has a length of 3 to 20 amino acids, e.g., 3, 4, 5, 6, 7, 8, 9, 10, 11, 12, 13, 14, 15, 16, 17, 18, 19, or 20 amino acids.

The small molecule compound is conjugated to one amino acid residue of the light chain CDR3 of the antibody or antigen-binding fragment thereof, preferably at position 1, 2, 3, 4, 5, 6, 7, 8, 9, 10, 11, 12, 13, 14, 15, 16, 17, 18, 19, or 20.

The light chain CDR3 includes QQ*YTTPPT (SEQ ID NO: 5), or a sequence extended by 1-5 amino acids (for example, 1, 2, 3, 4, or 5 amino acids) at the N-terminus and/or the C-terminus thereof. The extension refers to an extension in the N-terminal and/or C-terminal direction relative to the position of the light chain CDR3 within the light chain variable region of the antibody or antigen-binding fragment thereof in which the light chain CDR3 is present.

In a specific embodiment of the present disclosure, the light chain CDR3 includes QQ*YTTPPT (SEQ ID NO: 5), CQQ*YTTPPT (SEQ ID NO: 6), YCQQ*YTTPPT (SEQ ID NO: 7), YYCQQ*YTTPPT (SEQ ID NO: 8), TYYCQQ*YTTPPT (SEQ ID NO: 9), QQ*YTTPPTF (SEQ ID NO: 10), QQ*YTTPPTFG (SEQ ID NO: 11), QQ*YTTPPTFGQ (SEQ ID NO: 12), QQ*YTTPPTFGQG (SEQ ID NO: 13), CQQ*YTTPPTF (SEQ ID NO: 14), CQQ*YTTPPTFG (SEQ ID NO: 15), CQQ*YTTPPTFGQ (SEQ ID NO: 16), CQQ*YTTPPTFGQG (SEQ ID NO: 17), YCQQ*YTTPPTF (SEQ ID NO: 18), YCQQ*YTTPPTFG (SEQ ID NO: 19), YCQQ*YTTPPTFGQ (SEQ ID NO: 20), YCQQ*YTTPPTFGQG (SEQ ID NO: 21), YYCQQ*YTTPPTF (SEQ ID NO: 22), YYCQQ*YTTPPTFG (SEQ ID NO: 23), YYCQQ*YTTPPTFGQ (SEQ ID NO: 24), YYCQQ*YTTPPTFGQG (SEQ ID NO: 25), TYYCQQ*YTTPPTF (SEQ ID NO: 26), TYYCQQ*YTTPPTFG (SEQ ID NO: 27), TYYCQQ*YTTPPTFGQ (SEQ ID NO: 28), or TYYCQQ*YTTPPTFGQG (SEQ ID NO: 29), or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 5 to SEQ ID NO: 29, wherein * represents a natural amino acid or an unnatural amino acid and denotes a site where the small molecule compound is conjugated to the antibody or antigen-binding fragment thereof.

The small molecule compound is selected from PSB12379, 5-(2-(4-(N-(1,2,4-thiadiazol-5-sulfonamido)-2,5-difluorophenoxy)-5-chlorophenyl)benzisoxa zol-3-amine, or ZM241385.

The antibody or antigen-binding fragment thereof is site-specifically conjugated to the small molecule compound. Preferably, the site-specific conjugation is performed preferably by means of a Michael addition reaction or a Huisgen reaction.

The antibody or antigen-binding fragment thereof is conjugated to the small molecule compound via a linker group. Preferably, the linker group may be a linker peptide, a PEG moiety,

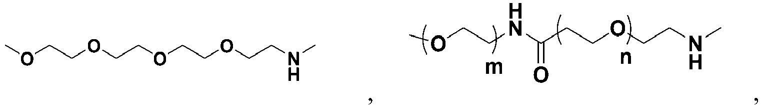

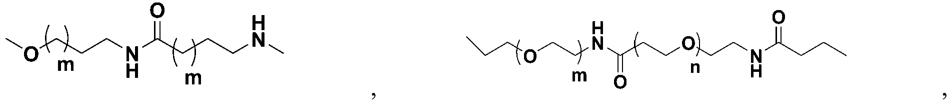

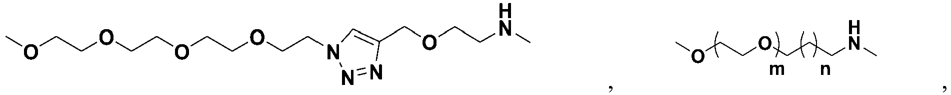

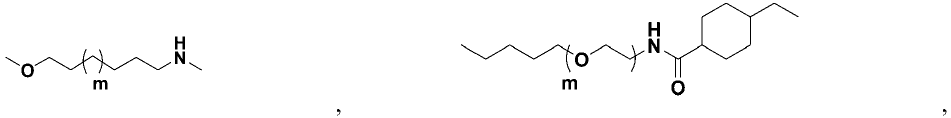

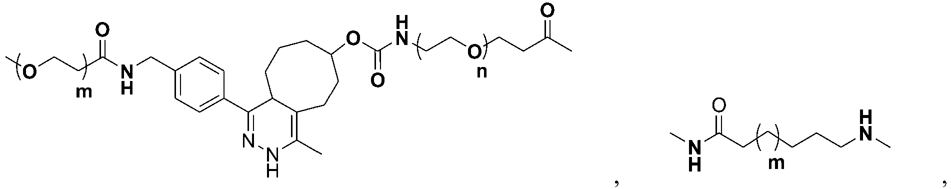

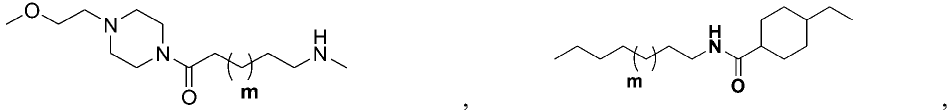

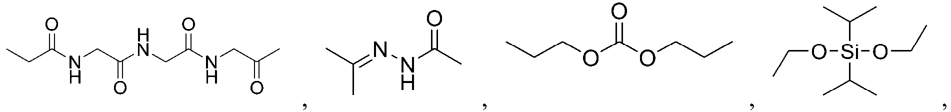

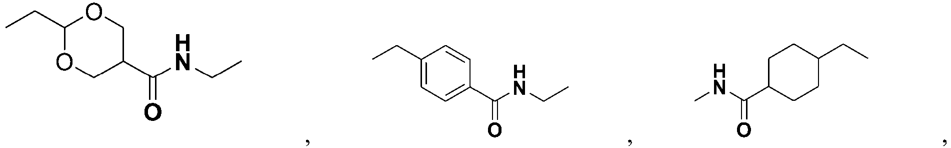

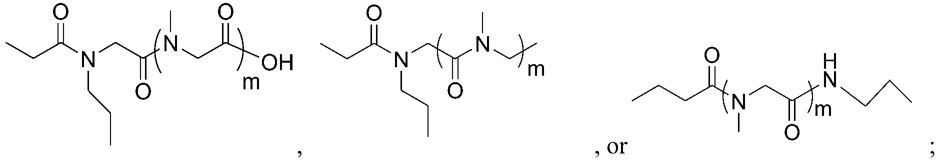

where m and n are each independently an integer selected from 1 to 20, for example, 1, 2, 3, 4, 5, 6, 7, 8, 9, 10, 11, 12, 13, 14, 15, 16, 17, 18, 19, or 20.

Preferably, the linker peptide may include LLTPPPPPLFPPPFF (SEQ ID NO: 240), (EAAAK)n (n = 2-5, wherein n = 2, 3, 4, and 5 correspond to SEQ ID NO: 241, 242, 243, and 244, respectively), E₄K₄ (SEQ ID NO: 245), (E₄R₄)₂ (SEQ ID NO: 246), (E₄K₄)₂ (SEQ ID NO: 148), KLYPYDVPDYA (SEQ ID NO: 139), PKPSTPPGSS (SEQ ID NO: 130), GSPAG (SEQ ID NO: 121), GGASPAGG (SEQ ID NO: 113), GGASPAAPAPAG (SEQ ID NO: 106), or AMGPSSGAPGGGGS (SEQ ID NO: 98), or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 98, 106, 113, 121, 130, 139, 148, and 240 to 246.

Two to ten PEG moieties are further present between the small molecule compound and the antibody or antigen-binding fragment thereof. Preferably, 3-5 PEG moieties are further present between the small molecule compound and the antibody or antigen-binding fragment thereof.

The smacAb library contains 10⁹ members of smacAb.

In a sixteenth aspect, the present disclosure provides a method for constructing a smacAb library. The method includes conjugating a displayed antibody or antigen-binding fragment thereof to a small molecule compound.

The display may be performed using a filamentous single-stranded phage display system, a λ phage display system, or a T4 phage display system.

The antibody or antigen-binding fragment thereof may be conjugated to one or more (e.g., 2, 3, 4, 5, 6, 7, 8, 9, 10, or more) small molecule compounds. When two small molecule compounds are conjugated, they may be identical or different. When three or more small molecule compounds are conjugated, they may be all identical, all different, or partially identical.

The small molecule compound is conjugated to a complementarity-determining region (CDR) of the antibody or antigen-binding fragment thereof. Preferably, the small molecule compound is conjugated to a heavy chain CDR or a light chain CDR of the antibody or antigen-binding fragment thereof, such as at least one of heavy chain CDR1, heavy chain CDR2, heavy chain CDR3, light chain CDR1, light chain CDR2, or light chain CDR3.

Preferably, the small molecule compound is conjugated to heavy chain CDR3 or light chain CDR3 of the antibody or antigen-binding fragment thereof. More preferably, the small molecule compound is conjugated to light chain CDR3 of the antibody or antigen-binding fragment thereof.

The CDR may have a length of 3 to 20 amino acids, e.g., 3, 4, 5, 6, 7, 8, 9, 10, 11, 12, 13, 14, 15, 16, 17, 18, 19, or 20 amino acids.

The conjugating may be at amino acid position 1, 2, 3, 4, 5, 6, 7, 8, 9, 10, 11, 12, 13, 14, 15, 16, 17, 18, 19, or 20 of the CDR.

The light chain CDR3 has a length of 3 to 20 amino acids.

The small molecule compound is conjugated to one amino acid residue of the light chain CDR3 of the antibody or antigen-binding fragment thereof, preferably at position 1, 2, 3, 4, 5, 6, 7, 8, 9, 10, 11, 12, 13, 14, 15, 16, 17, 18, 19, or 20.

The light chain CDR3 includes QQ*YTTPPT (SEQ ID NO: 5), CQQ*YTTPPT (SEQ ID NO: 6), YCQQ*YTTPPT (SEQ ID NO: 7), YYCQQ*YTTPPT (SEQ ID NO: 8), TYYCQQ*YTTPPT (SEQ ID NO: 9), QQ*YTTPPTF (SEQ ID NO: 10), QQ*YTTPPTFG (SEQ ID NO: 11), QQ*YTTPPTFGQ (SEQ ID NO: 12), QQ*YTTPPTFGQG (SEQ ID NO: 13), CQQ*YTTPPTF (SEQ ID NO: 14), CQQ*YTTPPTFG (SEQ ID NO: 15), CQQ*YTTPPTFGQ (SEQ ID NO: 16), CQQ*YTTPPTFGQG (SEQ ID NO: 17), YCQQ*YTTPPTF (SEQ ID NO: 18), YCQQ*YTTPPTFG (SEQ ID NO: 19), YCQQ*YTTPPTFGQ (SEQ ID NO: 20), YCQQ*YTTPPTFGQG (SEQ ID NO: 21), YYCQQ*YTTPPTF (SEQ ID NO: 22), YYCQQ*YTTPPTFG (SEQ ID NO: 23), YYCQQ*YTTPPTFGQ (SEQ ID NO: 24), YYCQQ*YTTPPTFGQG (SEQ ID NO: 25), TYYCQQ*YTTPPTF (SEQ ID NO: 26), TYYCQQ*YTTPPTFG (SEQ ID NO: 27), TYYCQQ*YTTPPTFGQ (SEQ ID NO: 28), or TYYCQQ*YTTPPTFGQG (SEQ ID NO: 29), or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 5 to SEQ ID NO: 29, wherein * represents a natural amino acid or an unnatural amino acid and denotes a site where the small molecule compound is conjugated to the antibody or antigen-binding fragment thereof.

The conjugation site on the antibody or antigen-binding fragment thereof may be mutated or may remain unmutated. For example, the site may be mutated to cysteine (Cys), or an azide group may be introduced by an unnatural amino acid, or another natural or unnatural amino acid suitable for conjugation to a small molecule compound or a linker may be introduced.

The small molecule compound may include a modification or may be unmodified. For example, the small molecule compound may include a modification that enhances binding to or inhibition of the target antigen, or a modification that substantially does not affect binding to or inhibition of the target antigen, or a modification that has no significant effect on binding to or inhibition of the target antigen. Specifically, the small molecule compound may include a modification suitable for conjugation to an amino acid or a linker, such as a modified maleimide terminal group, a modified cyclooctyne terminal group, or a derivative thereof.

The antibody or antigen-binding fragment thereof is site-specifically conjugated to the small molecule compound. Preferably, the site-specific conjugation is performed preferably by means of a Michael addition reaction or a Huisgen reaction.

Preferably, the Michael addition reaction includes: introducing a cysteine (Cys) at the conjugation site of the antibody or antigen-binding fragment thereof, modifying the small molecule compound with a maleimide terminal group, and performing a Michael addition reaction. For example, an amino acid residue at the conjugation site may be mutated to cysteine (Cys), followed by modification of the small molecule compound with a maleimide terminal group and performance of the Michael addition reaction. Alternatively, when the CDR region itself contains a cysteine residue, the cysteine residue may be directly subjected to a Michael addition reaction with a small molecule compound modified with a maleimide terminal group.

Preferably, the Huisgen reaction includes: introducing UAA-N₃ at the conjugation site of the antibody or antigen-binding fragment thereof, modifying the small molecule compound with a cyclooctyne terminal group or a derivative thereof, and performing a Huisgen reaction. The cyclooctyne terminal group or the derivative thereof is selected from DIFO, BCN, DIBAC, DIBO, ADIBO, or DBCO.

The antibody or antigen-binding fragment thereof is conjugated to the small molecule compound via a linker group. Preferably, the linker group may be a linker peptide, a PEG moiety,

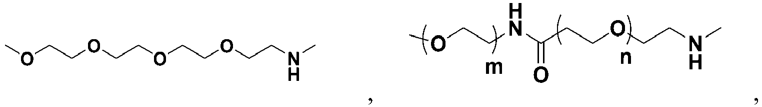

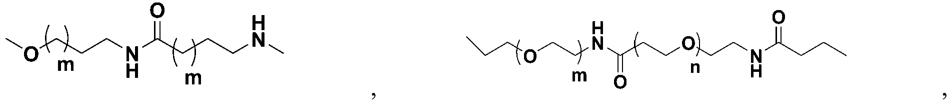

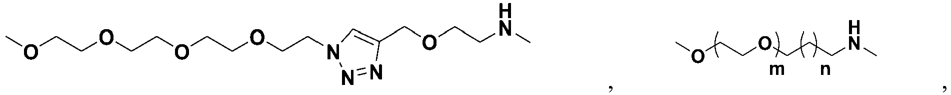

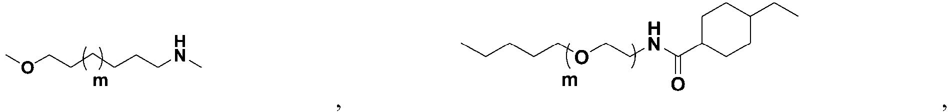

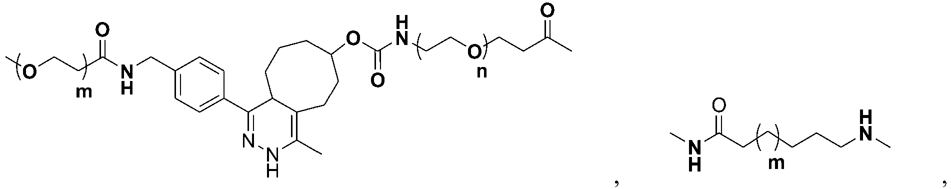

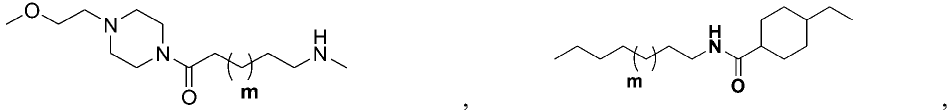

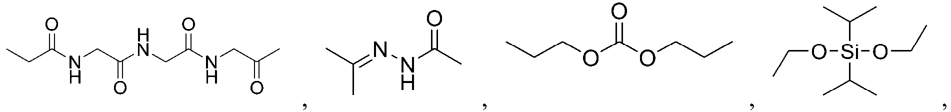

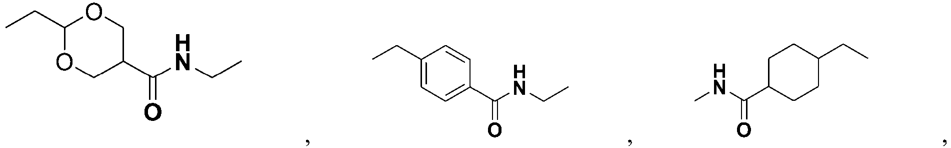

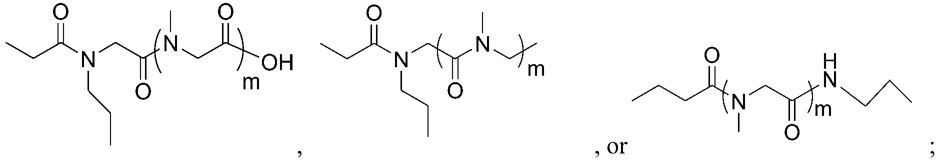

where m and n are each independently an integer selected from 1 to 20, for example, 1, 2, 3, 4, 5, 6, 7, 8, 9, 10, 11, 12, 13, 14, 15, 16, 17, 18, 19, or 20.

Preferably, the linker peptide may include LLTPPPPPLFPPPFF (SEQ ID NO: 240), (EAAAK)n (n = 2-5, wherein n = 2, 3, 4, and 5 correspond to SEQ ID NO: 241, 242, 243, and 244, respectively), E₄K₄ (SEQ ID NO: 245), (E₄R₄)₂ (SEQ ID NO: 246), (E₄K₄)₂ (SEQ ID NO: 148), KLYPYDVPDYA (SEQ ID NO: 139), PKPSTPPGSS (SEQ ID NO: 130), GSPAG (SEQ ID NO: 121), GGASPAGG (SEQ ID NO: 113), GGASPAAPAPAG (SEQ ID NO: 106), or AMGPSSGAPGGGGS (SEQ ID NO: 98), or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 98, 106, 113, 121, 130, 139, 148, and 240 to 246.

Two to ten (e.g., 2, 3, 4, 5, 6, 7, 8, 9, or 10) PEG moieties are further present between the small molecule compound and the antibody or antigen-binding fragment thereof. Preferably, 3-5 PEG moieties are further present between the small molecule compound and the antibody or antigen-binding fragment thereof.

Preferably, the small molecule compound is an inhibitor of the target antigen. Such small molecule compound is, for example, PSB12379, 5-(2-(4-(N-(1,2,4-thiadiazol-5-sulfonamido)-2,5-difluorophenoxy)-5-chlorobenzene) benzisoxazole)-3-amine, or ZM241385.

The target antigen may be a target protein, peptide, lipid, polysaccharide, or polynucleotide. Preferably, the target protein is selected from a membrane protein, a membrane transport protein, a receptor protein, a complex formed by a membrane protein and a membrane protein, or a complex formed by a membrane protein and another protein.

More preferably, the membrane protein is a peripheral membrane protein, an integral membrane protein, or a lipid-anchored protein.

More preferably, the membrane transport protein is a carrier protein or a channel protein. Preferably, the channel protein is a sodium ion channel protein, a calcium ion channel protein, a chloride ion channel protein, a potassium ion channel protein, an Ach receptor channel, or an amino acid receptor channel.

More preferably, the receptor protein is an intracellular receptor, a cell surface receptor, an ion channel receptor, a G-protein-coupled receptor (GPCR), or an enzyme-linked receptor.

For example: Naᵥ1.1, Naᵥ1.2, Naᵥ1.3, Naᵥ1.4, Naᵥ1.5, Naᵥ1.6, Naᵥ1.7, Naᵥ1.8, Naᵥ1.9, CD73, VEGFA, VEGFB, VEGFC, VEGFD, VEGFR, FGF, FGFR, P1GF, PDGF, ANG2, Endoglin (CD105), TGF, Integrin, Integrin receptor, IL-1β, IL-2, IL-3, IL-4, IL-6, IL-10, IL-12, IL-15, IL-17, IL-23, IL1R1, IL2Rα, IL3R, IL4Rα, IL6R, IL10R, IL12R, IL15Rα, IL17R, IL23R, PCSK9, TNF-α, TNFR, RANKL, complement protein C3, complement protein C5, GPCR, GLP1R, CD3, CD19, CD20, CD22, CD25, CD27, CD28, CD30, CD33, CD38, CD40, CD47, CD80, CD86, CD96, CD99, CD111, CD112, CD123, CD133, CD138, CD155, CD171, Claudin 18.2, OX40, ICOS, CTLA4, 4-1BB, TCR, B7-1, B7-2, BTLA, TIM-3, LAG3, Galectin-9, PD-L1, PD-L2, PD-1, TIGIT, EGFR, Her2, PSCA, CEA, FAP, EGFRVIII, BCMA, PSMA, CA125, EphA2, C-met, L1CAM, CS1, ROR1, EC, NY-ESO-1, MUC1, MUC16, mesothelin, LewisY, GPC3, GD2, EPG, DLL 3, A2aR, or 5T4.

The antibody or antigen-binding fragment thereof may be Fab, Fv, Fd, Fab', Fab'-SH, F(ab')2, a nanobody, a single-domain antibody, or a single-chain antibody.

In a specific embodiment of the present disclosure, the method for constructing a smacAb library includes:
A) acquiring CDR library fragments and assembling the CDR library fragments at corresponding positions of a framework antibody to obtain library phagemids;
B) introducing the library phagemids into *Escherichia coli* and, under the action of helper phage, displaying the antibody or antigen-binding fragment thereof on the phage surface; and
C) site-specifically conjugating (preferably via a Michael addition reaction or a Huisgen reaction) the displayed antibody or antigen-binding fragment thereof (preferably the CDR region) to a small molecule compound to obtain a smacAb library.

The CDR library fragments are derived from B lymphocytes. Preferably, the B lymphocytes are of human origin.

Said acquiring CDR library fragments includes isolating mononuclear lymphocytes from peripheral blood, extracting RNA, reverse-transcribing the RNA into cDNA as a template, and performing PCR.

The framework antibody is from Fab, Fv, Fd, Fab', Fab'-SH, F(ab')2, a nanobody, a single-domain antibody, a single-chain antibody, IgG, IgA, IgM, IgD, or IgE.

The framework antibody includes a light chain and/or a heavy chain.

The light chain is derived from kappa (κ) or lambda (λ) chain.

The heavy chain is derived from the mu (µ), gamma (γ), alpha (α), delta (δ), or epsilon (ε) chain.

The library phagemid further includes a cleavage sequence inserted at the fusion site between the framework antibody and the p3 protein. Preferably, the cleavage sequence is selected from any one of or a combination of two or more of SEQ ID NO: 1 to SEQ ID NO: 4.

In a seventeenth aspect, the present disclosure provides use of the smacAb library obtained by the above-mentioned method, or the above-mentioned smacAb library in antibody or smacAb screening.

In an eighteenth aspect, the present disclosure provides a method for screening an antibody or antigen-binding fragment thereof or a smacAb. The method includes adding a target antigen to the smacAb library obtained by the above-mentioned method or to the above-mentioned smacAb library, and collecting the antibody or antigen-binding fragment thereof or the smacAb that binds to the target antigen.

The target antigen may be a target antigen expressed on the surface of overexpressing cells, and screening may be performed using cells overexpressing the target antigen.

The collection of the antibody or antigen-binding fragment thereof that binds to the target antigen includes an elution step, which may be performed using an acidic solution (e.g., glycine-HCl buffer, pH 2-3) or an alkaline solution (e.g., 0.1 M triethylamine), or by enzymatic elution.

Preferably, the elution is performed by enzymatic elution, for example by recognizing and cleaving a cleavage site within a sequence.

The cleavage is mediated by thrombin or is sequence-specific nickel-assisted cleavage.

Preferably, the screening method includes one or more rounds of binding, elution, and amplification.

Preferably, the screening method may be a specificity screening method for identifying antibodies that bind to a specific protein subtype, for example, using proteins other than the specific protein subtype as negative selection targets.

Preferably, the screening method may be competitive screening or non-competitive screening.

In a nineteenth aspect, the present disclosure provides an antibody or antigen-binding fragment thereof, or a smacAb, obtained by the above-mentioned screening method.

In a twentieth aspect, the present disclosure provides a method for detecting a target antigen. The method includes bringing the target antigen into contact with the above-mentioned antibody derivative or the above-mentioned antibody or antigen-binding fragment thereof.

In a twenty-first aspect, the present disclosure provides use of the above antibody derivative, the above antibody or antigen-binding fragment thereof, the above nucleic acid molecule, the above vector, the above host cell or immune cell, or the above smacAb library in the manufacture of a product for diagnosing and/or treating a disease.

The disease is selected from a tumor, inflammation, and an infectious disease.

In a twenty-second aspect, the present disclosure provides a medicament or diagnostic kit, including the above antibody derivative, the above antibody or antigen-binding fragment thereof, the above nucleic acid molecule, the above vector, the above chimeric antigen receptor, the above host cell or immune cell, or the above smacAb.

In a twenty-third aspect, the present disclosure provides a method for treating a disease, the method including administering the above antibody derivative, the above antibody or antigen-binding fragment thereof, the above nucleic acid molecule, the above vector, the above chimeric antigen receptor, the above host cell or the above immune cell, or the above smacAb.

Preferably, the method includes administering an effective amount of the above antibody derivative, the above antibody or antigen-binding fragment thereof, the above nucleic acid molecule, the above vector, the above chimeric antigen receptor, the above host cell or the above immune cell, or the above smacAb to a subject in need thereof.

The disease may be a tumor, an infectious disease, or inflammation.

As used herein, the term "antibody or antigen-binding fragment thereof" refers to a structure conventionally similar to Fab, Fv, Fd, Fab', Fab'-SH, F(ab')2, a nanobody, a single-domain antibody, a single-chain antibody, or full-length antibody, and does not imply high or low binding strength to the target antigen, and such an antibody or an antigen-binding fragment may bind to the target antigen with high, very high, low, very low, or even no affinity. For example, the smacAb library constructed according to the present disclosure inevitably contains structures conventionally similar to Fab, Fv, Fd, Fab', Fab'SH, F(ab')2, nanobody, single-domain antibody, single-chain antibody, or full-length antibody exhibiting high, low, or no binding strength. For another example, the antibody derivative obtained by screening from the smacAb library inevitably exhibits very strong binding ability to the target antigen; however, this may be attributable to the small molecule compound, the antibody or antigen-binding fragment thereof, or more likely to a synergistic effect arising from conjugation of the small molecule compound to the antibody or antigen-binding fragment thereof.

As used herein, the term "antigen-binding fragment" refers to a portion of an antibody that retains the antibody's specific binding activity, i.e., any portion capable of specifically binding to an epitope on the antibody's target molecule. It includes, for example, Fab, Fv, Fd, Fab', Fab'-SH, F(ab')2, and variants thereof. For example, such antigen-binding fragment includes a heavy chain and/or light chain of an antibody; a heavy chain variable region and/or light chain variable region of an antibody; or one or more (e.g., two or more) CDRs derived from the heavy chain or light chain of an antibody. Among them, Fab is a monovalent fragment consisting of the VL, VH, CL, and CH1 domains. F(ab')2 is a bivalent fragment including two Fab fragments linked by disulfide bridge in the hinge region. Fd is a is an Fd fragment consisting of the VH and CH1 domains. Fv is an Fv fragment consisting of the VL and VH domains of one arm of an antibody. Fab' is a Fab fragment having one or more cysteine residues at the C-terminus of the CH1 domain. Fab'-SH is Fab' in which at least one cysteine residue in the constant domain carries a free thiol group. Herein, VH represents the heavy chain variable region, VL represents the light chain variable region, CL represents the light chain constant region, and CH1 is the CH1 domain of the heavy chain constant region.

As used herein, the term "unnatural amino acid" refers to a compound containing an amino group and a carboxyl group that is not naturally found in proteins. Preferably, the unnatural amino acid is any unnatural amino acid known in the art. More preferably, the unnatural amino acids include, but are not limited to, N-ethylasparagine, hydroxylysine, 3-hydroxyproline, 2-aminobutyric acid, β-alanine, β-aminopropionic acid, 2-aminoadipic acid, 3-aminoadipic acid, 4-aminobutyric acid, 6-aminocaproic acid, 2-aminoheptanoic acid, allo-isoleucine, isodesmosine, 4-hydroxyproline, allo-hydroxylysine, 2-aminoisobutyric acid, N-methylglycine, N-methylisoleucine, 3-aminoisobutyric acid, 6-N-methyllysine, 2,4-diaminobutyric acid, N-methylvaline, ornithine, norleucine, norvaline, desmosine, 2,2'-diaminopimelic acid, 2,3-diaminopropionic acid, N-ethylglycine, or 2-aminopimelic acid. Of course, the "unnatural amino acid" as described herein may also be a derivative obtained by modification of a natural amino acid.

As used herein, the term "method" may be for diagnostic or therapeutic purposes relating to diseases, or for non-disease diagnostic or non-disease treatment purposes.

As used herein, the term "infectious disease" is selected from bacterial infection, viral infection, or fungal infection; more preferably viral infection, pneumonia with septic shock, peritonitis, bacteremia, sepsis, or septicemia. The viral infection is selected from acute viral infection or chronic viral infection, preferably infection with influenza virus, parainfluenza virus, herpesvirus (e.g., HSV-1, EBV), measles virus, vesicular stomatitis virus, hepatitis B virus, hepatitis C virus, human immunodeficiency virus, lymphocytic choriomeningitis virus, or human papillomavirus.

As used herein, the term "inflammation" may occur in any tissue, including, but not limited to, adrenal gland, adrenal medulla, anus, appendix, bladder, blood, bone, bone marrow, brain, breast, cecum, central nervous system (including or excluding the brain), cerebellum, cervix, colon, duodenum, endometrium, epithelial cells (e.g., renal epithelial cells), gallbladder, esophagus, glial cells, heart, ileum, jejunum, kidney, lacrimal gland, larynx, liver, lung, lymph, lymph nodes, lymphoblasts, maxilla, mediastinum, mesentery, myometrium, nasopharynx, omentum, oral cavity, ovary, pancreas, parotid gland, peripheral nervous system, peritoneum, pleura, prostate, salivary gland, sigmoid colon, skin, small intestine, soft tissue, spleen, stomach, testis, thymus, thyroid, tongue, tonsils, trachea, uterus, vulva, or white blood cells. More preferably, inflammation is selected from systemic lupus erythematosus, rheumatoid arthritis, psoriatic arthritis, scleroderma, asthma, atopic dermatitis, organ-specific inflammatory diseases, allergies (e.g., allergic rhinitis), folliculitis, tonsillitis, pneumonia, hepatitis, nephritis, acne, autoimmune diseases, chronic prostatitis, glomerulonephritis, hypersensitivity reactions, colitis, inflammatory bowel disease, pelvic inflammatory disease, reperfusion injury, transplant rejection, vasculitis, or interstitial cystitis.

As used herein, the term "tumor" refers to any undesirable cell proliferation (or any disease characterized by undesirable cell proliferation), neoplasm, or an increased predisposition to or risk of undesirable cell proliferation, neoplasm, or tumor. It may be benign or malignant, and may be primary or secondary (metastatic). Neoplasm refers to any abnormal growth or proliferation of cells and may occur in any tissue. Examples of tissues include adrenal gland, adrenal medulla, anus, appendix, bladder, blood, bone, bone marrow, brain, breast, cecum, central nervous system (including or excluding the brain), cerebellum, cervix, colon, duodenum, endometrium, epithelial cells (e.g., renal epithelial cells), gallbladder, esophagus, glial cells, heart, ileum, jejunum, kidney, lacrimal gland, larynx, liver, lung, lymph, lymph nodes, lymphoblasts, maxilla, mediastinum, mesentery, myometrium, nasopharynx, omentum, oral cavity, ovary, pancreas, parotid gland, peripheral nervous system, peritoneum, pleura, prostate, salivary gland, sigmoid colon, skin, small intestine, soft tissue, spleen, stomach, testis, thymus, thyroid, tongue, tonsils, trachea, uterus, vulva, or white blood cells. More preferably, the tumor is selected from prostate cancer, breast cancer, liver cancer, glioma (e.g., neuroglioma), colorectal cancer, cervical cancer, non-small cell lung cancer, lung cancer, pancreatic cancer, gastric cancer, bladder cancer, skin cancer, rhabdomyosarcoma, tongue squamous cell carcinoma, nasopharyngeal carcinoma, ovarian cancer, choriocarcinoma, lymphoma (e.g., non-Hodgkin lymphoma, Hodgkin lymphoma, cutaneous T-cell lymphoma), leukemia, rectal adenocarcinoma, medulloblastoma, meningioma, neurofibroma (e.g., neurofibrosarcoma), ependymoma, schwannoma, astrocytoma, melanoma, mesothelioma, myeloma, chronic myeloid leukemia, acute myeloid leukemia, myelodysplastic syndrome, chronic lymphocytic leukemia, epidermoid carcinoma, colon cancer, thymic carcinoma, hematologic malignancies, head and neck cancer, or oropharyngeal cancer.

As used herein, the term "subject" refers to a human or non-human mammal, or a cell, organ, or tissue of a human or non-human mammal. The non-human mammal includes, but is not limited to, wild animals, zoo animals, livestock, companion animals, or laboratory animals. Preferably, the non-human mammal includes, but is not limited to, pig, cattle, sheep/goat, horse, donkey, fox, raccoon dog, mink, camel, dog, cat, rabbit, rodent (e.g., rat, mouse, guinea pig, hamster, gerbil, chinchilla, squirrel), or monkey.

As used herein, the term "effective amount" refers to an amount or dose of a medicament of the present disclosure that provides a desired therapeutic or preventive effect after administration in one or more doses to an individual or organ.

As used herein, the term "treat" or "treatment" refers to slowing, interrupting, arresting, controlling, stopping, alleviating, or reversing the progression or severity of a sign, symptom, disorder, condition, or disease after the disease has begun to develop, but does not necessarily entail complete elimination of all disease-related signs, symptoms, conditions, or disorders.

As used herein, the term "diagnose" refers to determining whether a patient has had, currently has, or will develop a disease or disorder, or determining the current progression of a disease or its potential future progression.

As used herein, the terms "comprise" or "include" are open-ended and, when used to describe a protein or nucleic acid sequence, indicate that the protein or nucleic acid may consist of the recited sequence or may include additional amino acids or nucleotides at one or both ends, while retaining the same or similar activity as the original sequence.

As used herein, the term "identity" refers to sequence identity or homology between a sequence used and a sequence obtained from the prior art, which may be adjusted by a person skilled in the art according to practical needs, and may exhibit (including but not limited to) at least 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 70%, 80%, 81%, 82%, 83%, 84%, 85%, 86%, 87%, 88%, 89%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, or 99.9% identity. To determine the percentage identity between two amino acid or nucleotide sequences, the sequences are aligned for optimal comparison (e.g., gaps may be introduced into one or both of the first and second amino acid or nucleic acid sequences to achieve optimal alignment, and non-homologous regions may be ignored for comparison). The amino acid residues or nucleotides at corresponding positions in the aligned sequences are then compared. A position is considered identical when the same amino acid residue or nucleotide occupies the corresponding position in both sequences. The percentage identity is determined as a function of the number of identical positions shared between the sequences, taking into account the number and length of gaps required for optimal alignment. For example, sequence comparison and determination of percentage identity may be performed using a BLOSUM62 scoring matrix with a gap penalty of 12, a gap extension penalty of 4, and a frameshift gap penalty of 5.

Beneficial Effects:
1. The conjugation of a small molecule compound to the CDR3 region enables facile antigen access and full exposure of the site, without compromising antibody expression levels or affinity. The conjugation is specific, preserves diversity within the smacAb library, and maintains a library capacity of about 10⁹.
2. The insertion of a cleavage sequence at the fusion site between herFab and the p3 protein enables specificity-based elution and highly efficient elution without impairing phage infectivity.
3. The smacAb prepared herein exhibits higher affinity, inhibitory activity, and specificity than either the small molecule compound or antibody alone, and binding of the small molecule compound to the target binding pocket enhances the antibody's affinity and compensates for epitope insufficiency.
4. The use of the library designed in the present application increases the probability of acquiring functional antibodies.
5. Through competitive inhibition screening, specific binding to target protein subtypes is performed to reverse the preference of ASF small molecules for NaV1.7, thereby enabling screening of smacAbs that specifically bind NaV1.7, NaV1.2, or NaV1.6.

### BRIEF DESCRIPTION OF DRAWINGS

Hereinafter, embodiments of the present disclosure will be described in detail with reference to the accompanying drawings.

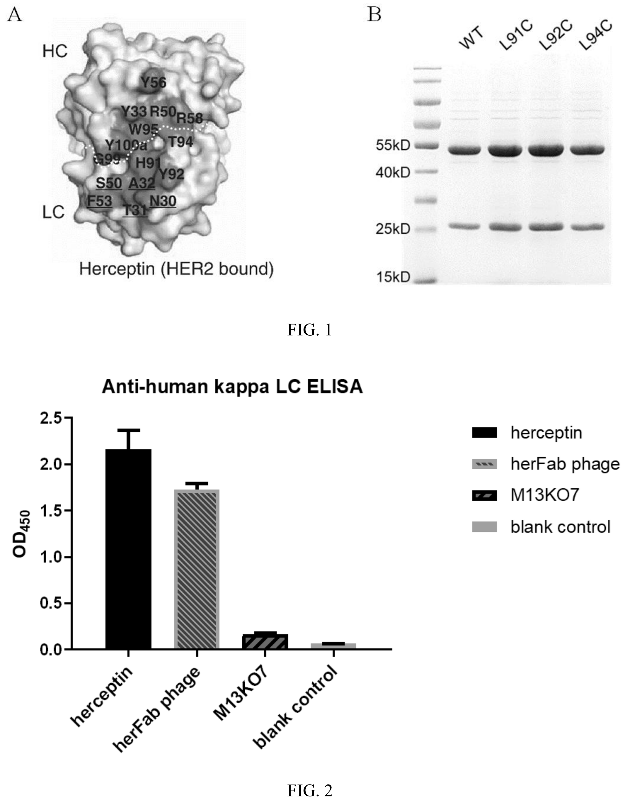

FIG. 1 shows the selection of site-directed mutation sites of Herceptin, wherein FIG. 1A shows the surface amino acid sites of Herceptin colored according to their degree of burial in the Herceptin-HER2 complex (red: >75%; orange: >50-75%; yellow: >25-50%), and the upper portion delineated by dashed lines represents heavy chain sites and the lower portion represents light chain amino acid sites; FIG. 1B shows reducing SDS-PAGE gel electrophoresis of Herceptin antibodies in which light chain residues 91, 92, and 94 are site-directed mutated to Cys, followed by Coomassie brilliant blue staining to visualize protein bands.
FIG. 2 shows phage ELISA detection of herFab expression.
FIG. 3 schematically shows the reaction between herFab phage and Cy5 detection molecule, wherein the upper panel shows a Michael addition reaction and the lower panel shows a click reaction (Huisgen cycloaddition).
FIG. 4 shows conjugation reaction between the site-directed mutated herFab phage and the corresponding Cy5 detection molecule, wherein FIG. 4A shows the chemical structure of UAA, and FIG. 4B shows specificity detection of the conjugation reaction between herFab-UAA and Cy5-DBCO, with Ctrl representing the wild-type herFab phage.
FIG. 5 shows a schematic workflow for evaluating elution efficiency of herFab phage in which four different cleavage sequences are inserted at the fusion site between herFab and the p3 protein.
FIG. 6 shows detection of elution efficiency of herFab phage containing four different cleavage sequences after binding to HER2 antigen coated on an ELISA plate and elution under different cleavage conditions, wherein FIG. 6A shows detection of residual phage remaining in ELISA wells after elution by adding HRP-labeled anti-M13 antibody, with data points presented as mean ± SD, n = 3; FIG. 6B shows infection of TG1 bacterial culture with eluted phage, followed by plating and overnight incubation for colony counting and calculation of eluted phage titers.
FIG. 7 shows PCR amplification of CDR fragment libraries and preparation of ssDNA, wherein FIG. 7A schematically shows PCR primer design, with primers located at corresponding positions of the herFab phagemid; FIG. 7B shows agarose gel electrophoresis identification of PCR-amplified CDR fragment libraries; FIG. 7C shows the principle of ssDNA preparation, where dsDNA products labeled with biotin on one side bind to streptavidin magnetic beads, and NaOH solution is used to denature the DNA duplex, releasing the non-biotin-labeled ssDNA into solution.
FIG. 8 shows detection of overlap PCR products of Fab library fragments, where HC represents heavy chain, LC represents light chain, and DNA ladder represents DNA fragments.
FIG. 9 shows clone sequencing results of the Fab-UAA library phagemid.
FIG. 10 shows conjugation efficiency between Fab-UAA library phage and Cy5-DBCO, where the Ctrl group represents wild-type herFab phage.
FIG. 11 shows a workflow for screening CD73-targeting smacAbs from a Fab-BN3 library.
FIG. 12 shows identification of CD73 protein, wherein FIG. 12A shows identification of CD73 purity by reducing SDS-PAGE followed by Coomassie Brilliant Blue staining, and FIG. 12B shows identification of CD73 purity by size-exclusion chromatography (SEC).
FIG. 13 shows inhibitory curves of small molecules PSB12379 (left panel) and BN3 (right panel) against CD73.
FIG. 14 shows clonal sequence analysis and clone selection of CD73 antibodies.
FIG. 15 shows inhibitory activity of 12.5 nM Ab-BN3 against CD73, wherein FIG. 15A shows a schematic structure of Ab-BN3, and FIG. 15B shows detection of phosphate concentration in reaction products after incubating CD73 with 12.5 nM Ab-BN3 followed by addition of AMP substrate.
FIG. 16 shows the dose-response curve of Ab-BN3 inhibition of CD73.
FIG. 17 shows detection of inhibitory activity of Fab-BN3 against CD73, wherein FIG. 17A shows a schematic structure of Fab-BN3, and FIG. 17B shows phosphate concentration in reaction products detected by malachite green phosphate assay after pre-incubation of CD73 with different concentrations of Fab-BN3 followed by addition of AMP substrate, with data points presented as mean ± SD, n = 2, and curve fitting performed using GraphPad.
FIG. 18 shows a workflow for screening the Fab-ASF library based on overexpressing cell lines.
FIG. 19 shows chemical structures of ASF and ASF-PEG-DBCO.
FIG. 20 shows inhibitory activity of ASF-PEG-DBCO molecules with different PEG chain lengths against NaV1.7.
FIG. 21 shows clonal sequencing analysis of NaV1.7 library screening.
FIG. 22 shows the inhibitory curve of Ab-PEG3-ASF against NaV1.7.
FIG. 23 shows inhibitory activity of NaV1.7-selective Ab-ASF against NaV1.6.
FIG. 24 shows inhibitory activity of NaV1.7-inhibitory Ab-ASF against NaV1.2.
FIG. 25 shows clone enrichment statistics of competitive NaV1.7 library screening.
FIG. 26 shows inhibitory activity of NaV1.7-selective Ab-ASF, wherein FIG. 26A shows inhibitory activity of 10 nM Ab-PEG3-ASF against NaV1.7, and FIG. 26B shows the dose-response curve of Ab-PEG3-ASF inhibition of NaV1.7, with data points presented as mean ± SD, n = 2-4, and curve fitting performed using GraphPad.
FIG. 27 shows selectivity testing of NaV1.7-targeting Ab-ASF.
FIG. 28 shows inhibitory activity of ASF-PEG-DBCO against NaV1.2 and NaV1.6. Specifically, HEK293-NaV1.2 or HEK293-NaV1.6 cells are treated with different concentrations of ASF-PEG-DBCO molecules, and inhibitory activity is evaluated using whole-cell patch-clamp techniques, with data presented as mean ± SD, n = 2-3.
FIG. 29 shows clone enrichment statistics of non-competitive NaV1.2 library screening.
FIG. 30 shows clone enrichment statistics of competitive NaV1.2 library screening.
FIG. 31 shows inhibitory activity of NaV1.2-selective Ab-ASF, wherein FIG. 31A shows inhibitory activity of 10 nM and 100 nM Ab-ASF against NaV1.2, and FIG. 31B shows dose-response curves of the two most potent Ab-PEG3-ASF molecules inhibiting NaV1.2.
FIG. 32 shows selectivity testing of NaV1.2-targeting Ab-ASF.
FIG. 33 shows clone enrichment statistics of NaV1.6 library screening.
FIG. 34 shows clone enrichment statistics of competitive NaV1.6 library screening.
FIG. 35 shows inhibitory activity testing of Ab-ASF against NaV1.6 after incubating HEK293-NaV1.6 cells with different concentrations of Ab-ASF at room temperature for 15 minutes, wherein FIG. 35A shows inhibitory activity of 10 nM and 100 nM Ab-ASF against NaV1.6, and FIG. 35B shows dose-response curves of the two most potent Ab-PEG3-ASF molecules inhibiting NaV1.6, with data points presented as mean ± SD, n = 2-4, and curve fitting performed using GraphPad.
FIG. 36 shows selectivity testing of 5A5-PEG3-ASF.
FIG. 37 shows activity testing results of H3-PEG3-ASF_{Mal}.
FIG. 38 shows inhibitory activity testing of ZP3.
FIG. 39 shows activity testing of Ab-ZP3.

### DETAILED DESCRIPTION

The technical solutions in the embodiments of the present disclosure will now be described more clearly and completely below in conjunction with the accompanying drawings of the embodiments of the present invention. Apparently, the described embodiments are only some embodiments of the present disclosure, rather than all embodiments thereof. Based on the embodiments of the present disclosure, all other embodiments obtained by a person of ordinary skill in the art without making creative efforts shall fall within the scope of protection of the present disclosure.

### Example 1: Preparation of Antibody Derivatives (smacAb)

### 1. Selection of Conjugation Site for Small Molecule Compound

An active small molecule compound was site-specifically conjugated to the light chain CDR3 of an antibody, enabling it to function as part of the antibody to bind an antigen and exert biological activity. Taking the Herceptin-HER2 complex (see FIG. 1A) as an example, in this example, the amino acid residue 91 of the Herceptin light chain CDR3, exhibiting the highest degree of surface burial, was selected, followed by residues 92 and 94. First, the amino acids at these three positions in the Herceptin light chain were mutated to cysteine (Cys), and the effect of these site-directed mutations on protein expression was observed.

The results are shown in FIG. 1B that all three site-directed mutant antibodies were successfully expressed, with no significant differences in expression levels. The amino acid residue 91 of the light chain was selected as the small-molecule conjugation site within the light chain CDR3 for subsequent experiments.

### 2. Preparation of herFab Phage

Herceptin (trastuzumab), a humanized monoclonal antibody, was approved by the FDA in 1998 for the treatment of HER2-overexpressing breast cancer and was widely used in both clinical therapy and scientific research. The framework of the Herceptin light chain variable region is derived from human kappa light chain variable region subgroup I (Human VL kappa subgroup I), the most abundant subgroup among kappa light chains; and the framework of the heavy chain variable region is derived from human heavy chain variable region subgroup III (Human VH subgroup III), the most abundant subgroup among heavy chains.

The herFab phagemid was introduced into *Escherichia coli* TG1, and phage-displayed herFab was prepared with the aid of M13KO7 helper phage. Phage ELISA experiment was performed to evaluate the display of herFab on the phage surface. Specifically, 0.5 µg/mL Herceptin antibody and each phage (diluted to OD₂₆₈ = 1.0) were coated onto ELISA plates. An HRP-labeled anti-human kappa LC antibody was added for binding, followed by addition of the HRP substrate TMB for color development, and the OD450 value was measured using a microplate reader. Data points are presented as mean ± SD, n = 3. The results are shown in FIG. 2 that herFab was successfully displayed on the phage surface.

### 3. Conjugating Method

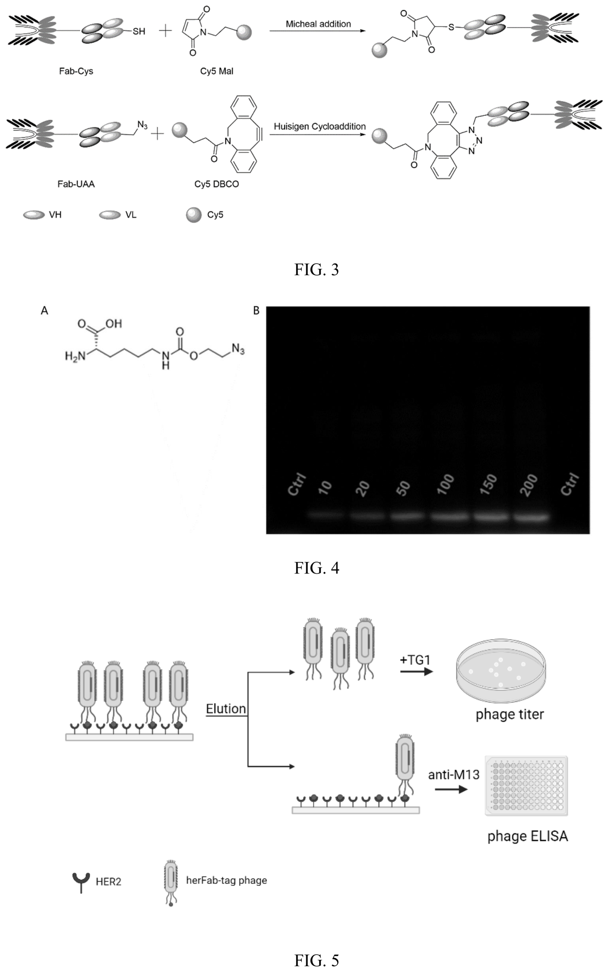

According to the design of smacAb, the small molecule compound was ensured to be specifically conjugated to the antibody mutation site without reacting with the remaining portions of the antibody or the phage coat proteins, thereby reducing the adverse effects of non-specifically conjugated small molecules on library screening. In this example, two specific conjugation strategies were selected. One is a Michael addition reaction between the thiol group of cysteine and a maleimide terminal group (see the upper panel of FIG. 3). The other is a click reaction between an azide group of an unnatural amino acid (UAA) and a DBCO group (Huisgen cycloaddition; see the lower panel of FIG. 3). To confirm the specificity of the conjugating reactions, the amino acid at the mutant site of the phage-displayed herFab was individually mutated to Cys or to a UAA (structures shown in FIG. 4A), and site-directed mutant herFab phages were prepared. The specificity of conjugating between the two conjugating reactions and the corresponding herFab-phages was then detected using Cy5-Mal and Cy5-DBCO, respectively.

The conjugating reactions were performed at room temperature for 3 h. The products were subjected to reducing SDS-PAGE gel electrophoresis, and the Cy5 bands were detected using Typhoon.

The detection results of the conjugation products obtained by reacting herFab-UAA phage with Cy5-DBCO at different molar ratios are shown in FIG. 4B. The control group (Ctrl) was wild-type herFab phage, in which no obvious band appeared after the reaction. A single band was observed at the light chain position for the herFab-UAA phage, indicating the occurrence of specific conjugation. As the molar ratio of Cy5-DBCO increased (up to 150-fold), the Cy5 band intensity gradually increased, indicating improved conjugation efficiency. No significant difference was observed between the 150-fold and 200-fold groups, indicating that the reaction was essentially saturated.

### Example 2: Optimization of Elution Method

In phage-display antibody library screening, acidic solutions (e.g., glycine-HCl buffer, pH 2-3) or basic solutions (e.g., 0.1 M triethylamine) are commonly used for elution. These elution methods dissociate antigen-antibody complexes, thereby releasing antigen-binding antibody-phage into the elution solution. Although acidic and alkaline elution methods are widely used in phage-display antibody library screening, stronger antigen-antibody binding makes dissociation more difficult, which is unfavorable for the elution of high-affinity antibodies. In addition, such elution conditions may adversely affect phage activity. Therefore, in this example, a cleavage sequence was inserted at the fusion junction between herFab and the p3 protein, and phage elution was achieved through specific cleavage at this sequence.

Two cleavage strategies were selected as phage elution methods, and their respective elution efficiencies were evaluated. The first strategy employed thrombin, which specifically recognizes the sequence LVPRGSG (SEQ ID NO: 1) and cleaves between R and G. The second strategy was sequence-specific nickel-assisted cleavage (SNAC), which is a Ni-ion-assisted chemical cleavage method (Dang et al., 2019). Three SNAC sequences were selected and designated as SNAC1, SNAC2, and SNAC3, with cleavage sequences NSLGSQHQAQ (SEQ ID NO: 2), GGSHHTDLPGGS (SEQ ID NO: 3), and PGSHHWG (SEQ ID NO: 4), respectively. The SNAC cleavage site is located between the first GS in the sequence.

The ability of these four cleavage sequences to achieve efficient phage elution was evaluated. The overall workflow is shown in FIG. 5. In the experiment, HER2 antigen was coated onto ELISA plates. After blocking, the four types of herFab phages were added for binding and thoroughly washed to remove unbound phage. Elution was then carried out at 37°C for different cleavage times. The eluted phages were collected and used to infect TG1 bacterial cultures to determine phage titers, and phage ELISA was performed to detect residual phage remaining on the ELISA plates.

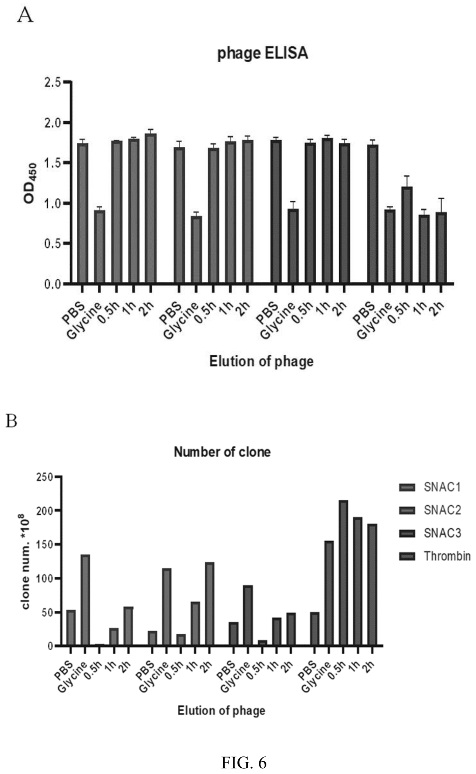

The Phage ELISA results are shown in FIG. 6A. All four types of herFab phage, including distinct cleavage sequences, were able to bind HER2, and the OD value of the positive control glycine elution group decreased significantly, indicating that a portion of the phage had been dissociated into the elution solution. The three SNAC sequence phages exhibited similar elution results: their OD values at 0.5 h, 1 h, and 2 h showed no significant differences and were comparable to those of the negative control PBS group, indicating that the cleavage efficiencies of the three SNAC sequences were relatively low. In contrast, the thrombin cleavage sequence phage showed better elution performance. The OD value of the 0.5 h cleavage group was slightly higher than that of the positive control glycine group, while when the cleavage time was extended to 1 h, its OD value was comparable to that of the glycine group. The OD values of the 1 h and 2 h cleavage groups were similar, indicating that cleavage was essentially completed within 1 h. The activity test results of the eluted phages are shown in FIG. 6B. The numbers of clones obtained from the three thrombin cleavage time groups were all higher than those from the glycine group, suggesting that controlling the cleavage time within 1 h may achieve optimal cleavage efficiency.

Based on a comprehensive evaluation of the phage elution efficiencies across the four cleavage sequences, the thrombin cleavage sequence was selected for subsequent use. This cleavage sequence enables separation of herFab from phage under mild conditions, with a cleavage efficiency comparable to that of glycine elution. In addition, compared with glycine elution, thrombin cleavage has a smaller impact on phage activity, which is advantageous for maintaining phage viability.

### Example 3: Fab-UAA Library Construction

The construction of the Fab-UAA library mainly includes three steps: (1) obtaining a fragment library of CDRs, wherein during library construction the CDR containing the mutation site, i.e., the light-chain CDR3, is kept unchanged, and the diversity of the antibody library is achieved through fragment libraries of the remaining five CDRs; (2) assembling the CDR fragment libraries into corresponding positions of a framework antibody to prepare Fab-UAA library phagemids; and (3) displaying the Fab-UAA library on the phage surface and re-verifying the specific conjugation between Fab-UAA and Cy5-DBCO.
1. Herceptin was used as the framework antibody for Fab library construction (see "2. Preparation of herFab Phage" in Example 1).
2. Acquisition of CDR Library Fragments

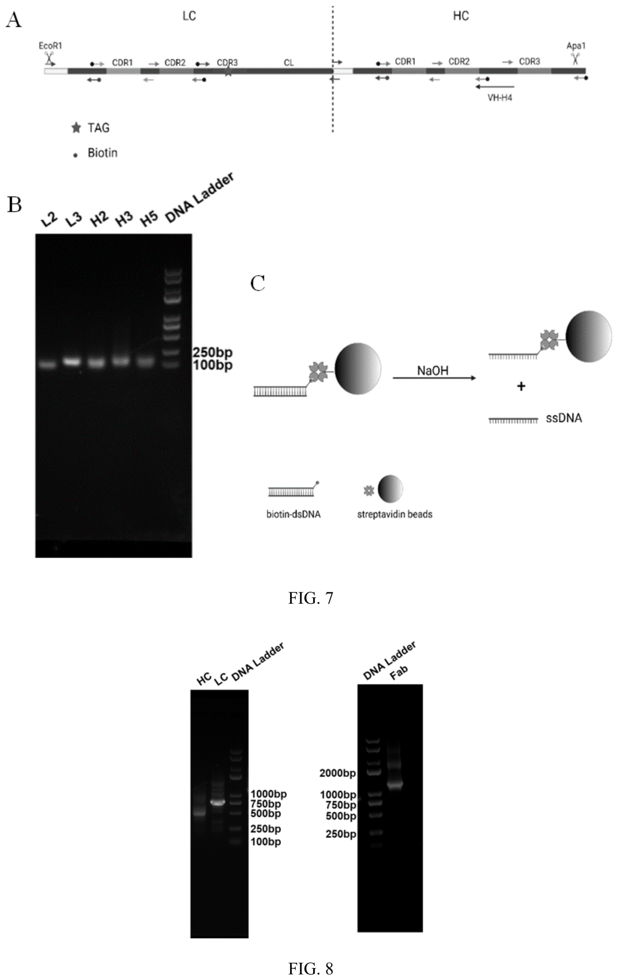

Human B lymphocyte genes were used as the source of CDR library fragments. In the experiment, peripheral blood mononuclear lymphocytes were isolated from the peripheral blood of five healthy people, and RNA was extracted and reverse-transcribed into cDNA. The cDNA was used as a template to obtain CDR library fragments by PCR amplification. The design of PCR primers is shown in FIG. 7A. Using the herFab-UAA phagemid as a vector, one-sided biotin-labeled primers were designed in the framework region (FR) of the antibody to perform PCR amplification to obtain the corresponding DNA fragments. Homologous sequences were designed at the junctions of adjacent fragments to facilitate subsequent assembly of the Fab sequence by overlap PCR. Five fragments containing CDRs were amplified by PCR using cDNA as a template. The agarose gel electrophoresis identification results of the PCR products are shown in FIG. 7B, wherein light chain CDR1 and CDR2 were located in products L2 and L3, respectively, and heavy chain CDR1, CDR2, and CDR3 were located in products H2, H3, and H5, respectively. The lengths of these product fragment range from 100 bp to 250 bp, consistent with the expected lengths. The remaining fragments were amplified by PCR using the herFab-UAA phagemid as a template.

To assemble the CDR library fragments into Fab-UAA library fragments, multi-fragment overlap PCR was employed to separately assemble the heavy chain and light chain sequences. To enable successful multi-fragment overlap PCR, each PCR fragment was prepared as single-stranded DNA (ssDNA). The preparation principle of ssDNA is shown in FIG. 7C. As the primers were designed with one-sided biotin label, each PCR product was bound to streptavidin magnetic beads, followed by dissociation of the DNA double strands using NaOH solution. The ssDNA strand without biotin labeling was released into the solution and recovered by purification, thereby obtaining ssDNA fragments for subsequent multi-fragment overlap PCR assembly experiment.

### 3. Assembly of Fab-UAA Library Phagemids

In the experiments, a two-step overlap PCR method was used to assemble the ssDNA fragments into Fab library fragments. First, the light chain and heavy chain were separately assembled into full-length chains by overlap PCR. The ssDNA fragments for the light chain included L1, L2, L3, and L4, and the expected size of the overlap PCR product was approximately 800 bp. The ssDNA fragments for the heavy chain included H1, H2, H3, H5, and an artificially synthesized framework ssDNA fragment VH-H4, and the expected size of the overlap PCR product was approximately 500 bp. The PCR products were identified by agarose gel electrophoresis, as shown in FIG. 8, and nucleic acid bands were observed under ultraviolet light. The positions of the light chain and heavy chain bands were consistent with the expected sizes, and thus the PCR products were excised and recovered from the gel. Subsequently, the light chain and heavy chain were assembled into Fab-UAA library fragments. The gel electrophoresis identification results are shown in FIG. 8. The expected length of the PCR product was approximately 1300 bp, and the band position was consistent with the expected length, indicating that the CDR library fragments were successfully assembled into Fab library fragments.

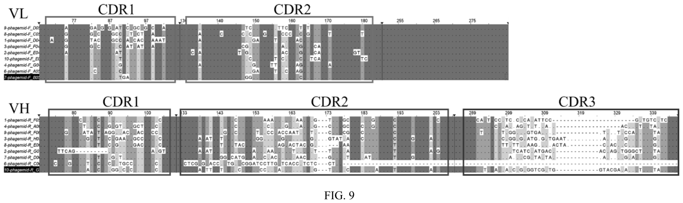

Finally, the Fab-UAA library fragments were cloned into phagemids by restriction digestion and ligation. In the experiment, the Fab library fragments were cloned into the herFab-UAA-thrombin phagemid to construct Fab-UAA library phagemids. To examine the assembly of the Fab-UAA library phagemids, the library phagemids were transformed into *E. coli* TG1, plated, and cultured to obtain single colonies. Ten single colonies were randomly selected for Sanger sequencing to analyze the Fab sequences contained therein. Multiple sequence alignment was performed using the ClustalW Multiple Alignment method. The sequencing results are shown in FIG. 9. Clone No. 5 failed sequencing, and clone No. 6 showed a deletion in the heavy chain. The remaining eight clones were correctly assembled and showed no duplicate clones, indicating that most clones in the library had correctly assembled CDRs and sufficient diversity, confirming successful construction of the Fab-UAA library phagemids.

### 4. Preparation of Fab-UAA Library Phage and Conjugation Assay

To generate a high-capacity phage-displayed Fab-UAA library, the Fab-UAA phagemids were introduced into *Escherichia coli* by electroporation. Since electroporation requires high-purity library phagemids with minimal salt content, the Fab-UAA library phagemids were first purified by column purification and eluted in ultrapure water. Meanwhile, the UAA expression helper plasmid 2104 was transformed into the TG1 strain to prepare TG1-2104 electrocompetent cells. Finally, the purified Fab-UAA library phagemids was electroporated into TG1-2104 electrocompetent cells and plated for colony counting. The calculated library capacity of the Fab-UAA library was approximately 10⁹, indicating successful construction of a high-capacity phage-displayed Fab-UAA library.

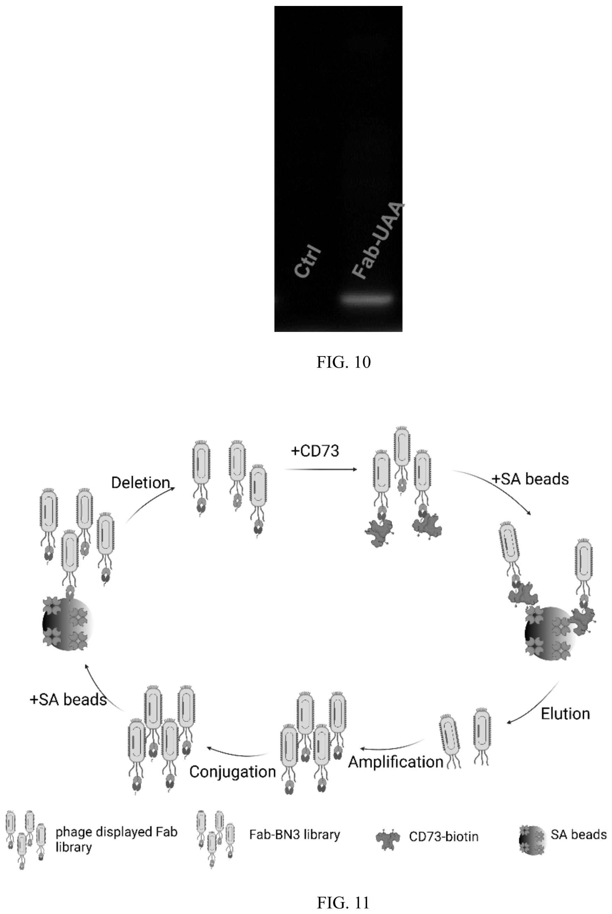

To verify that the Fab-UAA library phage can specifically conjugate with DBCO-modified small molecules, Cy5-DBCO was used to characterize the conjugation efficiency of the Fab-UAA phage. Specifically, Fab-UAA phage was conjugated to a 200-fold molar excess of Cy5-DBCO. The conjugation product was subjected to reducing SDS-PAGE gel electrophoresis, and Cy5-conjugated bands were detected using Typhoon. The Cy5 conjugation product results are shown in FIG. 10. After Fab-UAA phage was conjugated to Cy5-DBCO, a Cy5 band could be observed at the light chain position, similar to the conjugation result of herFab-UAA, demonstrating that the Fab-UAA library phage can achieve specific conjugation with DBCO-modified small molecules.

Thus, a phage-displayed Fab-UAA library with a library capacity of about 10⁹ was successfully constructed. The Fab-UAA library enables specific conjugation between the N₃ group carried by the UAA and target protein-active small molecule compounds bearing a DBCO group, thereby constructing a smacAb library suitable for smacAb screening against target proteins.

### Example 4: Screening and Identification of smacAbs Using CD73 as a Target Protein

A schematic workflow for screening smacAbs targeting CD73 using the Fab-BN3 library is shown in FIG. 11. The specific steps are as follows.

### 1. Expression, Purification, and Identification of CD73 Protein

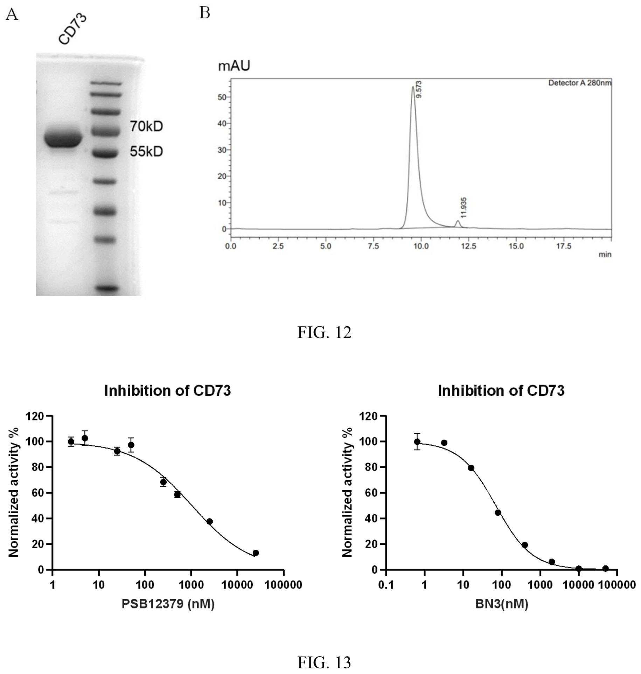

First, the purified CD73 protein antigen was acquired. Specifically, the pcDNA3.4-Chis-CD73 plasmid was transfected into 293 cells for expression. The cell culture supernatant containing CD73 protein was harvested and purified using a nickel-affinity column, and the obtained CD73 was stored at -80°C. The purity of the CD73 protein was identified by reducing SDS-PAGE gel electrophoresis followed by Coomassie Brilliant Blue staining. The theoretical molecular weight of CD73 is 58.8 kD. The electrophoresis results are shown in FIG. 12A that a single band was observed, preliminarily indicating high protein purity. The band molecular weight was 55-70 kD, consistent with the expected molecular weight. Subsequently, the purity of CD73 was further verified by size-exclusion chromatography (SEC). The results are shown in FIG. 12B that a single sharp peak was observed at 9.573 min, and the protein purity reached over 98%, indicating suitability for subsequent experiments.

### 2. Synthesis and Activity Evaluation of the CD73 Small-Molecule Inhibitor BN3

The competitive CD73 small-molecule inhibitor PSB12379 was selected as the parent core scaffold. A flexible PEG3 linker was inserted between PSB12379 and the conjugation group DBCO to synthesize the small-molecule inhibitor BN3.

It was necessary to determine whether the modified small molecule BN3 retained inhibitory activity against CD73. In the experiment, recombinantly expressed CD73 protein was used to evaluate the activity of the small-molecule inhibitors. CD73 protein hydrolyzes AMP to generate adenosine and PO₄³⁻, and PO₄³⁻ can be quantified using the malachite green phosphate assay. Accordingly, CD73 was preincubated with the small-molecule inhibitors, followed by addition of AMP substrate, and the amount of PO₄³⁻ in the reaction product was measured to evaluate inhibitory activity of the small molecules. Specifically, CD73 protein was preincubated with varying concentrations of the small-molecule inhibitors at 37°C for 30 min; AMP substrate was then added and reacted at room temperature for 15 min. Malachite green phosphate assay reagent was then added and incubated for 30 min before measuring OD₆₂₀. Data points are presented as mean ± SD (n = 2), and inhibition curves were fitted using GraphPad software.

In the experiment, the inhibitory activities of the parent compound PSB12379 and the modified compound BN3 were tested. The resulting inhibition curves are shown in FIG. 13, and the IC₅₀ values are summarized in Table 1. PSB12379 exhibited an IC₅₀ of 1040 nM, whereas BN3 showed an IC₅₀ of 70.46 nM. These results indicated that BN3 retained potent inhibitory activity and was suitable for subsequent conjugation and screening experiments.

**Table 1: Inhibitory activity of PSB12379 and BN3**

| Small molecule | IC₅₀ ± SEM (nM) |
|---|---|
| PSB12379 | 1040±120.4 |
| BN3 | 70.46± 4.778 |

### 2. Screening of Phage-Displayed Fab-BN3 Library

### 1) Screening of Fab-BN3 Against CD73

According to the library screening workflow design shown in FIG. 11, the freshly prepared Fab-BN3 library was used for CD73 screening in the experiment. Firstly, negative selection was performed using streptavidin magnetic beads: the Fab-BN3 library was incubated with streptavidin magnetic beads to remove clones that bound to the streptavidin magnetic beads from the Fab-BN3 library. The Fab-BN3 library after negative selection was collected, and CD73-biotin protein was added for binding. Then the CD73-biotin was captured using fresh streptavidin magnetic beads. After multiple washes to remove nonspecifically adsorbed phage, thrombin was added for enzymatic cleavage elution. The eluted phages were collected and used to infect TG1-2104, followed by amplification with the assistance of M13KO7 helper phage to prepare the Fab-BN3 sub-library for the next round of screening. Typically, 3-4 rounds of screening were performed.

### 2) Sequence Analysis and Clone Selection of Fab-BN3

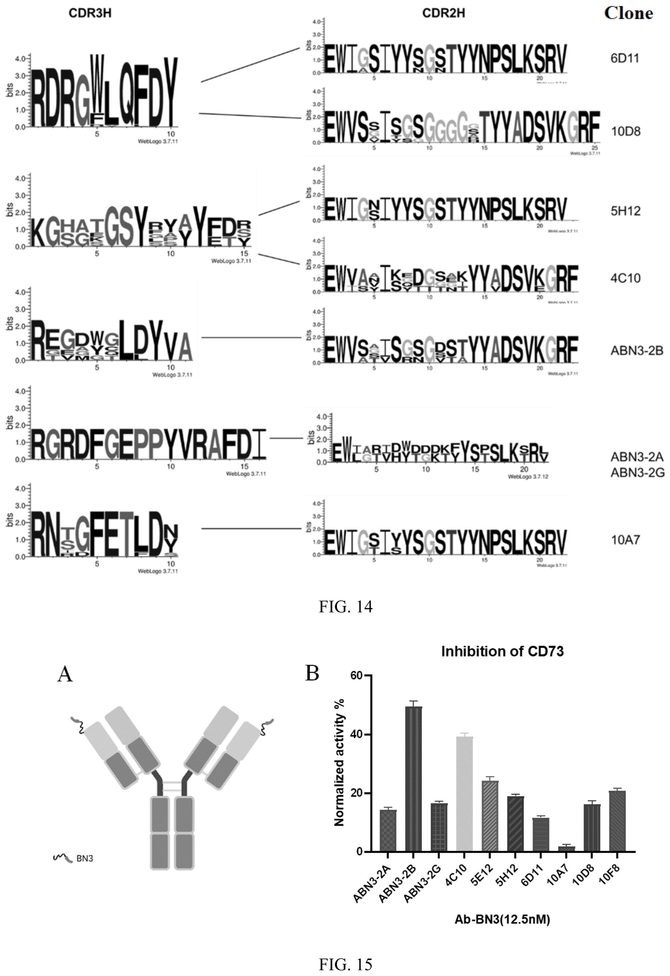

After 3-4 rounds of screening, duplicate clones were identified, necessitating selection of clones for subsequent activity testing. Duplicate clones 10A7 (10A7/10F9 group) and 10D8 (10D8/10G9 group) were first selected. In addition, the VH sequences of the remaining clones were analyzed, prioritizing CDR3 sequences. Multiple sequence alignment was performed to evaluate CDR3 enrichment. The results are shown in FIG. 14. Sequences were grouped into clusters based on CDR3 similarity, resulting in five clusters. Within each cluster, CDR2 sequences were further compared and classified into different subgroups based on sequence similarity, and representative clones from each subgroup were selected for subsequent experiments.

In this experiment, a total of 10 clones, 10A7, 10D8, ABN3-2A, ABN3-2B, ABN3-2G, 4C10, 5E12, 5H12, 6D11, and 10F8 were selected for subsequent expression and activity testing (wherein VL CDR3 sequences are SEQ ID NO: 5 to SEQ ID NO: 29, VL CDR1-2 sequences are listed in Table 2, and VH CDR1-3 sequences are listed in Table 3). Among these, 6D11 and 10D8 originated from the same cluster of CDRH3 sequence but belonged to different CDR2H subgroups; 5H12 and 4C10 also originated from the same cluster of CDRH3 sequence but belonged to different CDR2H subgroups; ABN3-2B and 10A7 originated from a unique CDRH3 cluster and a unique CDR2H subgroups; ABN3-2A and ABN3-2G belonged to the same cluster of CDRH3 sequence and the same CDR2H subgroups; and the remaining clones, 5E12 and 10F8, were clones identified in the sequencing data that showed no significant sequence similarity to any of the above sequences and were randomly selected clones.

**Table 2: VL CDR1-2**

| Antibody No. | VL CDR1 | VL CDR2 |
|---|---|---|
| 10D8 | RASQSISSWLA (SEQ ID NO: 30) | KLLIYDVSNVQTGVPS (SEQ ID NO: 40) |
| 10A7 | RASQGISRWLA (SEQ ID NO: 31) | NLLIYGASTLQSGVPP (SEQ ID NO: 41) |
| 4C10 | RATQAISNYLA (SEQ ID NO: 32) | KLLIYDVSNVQTGVPS (SEQ ID NO: 40) |
| 5E12 | RTSQGITDPLN (SEQ ID NO: 33) | KLLIYKTSSLESGVPS (SEQ ID NO: 42) |
| 5H12 | RASQGLSSWLA (SEQ ID NO: 34) | KLLIYDASNLETGVPS (SEQ ID NO: 43) |
| 6D11 | RASQNIGTYLA (SEQ ID NO: 35) | KLLIYDASNLETGVPS (SEQ ID NO: 43) |
| ABN3-2B | RASQGINNYLA (SEQ ID NO: 36) | KLLIYDASNLETGVPS (SEQ ID NO: 43) |
| ABN3-2G | QASHDISKYLN (SEQ ID NO: 37) | KLLIYDASNLETGVPS (SEQ ID NO: 43) |
| ABN3-2A | RASETIGSWLA (SEQ ID NO: 38) | NLLIYAASTLQSGVSS (SEQ ID NO: 44) |
| 10F8 | QASQDISNYLN (SEQ ID NO: 39) | NLLIYAASTLQSGVPS (SEQ ID NO: 45) |

**Table 3: VH CDR1-3**

| Antibody No. | VH CDR1 | VH CDR2 | VH CDR3 |
|---|---|---|---|
| ABN3-2A | FTLSTYAMH (SEQ ID NO: 46) | WLARIDWDDDKYYSTSLKTRL (SEQ ID NO: 56) | ARGRDFGEPPYVRAF DI (SEQ ID NO: 65) |
| ABN3-2B | FTFSSYWMH (SEQ ID NO: 47) | WVSAVSGSGDSTYYADSVKGRF (SEQ ID NO: 57) | AREGDWGLDY (SEQ ID NO: 66) |
| ABN3-2G | ITFGSYTMS (SEQ ID NO: 48) | WIGTVHYTGKTFYSPSLKSRV (SEQ ID NO: 58) | ARGRDFGEPPYVRAF DI (SEQ ID NO: 65) |
| 4C10 | FTFSSYWMN (SEQ ID NO: 49) | WVANIKQDGSEKYYVDSVKGRF (SEQ ID NO: 59) | VTGSYRYAY (SEQ ID NO: 67) |
| 5E12 | FTFSNYAMH (SEQ ID NO: 50) | WVSAISGSGGSTYYADSVKGRF (SEQ ID NO: 60) | ARGGGNYYDDY (SEQ ID NO: 68) |
| 5H12 | FTFSHYAMH (SEQ ID NO: 51) | WIGNIYYSGSTYYNPSLKSRV (SEQ ID NO: 61) | AKGHGSGSYFPYYFD Y (SEQ ID NO: 69) |
| 6D11 | FTFSGHWMH (SEQ ID NO: 52) | WIGSIYYSGSTYYNPSLKSRV (SEQ ID NO: 62) | ARDRGWLQFDY (SEQ ID NO: 70) |
| 10A7 | FAFSSYSMS (SEQ ID NO: 53) | WIGSIYYSGSTYYNPSLKSRV (SEQ ID NO: 62) | ARNSGFETLDY (SEQ ID NO: 71) |
| 10D8 | FTFSTYAIH (SEQ ID NO: 54) | WVSSISGSGGGGGTYYADSVKG RF (SEQ ID NO: 63) | ARDRGFLQFDY (SEQ ID NO: 72) |
| 10F8 | FTFSSYGMH (SEQ ID NO: 55) | WVAAISYDGSNELYADSVKGRF (SEQ ID NO: 64) | ARDRGWLQFDY (SEQ ID NO: 70) |

The amino acid sequences of the heavy chain variable region and light chain variable region of each antibody are as follows:
>10A7. VH 
EVQLVESGGGLVQPGGSLRLSCAASGFAFSSYSMSWVRQAPGKGLEWIG
SIYYSGSTYYNPSLKSRVTISADTSKNTAYLQMNSLRAEDTAVYYCARNSGFETLDYW
GQGTLVTVSS (SEQ ID NO: 73)
>10D8. VH 
EVQLVESGGGLVQPGGSLRLSCAASGFTFSTYAIHWVRQAPGKGLEWVS
SISGSGGGGGTYYADSVKGRFTISADTSKNTAYLQMNSLRAEDTAVYYCARDRGFLQ
FDYWGQGTLVTVSS (SEQ ID NO: 74)
>ABN3-2A. VH 
EVQLVESGGGLVQPGGSLRLSCAASGFTLSTYAMHWVRQAPGKGLEWL
ARIDWDDDKYYSTSLKTRLTISADTSKNTAYLQMNSLRAEDTAVYYCARGRDFGEPP
YVRAFDIWGQGTLVTVSS (SEQ ID NO: 75)
>ABN3-2B. VH 
EVQLVESGGGLVQPGGSLRLSCAASGFTFSSYWMHWVRQAPGKGLEW
VSAVSGSGDSTYYADSVKGRFTISADTSKNTAYLQMNSLRAEDTAVYYCAREGDWGL
DYWGQGTLVTVSS (SEQ ID NO: 76)
>ABN3-2G. VH 
EVQLVESGGGLVQPGGSLRLSCAASGITFGSYTMSWVRQAPGKGLEWIG
TVHYTGKTFYSPSLKSRVTISADTSKNTAYLQMNSLRAEDTAVYYCARGRDFGEPPYV
RAFDIWGQGTLVTVSS (SEQ ID NO: 77)
>4C10. VH 
EVQLVESGGGLVQPGGSLRLSCAASGFTFSSYWMNWVRQAPGKGLEWV
ANIKQDGSEKYYVDSVKGRFTISADTSKNTAYLQMNSLRAEDTAVYYCVTGSYRYAY
WGQGTLVTVSS (SEQ ID NO: 78)
>5E12. VH 
EVQLVESGGGLVQPGGSLRLSCAASGFTFSNYAMHWVRQAPGKGLEWV
SAISGSGGSTYYADSVKGRFTISADTSKNTAYLQMNSLRAEDTAVYYCARGGGNYYD
DYWGQGTLVTVSS (SEQ ID NO: 79)
>5H12. VH 
EVQLVESGGGLVQPGGSLRLSCAASGFTFSHYAMHWVRQAPGKGLEWI
GNIYYSGSTYYNPSLKSRVTISADTSKNTAYLQMNSLRAEDTAVYYCAKGHGSGSYFP
YYFDYWGQGTLVTVSS (SEQ ID NO: 80)
>6D11. VH 
EVQLVESGGGLVQPGGSLRLSCAASGFTFSGHWMHWVRQAPGKGLEWI
GSIYYSGSTYYNPSLKSRVTISADTSKNTAYLQMNSLRAEDTAVYYCARDRGWLQFD
YWGQGTLVTVSS (SEQ ID NO: 81)
>10F8. VH EVQLVESGGGLVQPGGSLRLSCAASGFTFSSYGMHWVRQAPGKGLEWV 
AAISYDGSNELYADSVKGRFTISADTSKNTAYLQMNSLRAEDTAVYYCARDRGWLQF
DYWGQGTLVTVSS (SEQ ID NO: 82)
>10A7. VL 
DIQMTQSPSSLSASVGDRVTITCRASQGISRWLAWYQQKPGKAPNLLIYG
ASTLQSGVPPRFSGSRSGTDFTLTISSLQPEDFATYYCQQ*YTTPPTFGQGTKLEI (SEQ
ID NO: 83)
>10D8. VL 
DIQMTQSPSSLSASVGDRVTITCRASQSISSWLAWYQQKPGKAPKLLIYD
VSNVQTGVPSRFSGSRSGTDFTLTISSLQPEDFATYYCQQ*YTTPPTFGQGTKLEI (SEQ
ID NO: 84)
>ABN3-2A VL 
DIQMTQSPSSLSASVGDRVTITCRASETIGSWLAWYQQKPGKAPNLLIYA
ASTLQSGVSSRFSGSRSGTDFTLTISSLQPEDFATYYCQQ*YTTPPTFGQGTKLEI (SEQ
ID NO: 85)
>ABN3-2B. VL 
DIQMTQSPSSLSASVGDRVTITCRASQGINNYLAWYQQKPGKAPKLLIYD
ASNLETGVPSRFSGSRSGTDFTLTISSLQPEDFATYYCQQ*YTTPPTFGQGTKLEI (SEQ
ID NO: 86)
>ABN3-2G. VL 
DIQMTQSPSSLSASVGDRVTITCQASHDISKYLNWYQQKPGKAPKLLIYD
ASNLETGVPSRFSGSRSGTDFTLTISSLQPEDFATYYCQQ*YTTPPTFGQGTKLEI (SEQ
ID NO: 87)
>4C10. VL 
DIQMTQSPSSLSASVGDRVTITCRATQAISNYLAWYQQKPGKAPKLLIYD
VSNVQTGVPSRFSGSRSGTDFTLTISSLQPEDFATYYCQQ*YTTPPTFGQGTKLEI (SEQ
ID NO: 88)
>5E12. VL 
DIQMTQSPSSLSASVGDRVTITCRTSQGITDPLNWYQQKPGKAPKLLIYK
TSSLESGVPSRFSGSRSGTDFTLTISSLQPEDFATYYCQQ*YTTPPTFGQGTKLEI (SEQ
ID NO: 89)
>5H12. VL 
DIQMTQSPSSLSASVGDRVTITCRASQGLSSWLAWYQQKPGKAPKLLIY
DASNLETGVPSRFSGSRSGTDFTLTISSLQPEDFATYYCQQ*YTTPPTFGQGTKLEI
(SEQ ID NO: 90)
>6D11. VL 
DIQMTQSPSSLSASVGDRVTITCRASQNIGTYLAWYQQKPGKAPKLLIYD
ASNLETGVPSRFSGSRSGTDFTLTISSLQPEDFATYYCQQ*YTTPPTFGQGTKLEI (SEQ
ID NO: 91)
>10F8. VL

DIQMTQSPSSLSASVGDRVTITCQASQDISNYLNWYQQKPGKAPNLLIYA
ASTLQSGVPSRFSGSRSGTDFTLTISSLQPEDFATYYCQQ*YTTPPTFGQGTKLEI (SEQ
ID NO: 92)

### 3) Antibody Expression and BN3 Conjugation

To prepare the selected clones as smacAbs for functional testing, the antibodies were first expressed in a full-length IgG1 format. The antibodies were then conjugated to the BN3 molecule and subjected to functional testing.

### 3. Functional Testing

### 1) Activity Evaluation of Ab-BN3

To evaluate the activity of the screened Ab-BN3 and to investigate the mode of interaction between Ab-BN3 and CD73, the inhibitory activities of Ab-BN3 and the corresponding unconjugated antibodies were tested. Ab-BN3 (FIG. 15A) was first tested for its inhibitory activity against CD73 at a concentration of 12.5 nM (about 2 µg/mL). The results are shown in FIG. 15B that all ten Ab-BN3 exhibited a certain degree of inhibitory activity.

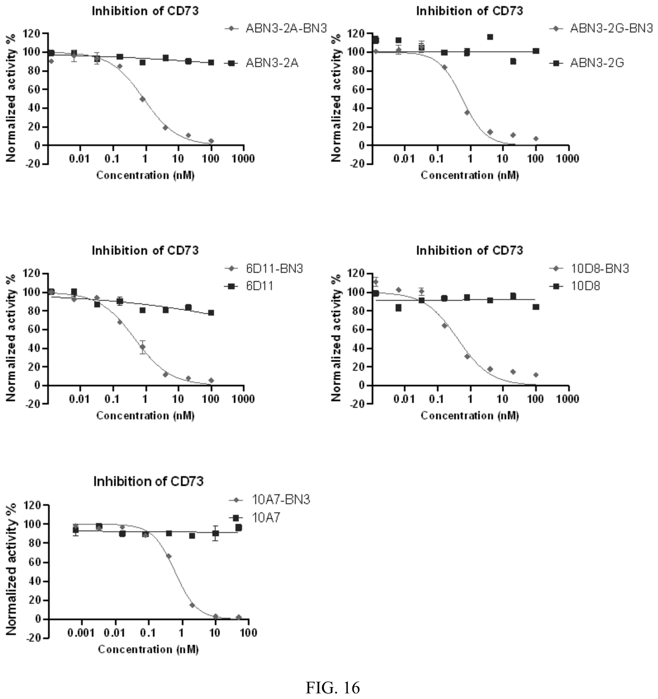

In the experiment, five Ab-BN3 molecules with relatively higher activity were selected for determination of inhibition curves. Specifically, CD73 was incubated with varying concentrations of Ab-BN3, followed by addition of AMP substrate to initiate the reaction. The phosphate concentration in the reaction products was measured using the malachite green phosphate assay. Data points are presented as mean ± SD, n = 2, and curves were fitted and plotted using GraphPad software. As shown in FIG. 16, the IC₅₀ values of the five Ab-BN3 molecules ranged from 0.2 to 0.8 nM (Table 4), and the IC₅₀ values among the Ab-BN3 molecules were relatively similar. However, the IC₉₀ values calculated from the inhibition curves varied significantly among the Ab-BN3 molecules. Among them, 10A7-BN3 exhibited the strongest inhibitory effect, with an IC₉₀ of 2.9 nM, followed by 6D11-BN3 with an IC₉₀ of 8.2 nM, while 10D8-BN3 exhibited the weakest inhibitory effect. The IC₅₀ and IC₉₀ values of the BN3 small molecule alone were 70.5 nM and 760.4 nM, respectively. Compared with BN3 alone, all five Ab-BN3 molecules showed markedly enhanced activity. In addition, it was observed that most of the antibodies not conjugated with BN3 did not exhibit inhibitory activity, with only 6D11 showing a weak inhibitory trend. Therefore, it can be concluded that the inhibitory activity of Ab-BN3 results from the synergistic action of BN3 and the antibody.

**Table 4: IC₅₀ and IC₉₀ values of Ab-BN3 for inhibition of CD73**

| Ab-BN3 | 10A7 | 10D8 | ABN3-2A | ABN3-2G | 6D11 |
|---|---|---|---|---|---|
| IC₅₀ ± SD (nM) | 0.6072 ±0.03865 | 0.4084 ±0.08681 | 0.8601 ±0.1037 | 0.5653 ±0.07136 | 0.4639 ±0.04723 |
| IC₉₀ (nM) | 2.9151 | >100 | 12.878 | 25.904 | 8.212 |

### 2) Activity Evaluation of Fab-BN3

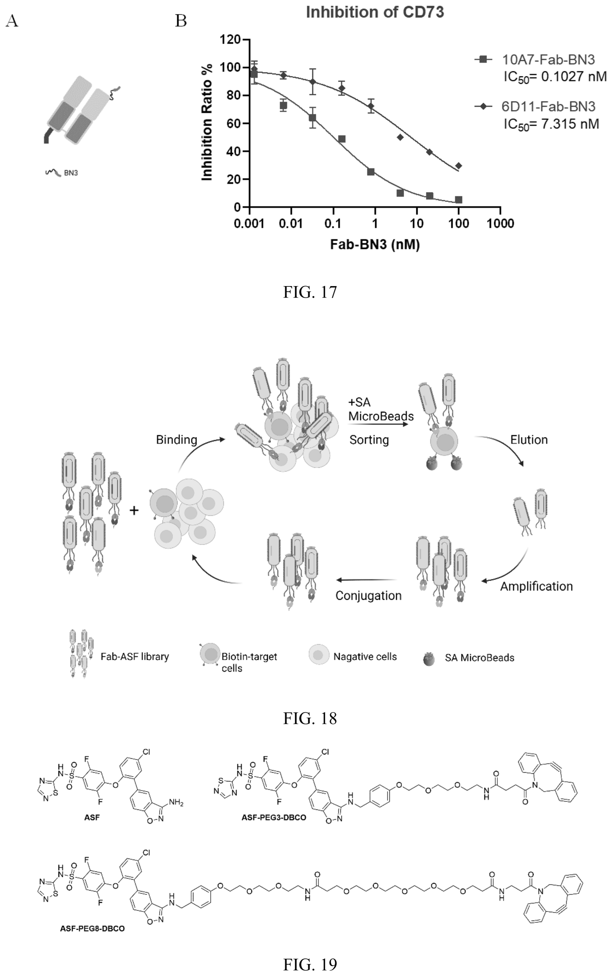

Monovalent antibody fragments, such as Fab and scFv, possess smaller molecular sizes and can more readily penetrate the tumor microenvironment, thereby potentially exerting enhanced tumor immunotherapeutic effects. According to the design of the smacAb library, smacAb screening was performed using a Fab library, suggesting that the screened smacAbs may function in a monovalent form. To demonstrate that monovalent smacAbs retain CD73 inhibitory activity, the IgG1 antibodies of the two most active clones, 6D11 and 10A7, were digested with papain and Fab fragments were recovered. The Fab fragments were then conjugated with BN3 to prepare monovalent Fab-BN3 (FIG. 17A), and their inhibitory activity against CD73 was evaluated. The inhibition curves are shown in FIG. 17B. Compared with the inhibitory activity of Ab-BN3, the two Fab-BN3 molecules exhibited opposite trends in activity change. 10A7-Fab-BN3 retained strong inhibitory activity, with an IC₅₀ value approximately one-fifth of that of 10A7-BN3. In contrast, the IC₅₀ of 6D11-Fab-BN3 was 7.315 nM, which was more than tenfold higher than that of 6D11-BN3, indicating a significant reduction in activity of the monovalent smacAb.

Overall, both Ab-BN3-derived monovalent Fab-BN3 molecules retained inhibitory activity against CD73, demonstrating that Ab-BN3 interacts with CD73 using Fab-BN3 as the recognition unit, consistent with the design concept of smacAbs. The different activity trends observed between the two Fab-BN3 molecules compared with their corresponding Ab-BN3 molecules indicate differences in their modes of action. Notably, 10A7-Fab-BN3 maintained strong inhibitory activity and shows potential for development as a monovalent antibody.

### 3) Affinity Evaluation of Ab-BN3

To further verify that the binding of Ab-BN3 results from the combined action of the Ab moiety and the BN3 moiety, the binding affinities of Ab-BN3 and the corresponding unconjugated antibodies were evaluated. The binding affinity of Ab-BN3 to CD73 was first analyzed using Octet BLI in the experiment. Ab-BN3 molecules were immobilized on protein A sensors and incubated with varying concentrations of CD73 to obtain binding signals. Data were collected and fitted for analysis using software. The affinity data for each Ab-BN3 are summarized in Table 5, with Herceptin-BN3 as the control (KD = 1.07 x 10⁻⁸ M). A total of eight Ab-BN3 molecules were tested in the experimental group, including the five Ab-BN3 exhibiting the strongest inhibitory activity, and the three Ab-BN3 showing the weakest activity among the ten Ab-BN3 corresponding to antibodies ABN-2B, 4C10, and 5E12. The KD values for the experimental group ranged from 2.97 x 10⁻¹⁰ to 4.37 x 10⁻⁹ M, overall lower than the KD of Herceptin-BN3, indicating significantly enhanced affinity of the screened Ab-BN3 for CD73. Moreover, the high-activity Ab-BN3 group exhibited lower KD values than the low-activity group, suggesting a correlation between high affinity and high inhibitory activity.

In addition, the affinities of the Ab corresponding to the five most active Ab-BN3 molecules for CD73 were also tested. The results showed that only a very weak binding signal can be observed when Ab was contacted with a 20 µM solution of CD73, indicating that Ab alone had a weak affinity for CD73.

**Table 5: Affinity data of Ab-BN3 for CD73**

| Ab-BN3 | KD (M) | KD Error | Kon (1/ms) | Kdis (1/s) | Full R² |
|---|---|---|---|---|---|
| ABN3-2A-BN3 | 2.97 x 10⁻¹⁰ | 7.80 x 10⁻¹² | 6.83 x 10⁵ | 2.03 x 10⁻⁴ | 0.9911 |
| ABN3-2B-BN3 | 3.16 x 10⁻⁹ | 5.87 x 10⁻¹¹ | 1.85 x 10⁵ | 5.82 x 10⁻⁴ | 0.9958 |
| ABN3-2G-BN3 | 8.87 x 10⁻¹⁰ | 1.02 x 10⁻¹¹ | 3.05 x 10⁵ | 2.71 x 10⁻⁴ | 0.9962 |
| 4C10-BN3 | 4.37 x 10⁻⁹ | 7.80 x 10⁻¹¹ | 1.16 x 10⁵ | 5.08 x 10⁻⁴ | 0.9947 |
| 5E12-BN3 | 4.04 x 10⁻⁹ | 5.84 x 10⁻¹¹ | 1.62 x 10⁵ | 6.56 x 10⁻⁴ | 0.9918 |
| 6D11-BN3 | 1.15 x 10⁻⁹ | 1.17 x 10⁻¹¹ | 1.78 x 10⁵ | 2.04 x 10⁻⁴ | 0.9982 |
| 10A7-BN3 | 8.88 x 10⁻¹⁰ | 1.53 x 10⁻¹¹ | 2.15 x 10⁵ | 1.91 x 10⁻⁴ | 0.9944 |
| 10D8-BN3 | 1.35 x 10⁻⁹ | 1.52 x 10⁻¹¹ | 2.04 x 10⁵ | 2.75 x 10⁻⁴ | 0.9956 |
| Herceptin-BN3 | 1.07 x 10⁻⁸ | 1.72 x 10⁻¹⁰ | 6.99 x 10⁴ | 7.51 x 10⁻⁴ | 0.991 |

In conclusion, the BN3 moiety in Ab-BN3 plays a critical role in affinity generation, and an appropriate antibody moiety contributes to affinity enhancement. Thus, both components participate in the binding of Ab-BN3 to CD73, consistent with the design concept of smacAbs.

### Example 5: Screening and Identification of smacAbs Using NaV1.7 as a Target Protein

The Fab-ASF library was incubated with biotinylated target cells and negative control cells, followed by magnetic bead sorting to collect target cells. The bound phage was eluted by thrombin cleavage, amplified, and re-prepared as a Fab-ASF library for subsequent rounds of screening.

### 1. Synthesis and Activity Evaluation of the ASF Small Molecule

To expand the functional range of the antibody, a flexible PEG linker was inserted between ASF and DBCO. In the experiment, ASF was separately conjugated with PEG3-DBCO and PEG8-DBCO to prepare ASF-PEG-DBCO molecules with PEG linkers of different lengths (structural formulas shown in FIG. 19).

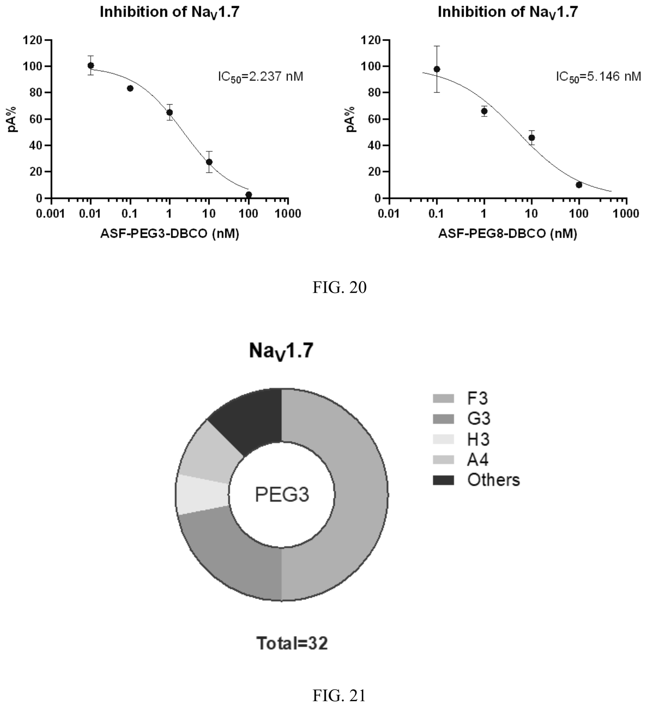

First, it was verified whether the ASF-PEG-DBCO molecules retained Nav1.7 inhibitory activity. Specifically, HEK293-Na_{V}1.7 cells were incubated with varying concentrations of ASF-PEG-DBCO for 15 min, followed by evaluation of inhibitory activity using the whole-cell patch clamp technique. Data points are presented as mean ± SD, n = 2-4, and curves were fitted and plotted using GraphPad software. The inhibition curves of the two ASF-PEG-DBCO molecules against Na_{V}1.7 are shown in FIG. 20. Both ASF-PEG-DBCO molecules exhibited inhibitory activity against Na_{V}1.7, but ASF-PEG3-DBCO displayed stronger activity, with IC₅₀ = 2.237 nM, indicating that longer PEG linkers have a certain impact on ASF activity. Accordingly, ASF-PEG3-DBCO was selected for subsequent screening.

### 2. Preparation and Screening of the Fab-ASF Library

Since ASF-PEG3-DBCO exhibited higher NaV1.7 inhibitory activity, ASF-PEG3-DBCO was conjugated with the newly constructed Fab-UAA library to prepare a Fab-PEG3-ASF library for NaV1.7 screening. The screening experiment was conducted according to the designed screening workflow (FIG. 18). In the first round of screening, only Nav1.7-overexpressing cells were used for screening, and in subsequent screening, a six-fold excess of HEK293 cells were added as negative control cells for screening.

### 1) Screening of the Fab-ASF Library Against NaV1.7

Screening of the library against the Na_{V}1.7 protein was performed according to the designed screening workflow (FIG. 18). HEK293-Na_{V}1.7 overexpressing cells were used as target cells, and HEK293 cells were used as negative control cells. A total of four rounds of screening were conducted in this experiment. After screening, 24 clones from the third-round output and 48 clones from the fourth-round output were randomly selected for Sanger sequencing analysis, yielding 22 and 37 valid sequences, respectively. No duplicate clones were observed among the third-round output. The eluted phage from the fourth-round screening was used to infect TG1 cells, plated, and grown into single colonies. Forty-eight single clones were randomly selected for Sanger sequencing. The VH amino acid sequences of the Fab fragments were analyzed to assess clone enrichment. The results are shown in FIG. 21 that four recurrent sequences, including F3, G3, H3, and A4, were enriched in the fourth-round output (wherein the VL CDR3 sequences are shown in SEQ ID NO: 5 to SEQ ID NO: 29, the VL CDR1-2 sequences are listed in Table 6, and the VH CDR1-3 sequences are listed in Table 7). Among them, F3 and A4 were also detected in the third-round output. The degree of clone enrichment in the fourth-round output increased significantly, and recurrent sequences were identified, indicating that for the Nav1.7 screening strategy, four rounds of screening may be necessary. Based on the screening results, four clones, F3, G3, H3, and A4, were selected for antibody expression and conjugation, and their inhibitory activity and selectivity against Na_{V}1.7 were tested.

**Table 6: VL CDR1-2**

| Antibody No. | VL CDR1 | VL CDR2 |
|---|---|---|
| F3 | RASQVIGNDLG (SEQ ID NO: 93) | KLLIYDVSNVQTGVPS (SEQ ID NO: 101) |
| G3 | RASQYISTYLN (SEQ ID NO: 94) | KVLISKTSYLESGVPS (SEQ ID NO: 102) |
| H3 | RASQSISSWLA (SEQ ID NO: 95) | NLLMYAASSLQSGVPS (SEQ ID NO: 103) |
| A4 | QASQDIRNDLN (SEQ ID NO: 96) | KSLIYAASRLQSGVPS (SEQ ID NO: 104) |
| 15F7 | RASQGISSDLG (SEQ ID NO: 97) | KLLIYDVSNVQTGVPS (SEQ ID NO: 101) |
| 15F9 | RASQSISSWLA (SEQ ID NO: 95) | KLLIYDASSLESGVPS (SEQ ID NO: 105) |
| 15A10 | RASQSIGSSLH (SEQ ID NO: 99) | TVLIYAASSLQTGVPS (SEQ ID NO: 107) |
| 15C12 | QASQDISNSLN (SEQ ID NO: 100) | NLLIYATSNLQSGVPS (SEQ ID NO: 108) |

**Table 7: VH CDR1-3**

| Antibody No. | VH CDR1 | VH CDR2 | VH CDR3 |
|---|---|---|---|
| F3 | FTFSGHWMH (SEQ ID NO: 109) | WVASISDSSSYIYYADSV RGRF (SEQ ID NO: 115) | ARDYEGATRAADY (SEQ ID NO: 124) |
| G3 | FTFSASYMS (SEQ ID NO: 110) | WIGSIYYSGSTYYNPSLK SRV (SEQ ID NO: 116) | ARGAPYYEGAFDI (SEQ ID NO: 125) |
| H3 | FTFSDYWIS (SEQ ID NO: 111) | WIGNVYYSGNAYYNPSL ESRV (SEQ ID NO: 117) | ARNTGFETLDN (SEQ ID NO: 126) |
| A4 | FTFSGHWMH (SEQ ID NO: 109) | WVSGISGSGGSTYYADS VKGRF (SEQ ID NO: 118) | ARELTMATTTDAFDI (SEQ ID NO: 127) |
| 15F7 | FTFSGHWMH (SEQ ID NO: 109) | WVSAISGSGGSTYYADS VKGRF (SEQ ID NO: 119) | AREVTMVRGVTDAFDI (SEQ ID NO: 128) |
| 15F9 | FTFSKFWMH (SEQ ID NO: 112) | WVSSISSSSSYIYYADSV KGRF (SEQ ID NO: 120) | ARDPISTGAFDI (SEQ ID NO: 129) |
| 15A10 | FPFSGHWMH (SEQ ID NO: 114) | WVSGISGTGATSHYADS VKGRF (SEQ ID NO: 122) | AREVITAASTDAFDL (SEQ ID NO: 131) |
| 15C12 | FTFSGHWMH (SEQ ID NO: 109) | WVSAISGSGGSTYYADS VKGRF (SEQ ID NO: 123) | ARELTMASTTDAFDF (SEQ ID NO: 132) |

The amino acid sequences of the heavy chain variable region and light chain variable region of each antibody are as follows:
>F3. VL DIQMTQSPSSLSASVGDRVTITCRASQVIGNDLGWYQQKPGKAPKLLIYD 
VSNVQTGVPSRFSGSRSGTDFTLTISSLQPEDFATYYCQQ*YTTPPTFGQGTKLEI (SEQ
ID NO: 133)
>G3. VL 
DIQMTQSPSSLSASVGDRVTITCRASQYISTYLNWYQQKPGKAPKVLISK
TSYLESGVPSRFSGSRSGTDFTLTISSLQPEDFATYYCQQ*YTTPPTFGQGTKLEI (SEQ
ID NO: 134)
>H3. VL 
DIQMTQSPSSLSASVGDRVTITCRASQSISSWLAWYQQKPGKAPNLLMY
AASSLQSGVPSRFSGSRSGTDFTLTISSLQPEDFATYYCQQ*YTTPPTFGQGTKLEI
(SEQ ID NO: 135)
>A4. VL 
DIQMTQSPSSLSASVGDRVTITCQASQDIRNDLNWYQQKPGKAPKSLIYA
ASRLQSGVPSRFSGSRSGTDFTLTISSLQPEDFATYYCQQ*YTTPPTFGQGTKLEI (SEQ
ID NO: 136)
>15F7.VL 
DIQMTQSPSSLSASVGDRVTITCRASQGISSDLGWYQQKPGKAPKLLIYD
VSNVQTGVPSRFSGSRSGTDFTLTISSLQPEDFATYYCQQ*YTTPPTFGQGTKLEI (SEQ
ID NO: 137)
>15F9. VL 
DIQMTQSPSSLSASVGDRVTITCRASQSISSWLAWYQQKPGKAPKLLIYD
ASSLESGVPSRFSGSRSGTDFTLTISSLQPEDFATYYCQQ*YTTPPTFGQGTKLEI (SEQ
ID NO: 138)
>15A10. VL 
DIQMTQSPSSLSASVGDRVTITCRASQSIGSSLHWYQQKPGKAPTVLIYA
ASSLQTGVPSRFSGSRSGTDFTLTISSLQPEDFATYYCQQ*YTTPPTFGQGTKLEI (SEQ
ID NO: 140)
>15C12. VL 
DIQMTQSPSSLSASVGDRVTITCQASQDISNSLNWYQQKPGKAPNLLIYA
TSNLQSGVPSRFSGSRSGTDFTLTISSLQPEDFATYYCQQ*YTTPPTFGQGTKLEI (SEQ
ID NO: 141)
>F3. VH 
EVQLVESGGGLVQPGGSLRLSCAASGFTFSGHWMHWVRQAPGKGLEW
VASISDSSSYIYYADSVRGRFTISRDNAKKTLYLQMNSLRAEDTAVYYCARDYEGATR
AADYWGQGTLVTVSS (SEQ ID NO: 142)
>G3. VH 
EVQLVESGGGLVQPGGSLRLSCAASGFTFSASYMSWVRQAPGKGLEWIG
SIYYSGSTYYNPSLKSRVTISADTSKNTAYLQMNSLRAEDTAVYYCARGAPYYEGAFD
IWGQGTLVTVSS (SEQ ID NO: 143)
>H3. VH 
EVQLVESGGGLVQPGGSLRLSCAASGFTFSDYWISWVRQAPGKGLEWIG
NVYYSGNAYYNPSLESRVTISADTSKNTAYLQMNSLRAEDTAVYYCARNTGFETLDN
WGQGTLVTVSS (SEQ ID NO: 144)
>A4. VH 
EVQLVESGGGLVQPGGSLRLSCAASGFTFSGHWMHWVRQAPGKGLEW
VSGISGSGGSTYYADSVKGRFTISADTSKNTAYLQMNSLRAEDTAVYYCARELTMATT
TDAFDIWGQGTLVTVSS (SEQ ID NO: 145)
>15F7.VH 
EVQLVESGGGLVQPGGSLRLSCAASGFTFSGHWMHWVRQAPGKGLEW
VSAISGSGGSTYYADSVKGRFTISADTSKNTAYLQMNSLRAEDTAVYYCAREVTMVR
GVTDAFDIWGQGTLVTVSS (SEQ ID NO: 146)
>15F9. VH 
EVQLVESGGGLVQPGGSLRLSCAASGFTFSKFWMHWVRQAPGKGLEW
VSSISSSSSYIYYADSVKGRFTISADTSKNTAYLQMNSLRAEDTAVYYCARDPISTGAF
DIWGQGTLVTVSS (SEQ ID NO: 147)
>15A10. VH 
EVQLVESGGGLVQPGGSLRLSCAASGFPFSGHWMHWVRQAPGKGLEW
VSGISGTGATSHYADSVKGRFTISADTSKNTAYLQMNSLRAEDTAVYYCAREVITAAS
TDAFDLWGQGTLVTVSS (SEQ ID NO: 149)
>15C12. VH

EVQLVESGGGLVQPGGSLRLSCAASGFTFSGHWMHWVRQAPGKGLEW

VSAISGSGGSTYYADSVKGRFTISADTSKNTAYLQMNSLRAEDTAVYYCARELTMAST
TDAFDFWGQGTLVTVSS (SEQ ID NO: 150)

### 2) Antibody Expression and Conjugation with ASF

To evaluate the inhibitory activity of the screened smacAbs against Na_{V}1.7, each clone was first expressed as a full-length IgG1 antibody. The resulting Ab-UAA was then conjugated with ASF-PEG3-DBCO to prepare Ab-PEG3-ASF, which was subsequently evaluated for inhibitory activity and selectivity.

### 3. Inhibitory Activity of Ab-PEG3-ASF Against Na_{V}1.7

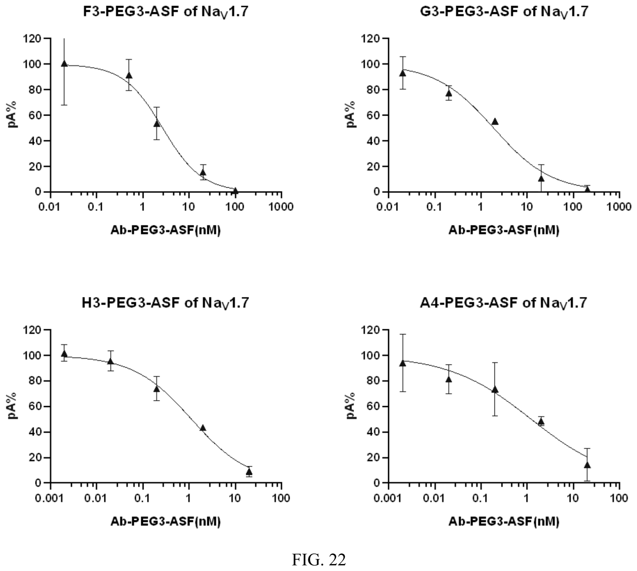

To evaluate the inhibitory activity of the screened smacAbs against Na_{V}1.7, the whole-cell patch clamp technique was used in the experiment to measure the inhibitory effect of Ab-PEG3-ASF on Na_{V}1.7. Specifically, HEK293-Nav1.7 cells were incubated with different concentrations of Ab-PEG3-ASF for 15 min, followed by measurement of sodium currents using the whole-cell patch clamp technique. Data points are presented as mean ± SD, n = 2-3, and curves were fitted and plotted using GraphPad. The inhibition curve of Ab-PEG3-ASF against Na_{V}1.7 is shown in FIG. 22, and the IC₅₀ values are listed in Table 8. The IC₅₀ of Ab-PEG3-ASF in inhibiting Na_{V}1.7 ranges from 1.0 nM to 3.0 nM, comparable to that of the small-molecule ASF-PEG3-DBCO. Among the four smacAbs, H3-PEG3-ASF exhibited the strongest inhibitory activity, with an IC₅₀ of about 1 nM.

**Table 8: IC₅₀ of Ab-PEG3-ASF in inhibiting Nav1.7**

| Ab-PEG3-ASF | IC₅₀ ± SEM (nM) |
|---|---|
| F3-PEG3-ASF | 2.692± 0.6870 |
| G3-PEG3-ASF | 1.880± 0.3943 |
| H3-PEG3-ASF | 1.129± 0.1913 |
| A4-PEG3-ASF | 1.218± 0.5341 |

The experimental results demonstrate that all four Ab-PEG3-ASF molecules selected from the Fab-PEG3-ASF library, based on the duplicate clone priority principle, exhibited Na_{V}1.7 inhibitory activities comparable to that of the small-molecule ASF-PEG3-DBCO. Therefore, screening of the smacAb library enables the identification of Ab-PEG3-ASF molecules with potent NaV1.7 inhibitory activity, which preliminarily verifies the applicability of the smacAb design and library screening strategy for functional antibody discovery against complex membrane proteins.

### 4. Specificity Assessment of Ab-PEG3-ASF Inhibitory Activity

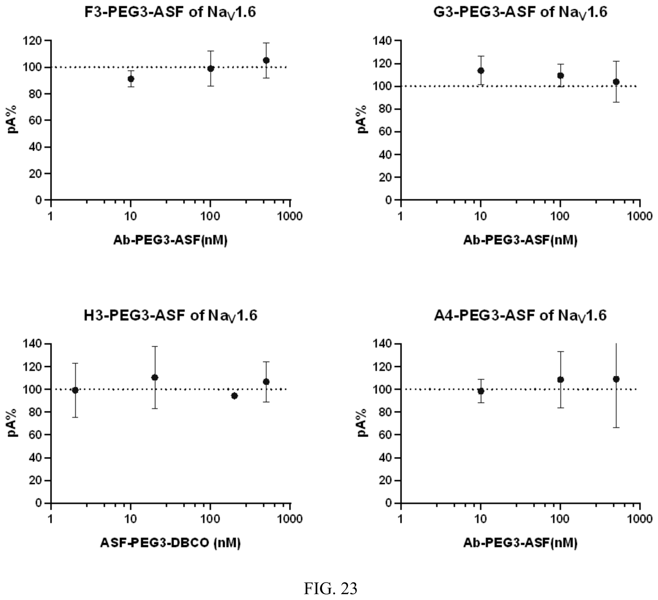

According to literature reports, the ASF small molecule also exhibits certain inhibitory activity against Na_{V}1.2 and Na_{V}1.6. To determine whether the screened ASF smacAbs retain Na_{V}1.7 inhibitory activity while achieving subtype-selective inhibition, the inhibitory effects of the four Ab-PEG3-ASF molecules against Nav1.2 and Na_{V}1.6 were evaluated. For each target, 3-4 concentration points were tested, with a maximum concentration of 500 nM or 1 µM. Specifically, HEK293-Na_{V}1.6 cells were incubated with different concentrations of Ab-PEG3-ASF at room temperature for 15 minutes prior to testing. Data points are presented as mean ± SD, n = 2-3, and curves were fitted and plotted using GraphPad. The inhibitory activity against Na_{V}1.6 is shown in FIG. 23, and none of the four Ab-PEG3-ASF molecules exhibited inhibitory activity against Nav1.6.

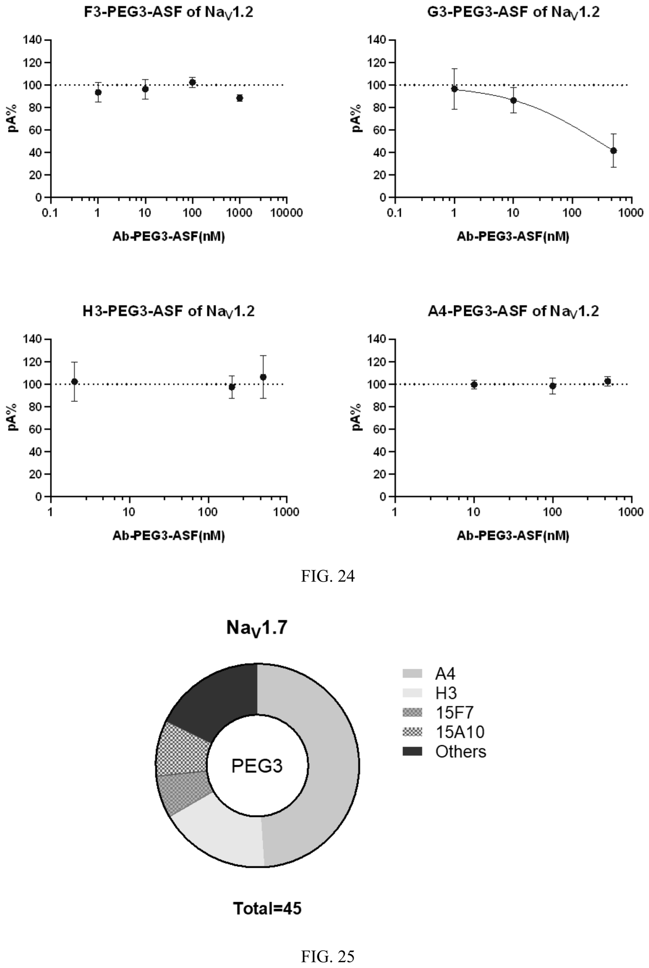

HEK293-Na_{V}1.2 cells were incubated with different concentrations of Ab-PEG3-ASF at room temperature for 15 minutes prior to testing. Data points are presented as mean ± SD, n = 2-3, and curves were fitted and plotted using GraphPad. The inhibitory activity against Na_{V}1.2 is shown in FIG. 24. G3-PEG3-ASF exhibited certain inhibitory activity against Na_{V}1.2, achieving approximately 60% inhibition at the highest tested concentration of 500 nM. In contrast, the other three Ab-PEG3-ASF molecules did not exhibit any inhibitory activity against Na_{V}1.2 at concentrations up to 500 nM.

In conclusion, screening of the phage-displayed Fab-PEG3-ASF library using Na_{V}1.7 as the target yielded four Ab-PEG3-ASF molecules with Na_{V}1.7 inhibitory activity comparable to that of the ASF small molecule. Among these, three Ab-PEG3-ASF molecules did not exhibit the inhibitory activities against Na_{V}1.2 or Na_{V}1.6, indicating that Ab-PEG3-ASF had selective Na_{V}1.7 inhibitory activity, and smacAb design can achieve selective inhibition of ion channel subtype proteins. However, G3-ASF-PEG3 with inhibitory activity against Nav1.2 was also enriched in the screening output. Therefore, the library screening strategy was optimized in the next step to increase the selectivity of the screening output for Na_{V}1.7.

### 5. Optimization and Identification of smacAb Library Screening

To further improve the selectivity of the library screening output for Na_{V}1.7, the library screening strategy was optimized. During library screening, HEK293-Na_{V}1.2 cells and HEK293-Nav1.6 cells were used as negative cells instead of HEK293 cells to perform subtype competitive screening (referred to as competitive screening) of the library, aiming to thoroughly remove clones capable of binding to Na_{V}1.2 and Na_{V}1.6 during screening. Similarly, monoclonal colonies from the library screening output were subjected to Sanger sequencing, and the Fab genes of the resulting clones were analyzed. Clones were selected based on the duplicate clone priority principle, expressed as Ab-UAA, and conjugated to ASF-PEG3-DBCO to prepare Ab-PEG3-ASF. The inhibitory activity and specificity against Na_{V}1.7 were then determined.

Competitive Fab-ASF Library Screening Targeting Nav1.7: Competitive library screening targeting Na_{V}1.7 was performed using the Fab-PEG3-ASF library. In the first round of screening, only target cells, i.e., Na_{V}1.7-overexpressing cells, were used. From the second round onward, HEK293-Na_{V}1.2 and HEK293-Na_{V}1.6 cells were employed as negative cells for competitive screening of library, for a total of four rounds of screening. Monoclonal colonies were randomly selected from the screening output for analysis of Fab variable region genes to evaluate sequence enrichment in the screening output. Monoclonal colonies were randomly selected from the Na_{V}1.7-targeted competitive library screening output for Sanger sequencing for analysis of Fab variable region sequences to statistically evaluate the sequence enrichment. The sequence enrichment results of the competitive library screening output are shown in FIG. 25. Five recurrent sequences were identified, including two clones, A4 and H3, that had also appeared in the non-competitive library screening output and had been verified to exhibit selective inhibitory activity against Nav1.7; and two additional recurrent sequences corresponded to newly identified clones 15F7 and 15A10. In addition, a clone (15C12) with a sequence highly similar to A4 was identified.

Considering sequencing results of the clones, four clones 15F7, 15A10, and 15C12 (wherein the VL CDR3 sequences are show in SEQ ID NO: 5-29; VL CDR1-2 sequences are listed in Table 6; and VH CDR1-3 sequences are listed in Table 7), exhibiting high sequence enrichment, were selected for subsequent experiments, along with one additional clone, 15F9, selected at random. In subsequent experiments, these five clones were expressed and prepared as Ab-PEG3-ASF and evaluated for inhibitory activity and selectivity.

### 6. Inhibitory Activity of Ab-PEG3-ASF Against Na_{V}1.7

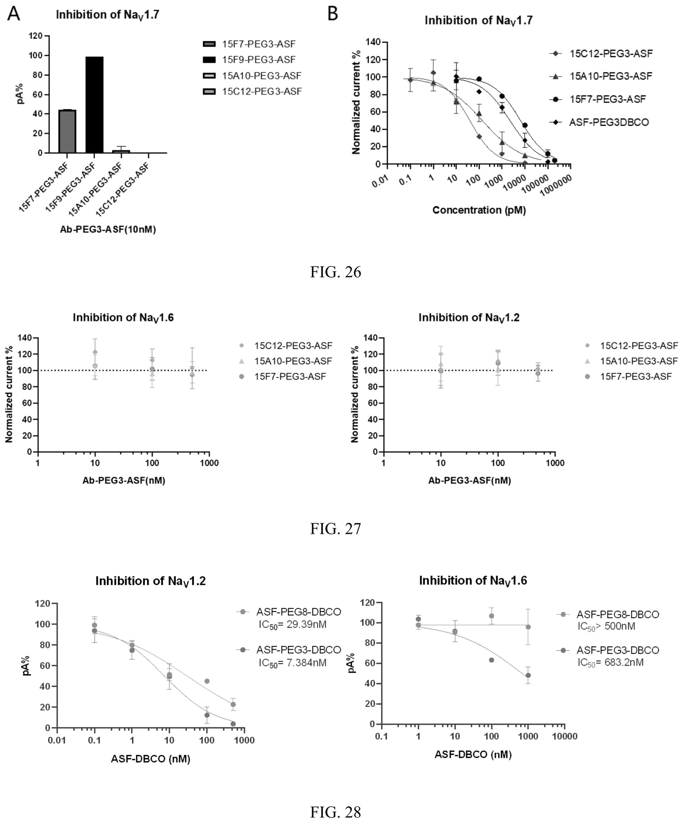

Since the smacAbs screened from the Na_{V}1.7 competitive library have been successfully prepared in the above experiments, the inhibitory activity of each Ab-PEG3-ASF against the target protein Na_{V}1.7 was evaluated in this experiment. The evaluation of activity of Ab-PEG3-ASF was performed by the whole-cell patch clamp technique using Na_{V}1.7-overexpressing cells. The inhibitory activity of 10 nM Ab-PEG3-ASF against Na_{V}1.7 was tested first. HEK293-Na_{V}1.7 cells were incubated with varying concentrations of Ab-PEG3-ASF at room temperature for 15 minutes prior to testing. The results are shown in FIG. 26A. 15F9-PEG3-ASF exhibited essentially no inhibitory activity, whereas the inhibition rate of 15F7-PEG3-ASF was about 60%. The remaining three Ab-PEG3-ASF molecules achieved nearly complete inhibition of Na_{V}1.7 at 10 nM. Therefore, concentration gradients of the four Ab-PEG3-ASFs (excluding 15F9-PEG3-ASF) were selected to determine the concentration-response curve for Na_{V}1.7 inhibition.

The inhibition curves of the four Ab-PEG3-ASF molecules against Nav1.7 are shown in FIG. 26B. IC₅₀ values calculated by curve fitting are listed in Table 9. Among them, 15F7-PEG3-ASF exhibited the weakest inhibitory activity, with an IC₅₀ of 6.484 nM. The IC₅₀ values of the other three Ab-PEG3-ASF were all below 0.2 nM, showing a significant improvement in inhibitory activity compared to ASF-PEG3-DBCO, indicating that the optimized library screening can acquire Ab-ASF with stronger inhibitory activity than the ASF small molecule.

**Table 9: IC₅₀ values of Na_{V}1.7-selective Ab-ASF**

| Ab-ASF | IC₅₀ ± SEM |
|---|---|
| 15F7-PEG3-ASF | 6.484 ± 1.199 nM |
| 15A10-PEG3-ASF | 138.2 ± 41.36 pM |
| 15C12-PEG3-ASF | 39.45 ± 9.684 pM |

### 7. Specificity of Ab-PEG3-ASF Inhibitory Activity

To verify the higher specificity of Ab-PEG3-ASF derived from Nav1.7 screening compared to the ASF molecules, the inhibitory activity of Ab-PEG3-ASF against Na_{V}1.2 and Na_{V}1.6 was tested, respectively. HEK293-Na_{V}1.6 cells and HEK293-Na_{V}1.2 cells were incubated with the corresponding concentrations of Ab-PEG3-ASF at room temperature for 15 min prior to the inhibitory activity test. Three concentrations were tested: 10 nM, 100 nM, and 500 nM. Data points are presented as mean ± SD, n = 2-4. The inhibitory activity results are shown in FIG. 27. In this experiment, the inhibitory activities against Nav1.2 and Nav1.6 were detected at three concentrations: 10 nM, 100 nM, and 500 nM. All four Ab-PEG3-DBCO showed no inhibitory activity against Na_{V}1.2 or Na_{V}1.6, indicating that the Ab-ASF molecules obtained via competitive library screening exhibit specific inhibitory activity against Na_{V}1.7.

In conclusion, competitive library screening targeting Na_{V}1.7 yielded five recurrent sequences, including clones H3 and A4 previously identified in non-competitive screening as selectively inhibiting Na_{V}1.7, as well as two newly identified recurrent sequences, 15F7 and 15A10. In addition, clone 15C12, exhibiting sequence similarity to H3, was identified in the sequencing results. The activity test experiment confirmed that Ab-PEG3-ASF prepared from these four new clones exhibited Na_{V}1.7 inhibitory activity. Among them, 15F7-PEG3-ASF showed the weakest inhibitory activity, with IC₅₀ = 6.5 nM, which was two-fold higher than that of ASF-PEG3-DBCO, whereas the inhibitory activities of the remaining three clones were all stronger than that of the small molecule itself. Moreover, both the H3 and A4 clones and the newly identified clones exhibited highly specific inhibitory activity against Na_{V}1.7, with no detectable inhibition against Na_{V}1.2 or Na_{V}1.6 even at concentrations up to 500 nM. Notably, clone G3, which exhibited Nav1.2 inhibitory activity, was not observed among the competitive screening output, indicating that competitive screening facilitated the identification of highly selective clones. These results indicate that competitive library screening targeting Na_{V}1.7 enables the identification of Ab-PEG3-ASF molecules with inhibitory activity and specificity superior to those of the ASF small molecule, further validating the applicability of the smacAb screening strategy for functional antibody discovery targeting NaV1.7.

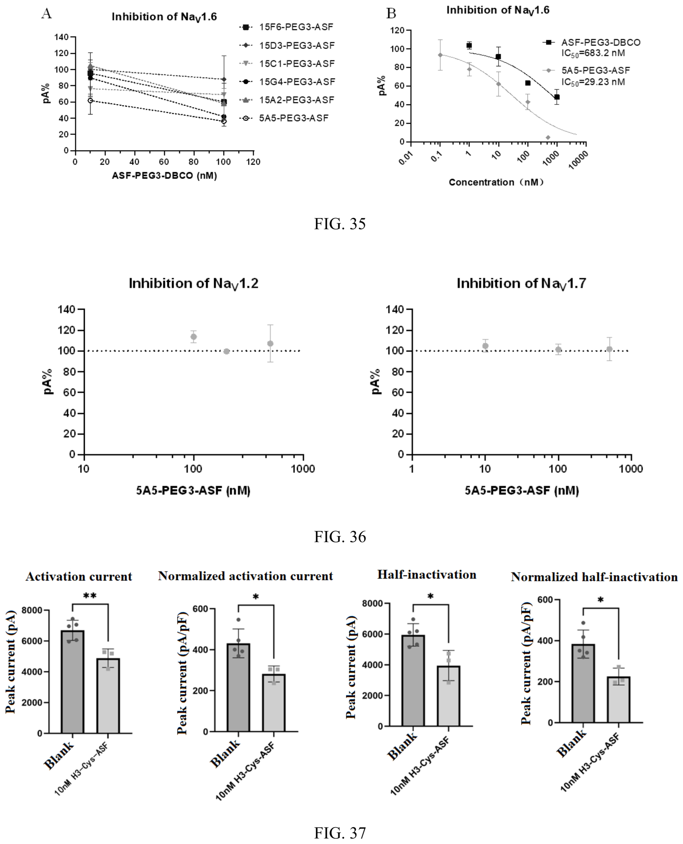

Additionally, alternative conjugation approaches were explored to conjugate small molecules with antibodies for smacAb preparation. In this example, H3-PEG3-ASF targeting NaV1.7 was selected as a model smacAb to assess whether an active H3-PEG3-ASF_{Mal} could be prepared via a Michael addition reaction. In this experiment, the conjugation site of H3, i.e., amino acid residue 91 of the light chain, was mutated to cysteine to introduce a free thiol group, and the full-length antibody H3-Cys was expressed using a HEK293F cell system. Meanwhile, the ASF molecule was modified with a PEG3-maleimide reactive group to prepare ASF-PEG3-Mal. H3-Cys and ASF-PEG3-Mal were conjugated via a Michael addition reaction to prepare H3-PEG3-ASF_{Mal}, and its inhibitory activity against Na_{V}1.7 was evaluated using the patch clamp technique. The activity test results for H3-PEG3-ASF_{Mal} are shown in FIG. 37. The results indicate that the cysteine-maleimide conjugation reaction is also suitable for smacAb construction.

In conclusion, the smacAb corresponding to H3 was successfully prepared via a Michael addition reaction, and the resulting smacAb retained inhibitory activity. Therefore, the smacAb preparation process is compatible with other common site-directed conjugation techniques.

### Example 6: Screening and Identification of smacAbs Using NaV1.2 and NaV1.6 as a Target Protein

### 1. Inhibitory Activity test of ASF Against Na_{V}1.2 and Na_{V}1.6

Prior to screening the Fab-ASF library targeting Na_{V}1.2 and Na_{V}1.6, it was necessary to confirm that the DBCO-modified ASF small molecule retained inhibitory activity against Nav1.2 and Nav1.6. Specifically, HEK293 overexpressing cell lines were incubated with various concentrations of the small molecule for 15 min at room temperature prior to the activity test. Data are presented as mean ± SD, n = 2-4, and curves were fitted and plotted using GraphPad. The inhibitory activities of ASF-DBCO molecules against Na_{V}1.2 and Na_{V}1.6 were evaluated in the experiment using the constructed HEK293-Na_{V}1.2 and HEK293-Na_{V}1.6 overexpressing cell lines. The experimental results are shown in FIG. 28 that both ASF-PEG3-DBCO and ASF-PEG8-DBCO exhibited inhibitory activity against Nav1.2; however, elongation of the PEG chain adversely affected the Na_{V}1.2 inhibitory activity of ASF-DBCO. ASF-PEG3-DBCO retained inhibitory activity against Nav1.6, with a high IC₅₀ of 683.2 nM, whereas ASF-PEG8-DBCO showed no inhibitory activity against Na_{V}1.6. These results indicate that the PEG chain elongation reduces the inhibitory activity of ASF-DBCO against Na_{V}1.6, consistent with the observations from the Na_{V}1.2 inhibitory activity test experiment.

In conclusion, the ASF-PEG3-DBCO molecule retained inhibitory activity against both Na_{V}1.2 and Na_{V}1.6. In contrast, elongation of the PEG chain led to reduced inhibitory activity, with ASF-PEG8-DBCO exhibiting weaker activity against Na_{V}1.2 relative to ASF-PEG3-DBCO and no inhibitory activity against Na_{V}1.6.

### 2. Screening and Identification of smacAbs Targeting Na_{V}1.2

Fab-ASF Library Screening Targeting Na_{V}1.2: Two small molecules with different PEG chain lengths, ASF-PEG3-DBCO and ASF-PEG8-DBCO, were separately conjugated to the newly prepared Fab-UAA phage to prepare Fab-PEG3-ASF library and Fab-PEG8-ASF library for NaV1.2 screening. Library screening was performed following the Na_{V}1.7 screening workflow (FIG. 18), using HEK293-Na_{V}1.2 overexpressing cells as target cells. For non-competitive screening, HEK293 cells were employed as negative control cells, while for competitive screening, HEK293-Nav1.6 and HEK293-Nav1.7 cells were used as negative control cells. A total of four rounds of screening were performed.

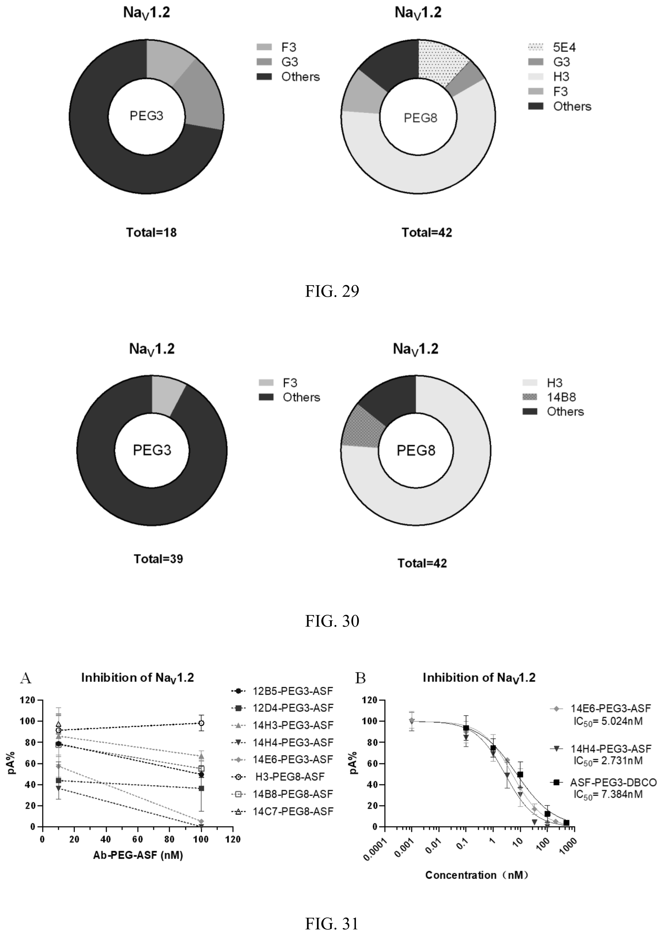

First, non-competitive library screen targeting Nav1.2 was conducted. Phages eluted in the fourth round of the Na_{V}1.2-targeted non-competitive library screening were used to infect TG1 cells, plated, and grown into monoclonal colonies. Single clones were randomly selected for Sanger sequencing. Fab variable region gene sequences were translated into amino acid sequences and aligned, and the frequency of sequence recurrence was analyzed. The statistical results of clone enrichment from library screening output are shown in FIG. 29. Two recurrent sequences, G3 and F3, were observed in the PEG3 library screening output. However, both recurrent sequences were Na_{V}1.7-selective antibody sequences. F3-PEG3-ASF showed no inhibitory activity against Na_{V}1.2 at concentrations up to 500 nM, whereas G3-PEG3-ASF exhibited only weak inhibitory activity against Na_{V}1.2. These results indicated that the current Fab-PEG3-ASF library screen failed to acquire smacAbs with Na_{V}1.2-selective inhibitory activity. Four recurrent sequences were identified in the PEG8 library screening output, three of which were F3, G3, and H3, all corresponding to Na_{V}1.7-selective smacAbs in their Ab-PEG3-ASF format. Additionally, a novel recurrent sequence, 5E4 (observed five times), was identified. In conclusion, through non-competitive library screening targeting Na_{V}1.2, the recurrent sequences obtained in the PEG3 group were Na_{V}1.7 selective clones F3 and G3. Although the novel clone 5E4 was detected in the PEG8 group, the most frequently observed clone in this group was H3, indicating that the screening results of the PEG8 group were also not ideal. These results suggest that non-competitive screening may be insufficient to identify Ab-ASF molecules with selective Na_{V}1.2-specific inhibitory activity, prompting the use of competitive screening strategies.

To acquire Ab-ASF molecules with Na_{V}1.2-selective inhibitory activity, competitive library screen targeting Nav1.2 was performed. Phages eluted in the fourth round of the Na_{V}1.2-targeted competitive library screening were used to infect TG1 cells, plated and grown into monoclonal colonies. Single clones were randomly selected for Sanger sequencing; Fab variable region gene sequences were translated into amino acid sequences and aligned, and the frequency of sequence recurrence was analyzed. The statistical results of sequence enrichment of the competitive library screening output are shown in FIG. 30. The sequence enrichment of the Fab-PEG3-ASF library screening output was insufficiently high, and the only recurrent sequence was the Na_{V}1.7-specific F3 clone. Clones from the Fab-PEG8-ASF library screening output exhibited a relatively high degree of sequence enrichment. Two recurrent sequences were identified: one was the Na_{V}1.7-selective clone H3, and the other type was the novel clone 14B8. In addition, clone 14C7, whose sequence was highly similar to that of 14B8, was also detected.

Clone enrichment results of non-competitive screening and competitive screening targeting Na_{V}1.2 were analyzed to explore whether competitive screening was conducive to enrichment of clones of target proteins. Through screening of the Fab-PEG3-ASF library, two Na_{V}1.7-selective clones, F3 and G3, were observed among the non-competitive screening output, whereas only clone F3 appeared in the competitive screening outputs Through screening of the Fab-PEG8-ASF library, three Na_{V}1.7-selective clones, including F3, G3, and H3, were enriched among the non-competitive screening output, whereas only clone H3 was detected in the competitive screening output. These results indicate that during the screening process of both PEG3 and PEG8 libraries, the competitive library screening strategy eliminated some Na_{V}1.7-selective clones, thereby increasing the probability of detecting Na_{V}1.2-selective clones. Therefore, clones selected from the competitive screening output were expressed and prepared as Ab-ASF conjugates and subsequently tested for inhibitory activity and selectivity.

The sequence enrichment profiles of the competitive library screening output were analyzed to select clones for downstream experiments. Most cloned sequences from the PEG3 library screening output were not recurrent sequences; however, clustering of CDRs was observed. Therefore, one clone each was selected from several sequences exhibiting relatively high CDR enrichment for downstream experiments, including 12B5, 12D4, 14H3, 14H4, and 14E6 (wherein the VL CDR3 sequences are show in SEQ ID NO: 5 to SEQ ID NO: 29; the VL CDR1-2 sequences are listed in Table 10; and the VH CDR1-3 sequences are listed in Table 11). For the PEG8 library screening output, clones H3, 14B8, and 14C7 (wherein the VL CDR3 sequences are shown in SEQ ID NO: 5 to SEQ ID NO: 29; the VL CDR1-2 sequences are listed in Table 10; and the VH CDR1-3 sequences are listed in Table 11) were selected for downstream experiments, as the Na_{V}1.2-selective inhibitory activity of H3-PEG8-ASF remained undetermined.

**Table 10: VL CDR1-2**

| Antibody No. | VL CDR1 | VL CDR2 |
|---|---|---|
| H3 | RASQSISSWLA (SEQ ID NO: 95) | NLLMYAASSLQSGVPS (SEQ ID NO: 103) |
| G3 | RASQYISTYLN (SEQ ID NO: 94) | KVLISKTSYLESGVPS (SEQ ID NO: 102) |
| 12B5 | QASQGISHALA (SEQ ID NO: 151) | KLLIYAASSLQSGVPS (SEQ ID NO: 157) |
| 14H3 | RASQTISDWLA (SEQ ID NO: 152) | KLLIYDASSLESGVPS (SEQ ID NO: 158) |
| 14H4 | RASQSISSDLN (SEQ ID NO: 153) | KLLIYRATFLESGVPS (SEQ ID NO: 159) |
| 12D4 | QASQDISNYLN (SEQ ID NO: 154) | KLLIYAASRLQSGVPS (SEQ ID NO: 160) |
| 14B8 | RASQSISSWLA (SEQ ID NO: 95) | NLLMYAASSLQSGVPS (SEQ ID NO: 161) |
| 14C7 | RTSQSVNNHLN (SEQ ID NO: 155) | KLLIYRASSLESGVPS (SEQ ID NO: 162) |
| 14E6 | RASQPISDWLA (SEQ ID NO: 156) | KSLIYDAYNLQSGVPP (SEQ ID NO: 163) |

**Table 11: VH CDR1-3**

| | | | |
|---|---|---|---|
| G3 | FTFSASYMS (SEQ ID NO: 110) | WIGSIYYSGSTYYNPSLKSRV (SEQ ID NO: 116) | ARGAPYYEGAFDI (SEQ ID NO: 125) |
| H3 | FTFSDYWIS (SEQ ID NO: 111) | WIGNVYYSGNAYYNPSLESRV (SEQ ID NO: 117) | ARNTGFETLDN (SEQ ID NO: 126) |
| 12B5 | FTFSTYVMH (SEQ ID NO: 164) | WLANIDTGGGVTNYADSVKGRF (SEQ ID NO: 171) | ARNSGFETLDY (SEQ ID NO: 178) |
| 12D4 | FTFSDYAMT (SEQ ID NO: 165) | VWVSRIKHDGSSTAYADSVKGRF (SEQ ID NO: 172) | AREGNYCLDY (SEQ ID NO: 179) |
| 14H3 | FTFSSYCMN (SEQ ID NO: 166) | WVSSVTGSAQRTNYADSVKGRF (SEQ ID NO: 173) | ARNTGFETLDN (SEQ ID NO: 126) |
| 14H4 | FSFSKFWMH (SEQ ID NO: 167) | WVAFMAHDESNKNYADSVKGRF (SEQ ID NO: 174) | ARDKVCNTFDI (SEQ ID NO: 180) |
| 14E6 | FTFSNFAMH (SEQ ID NO: 168) | YVSAISGGNGGGTYYADSAKGRF (SEQ ID NO: 175) | AREGNYCLDY (SEQ ID NO: 181) |
| 14C7 | FTFSSFSMS (SEQ ID NO: 169) | WVANIKEDGSDKYYVDSVTGRF (SEQ ID NO: 176) | ARDWGAAAVLHH (SEQ ID NO: 182) |
| 14B8 | FTFSGHWMH (SEQ ID NO: 170) | WVAVISSDGSYKSYADSVKGRF (SEQ ID NO: 177) | ASRTVAGTLDY (SEQ ID NO: 183) |

The amino acid sequences of the heavy chain variable region and light chain variable region of each antibody are as follows:
>12B5. VL 
DIQMTQSPSSLSASVGDRVTITCQASQGISHALAWYQQKPGKAPKLLIYA
ASSLQSGVPSRFSGSRSGTDFTLTISSLQPEDFATYYCQQ*YTTPPTFGQGTKLEI (SEQ
ID NO: 184)
>14H3. VL 
DIQMTQSPSSLSASVGDRVTITCRASQTISDWLAWYQQKPGKAPKLLIYD
ASSLESGVPSRFSGSRSGTDFTLTISSLQPEDFATYYCQQ*YTTPPTFGQGTKLEI (SEQ
ID NO: 185)
>14H4. VL 
DIQMTQSPSSLSASVGDRVTITCRASQSISSDLNWYQQKPGKAPKLLIYR
ATFLESGVPSRFSGSRSGTDFTLTISSLQPEDFATYYCQQ*YTTPPTFGQGTKLEI (SEQ
ID NO: 186)
>12D4. VL 
DIQMTQSPSSLSASVGDRVTITCQASQDISNYLNWYQQKPGKAPKLLIYA
ASRLQSGVPSRFSGSRSGTDFTLTISSLQPEDFATYYCQQ*YTTPPTFGQGTKLEI (SEQ
ID NO: 187)
>14E6. VL 
DIQMTQSPSSLSASVGDRVTITCRASQPISDWLAWYQQKPGKAPKSLIYD
AYNLQSGVPPRFSGSRSGTDFTLTISSLQPEDFATYYCQQ*YTTPPTFGQGTKLEI (SEQ
 ID NO: 188)
>14C7. VL 
DIQMTQSPSSLSASVGDRVTITCRTSQSVNNHLNWYQQKPGKAPKLLIYR
ASSLESGVPSRFSGSRSGTDFTLTISSLQPEDFATYYCQQ*YTTPPTFGQGTKLEI (SEQ
ID NO: 189)
>14B8. VL 
DIQMTQSPSSLSASVGDRVTITCRASQSISSWLAWYQQKPGKAPNLLMY
AASSLQSGVPSRFSGSRSGTDFTLTISSLQPEDFATYYCQQ*YTTPPTFGQGTKLEI
(SEQ ID NO: 190)
>12B5. VH 
EVQLVESGGGLVQPGGSLRLSCAASGFTFSTYVMHWVRQAPGKGLEWL
ANIDTGGGVTNYADSVKGRFTISADTSKNTAYLQMNSLRAEDTAVYYCARNSGFETL
DYWGQGTLVTVSS (SEQ ID NO: 191)
>12D4. VH 
EVQLVESGGGLVQPGGSLRLSCAASGFTFSDYAMTWVRQAPGKGLVWV
SRIKHDGSSTAYADSVKGRFTISADTSKNTAYLQMNSLRAEDTAVYYCAREGNYCLD
YWGQGTLVTVSS (SEQ ID NO: 192)
>14H3. VH 
EVQLVESGGGLVQPGGSLRLSCAASGFTFSSYCMNWVRQAPGKGLEWV
SSVTGSAQRTNYADSVKGRFTISADTSKNTAYLQMNSLRAEDTAVYYCARNTGFETL
DNWGQGTLVTVSS (SEQ ID NO: 193)
>14H4. VH 
EVQLVESGGGLVQPGGSLRLSCAASGFSFSKFWMHWVRQAPGKGLEWV
AFMAHDESNKNYADSVKGRFTISADTSKNTAYLQMNSLRAEDTAVYYCARDKVCNT
FDIWGQGTLVTVSS (SEQ ID NO: 194)
>14E6. VH 
EVQLVESGGGLVQPGGSLRLSCAASGFTFSNFAMHWVRQAPGKGLEYV
SAISGGNGGTYYADSAKGRFTISADTSKNTAYLQMNSLRAEDTAVYYCAREGNYCL
DYWGQGTLVTVSS (SEQ ID NO: 195)
>14C7. VH 
EVQLVESGGGLVQPGGSLRLSCAASGFTFSSFSMSWVRQAPGKGLEWVA
NIKEDGSDKYYVDSVTGRFTISADTSKNTAYLQMNSLRAEDTAVYYCARDWGAAAV
LHHWGQGTLVTVSS (SEQ ID NO: 196)
>14B8. VH

EVQLVESGGGLVQPGGSLRLSCAASGFTFSGHWMHWVRQAPGKGLEW
VAVISSDGSYKSYADSVKGRFTISADTSKNTAYLQMNSLRAEDTAVYYCASRTVAGTL
DYWGQGTLVTVSS (SEQ ID NO: 197)

### 3. Inhibitory Activity of Ab-ASF Against Na_{V}1.2

The inhibitory activity of the expressed and prepared Ab-ASF molecules against Na_{V}1.2 was evaluated. Due to the large number of Ab-ASF molecules, inhibitory activities of Ab-ASF against Na_{V}1.2 were first detected at 10 nM and 100 nM. Specifically, HEK293-Na_{V}1.2 cells were incubated with varying concentrations of Ab-ASF for 15 min at room temperature prior to the inhibitory activity test. Data points are presented as mean±SD, n = 2-4, and curves were fitted and plotted using GraphPad. The test results are shown in FIG. 31A. Except for H3-PEG8-ASF, all Ab-ASF molecules exhibited certain inhibitory activity against Na_{V}1.2 at 100 nM. Ab-PEG8-ASF showed weaker inhibitory activity, likely attributable to the reduced activity of ASF-PEG8-DBCO. Among the Ab-ASF molecules, 14H4-PEG3-ASF and 14E6-PEG3-ASF displayed the strongest inhibitory activity, achieving near-complete inhibition at 100 nM. Therefore, concentration-response inhibition experiments were conducted for 14H4-PEG3-ASF and 14E6-PEG3-ASF, and the resulting inhibition curves are shown in FIG. 31B. Curve fitting yielded IC₅₀ values of 2.731 nM for 14H4-PEG3-ASF and 5.024 nM for 14E6-PEG3-ASF. By comparison, the IC₅₀ of the ASF-PEG3-DBCO small molecule against Nav1.2 was 7.384 nM. These results demonstrate that competitive library screening targeting Na_{V}1.2 yielded Ab-PEG3-ASF with slightly stronger inhibitory activity relative to the ASF small molecule.

### 4. Specificity of Ab-ASF Inhibitory Activity

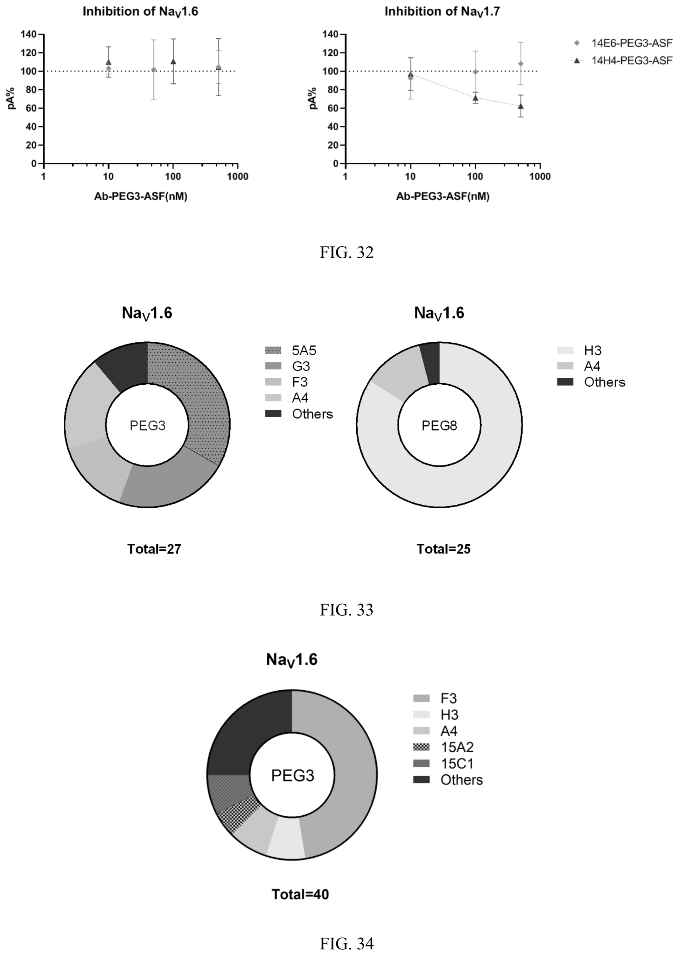

To assess the selectivity of Na_{V}1.2-targeting Ab-ASF molecules, the two Ab-ASF molecules exhibiting the strongest inhibitory activity against Na_{V}1.2 were tested for their inhibitory activity against Na_{V}1.7 and Na_{V}1.6. The inhibitory activities of Ab-ASF molecules against Na_{V}1.6 and Na_{V}1.7 were detected in the experiment at concentrations of 10 nM, 100 nM, and 500 nM. HEK293-Na_{V}1.6 and HEK293-Na_{V}1.7 cells were incubated with corresponding concentrations of Ab-PEG3-ASF for 15 min at room temperature prior to the inhibitory activity test, at concentrations of 10 nM, 100 nM, and 500 nM. Data points are presented as mean ± SD, n = 2-4. The detection results are shown in FIG. 32 that neither two Ab-ASF exhibited inhibition of Na_{V}1.6. Cell-based inhibition data for Na_{V}1.7 reveal that 14E6-PEG3-ASF exhibited no inhibitory activity against Na_{V}1.7, whereas 14H4-PEG3-ASF showed modest inhibitory activity against Nav1.7, with an inhibition rate of about 30% at 100 nM and about 40% at 500 nM. Compared with the ASF-PEG3-DBCO small molecule, Ab-PEG3-ASF exhibited markedly reduced inhibitory activity against Na_{V}1.7, indicating that the selectivity of Ab-PEG3-ASF for NaV 1.2 was significantly improved.

In conclusion, competitive library screening targeting Nav1.2 verified that this strategy effectively eliminates clones binding non-specifically to off-target proteins during selection, thereby increasing the likelihood of identifying target-specific clones. Using this screening strategy, two Ab-PEG3-ASF molecules with superior inhibitory activity against Na_{V}1.2 relative to ASF-PEG3-DBCO were successfully identified. Neither of the two Ab-PEG3-ASF molecules showed inhibitory activity against Na_{V}1.6; 14E6-PEG3-ASF also showed no inhibitory activity against Na_{V}1.7, while 14H4-PEG3-ASF exhibited weak inhibitory activity against Na_{V}1.7, with an IC₅₀ greater than 500 nM as determined by curve fitting. These results demonstrate that smacAbs with Nav1.2-selective inhibitory activity were obtained via competitive library screening in the experiment, indicating that smacAb library screening can reverse the preference of the ASF small molecule for Na_{V}1.7 and yield selective functional antibodies targeting non-dominant targets of the small molecule. Moreover, these results confirme that the smacAb and smacAb library screening strategies are also applicable to the screening of inhibitory antibodies targeting Na_{V}1.2, which further validates the utility of this strategy for functional antibody discovery against complex membrane protein targets.

### 5. Screening and Identification of smacAbs Targeting Na_{V}1.6

Fab-ASF Library Screening Targeting Na_{V}1.6: Screening of smacAbs targeting Na_{V}1.6 was also performed using two libraries: Fab-PEG3-ASF and Fab-PEG8-ASF. HEK293-Na_{V}1.6 overexpressing cells were used as target cells. For non-competitive screening, HEK293 cells were used as negative control cells, whereas for competitive library screening, HEK293-Na_{V}1.2 and HEK293-Na_{V}1.7 cells were used as negative control cells, each subjected to four rounds of screening. Monoclonal colonies were randomly selected from the fourth round of screening for analysis of clone enrichment, followed by expression of selected clones as smacAbs and evaluation of inhibitory activity and selectivity.

Phages eluted in the fourth round of the Na_{V}1.6-targeted non-competitive library screening were used to infect TG1 cells, plated and grown into monoclonal colonies. Single clones were randomly selected for Sanger sequencing. Fab variable region gene sequences were translated into amino acid sequences and aligned, and the frequency of sequence recurrence was analyzed. The statistical results of clone enrichment from non-competitive library screening are shown in FIG. 33. Three of the four recurrent sequences obtained by sequencing from the PEG3 screening group were Na_{V}1.7 selective clones, and only one unique clone was left, 5A5. In the PEG8 screening group, only two duplicate clones were detected, and both were Nav1.7-selective clones. These results indicate that Nav1.7-selective clones were highly enriched in the non-competitive library screening output targeting Na_{V}1.6. Therefore, competitive library screening was attempted to observe whether it could reduce the prevalence of Na_{V}1.7-selective clones.

To obtain smacAbs with Na_{V}1.6-selective inhibitory activity, competitive library screen targeting Na_{V}1.6 was performed. Given the weak inhibitory activity of ASF-PEG8-DBCO against Nav1.6 and the observation that the novel clone enriched in the non-competitive screening appeared in the screening output of PEG3 library, the competitive library screening experiment targeting Na_{V}1.6 was conducted using the Fab-PEG3-ASF library. Monoclonal colonies from the competitive library screening output were randomly selected for Sanger sequencing, and the variable region sequences of Fab were analyzed for analysis of clone enrichment frequency. The statistical results of sequence enrichment are shown in FIG. 34. A total of five recurrent sequences were identified in the library screening output: three clones, F3, H3, and A4, were Na_{V}1.7-selective clones, and two novel recurrent sequences, 15A2 and 15C1, were also identified.

Clone enrichment results of non-competitive screening and competitive screening targeting Na_{V}1.6 were analyzed to explore whether competitive screening was conducive to enrichment of Na_{V}1.6-selective clones. Via Fab-PEG3-ASF library screening, three Na_{V}1.7-selective clones, F3, G3, and A4, were observed in the non-competitive screening output, while three Na_{V}1.7-selective clones, F3, H3, and A4, were observed in the competitive screening output. These results show that, during library screening targeting Na_{V}1.6, the competitive screening strategy led to only minor changes in the repertoire of enriched Nav1.7-selective clones and did not significantly reduce the diversity of Na_{V}1.7-selective clones in the screening output. Overall, when the library competitive screening strategy was applied to the library screen targeting Nav1.6, the observed results were not as expected. This may be attributable to substantial differences between the ASF-binding pocket on Na_{V}1.6 and those on Na_{V}1.2 and Nav1.7, preventing Na_{V}1.2 and Na_{V}1.7 from exerting competitive binding effects during the screening process. In addition, the weak affinity of ASF for Nav1.6 may result in a higher proportion of non-target clones in the screening outputs, necessitating an increase in the number of clones subjected to activity test to acquire smacAbs with selective inhibitory activity.

Therefore, clones were selected from the screening outputs of the two screening strategies based on the recurrent sequence priority principle for subsequent experiments. First, duplicate clones 5A5, 15C1, and 15A2 were selected (wherein the VL CDR3 sequences are shown in SEQ ID NO: 5 to SEQ ID NO: 29; the VL CDR1-2 sequences are listed in Table 12; and the VH CDR1-3 sequences are listed in Table 13). Subsequently, three clones of non-recurrent sequences, including 15F6, 15G4, and 15D3, were also selected based on comprehensive assessment of sequence similarity among non-recurrent sequences (wherein the VL CDR3 sequences are show in SEQ ID NO: 5 to SEQ ID NO: 29; the VL CDR1-2 sequences are listed in Table 12; and the VH CDR1-3 sequences are listed in Table 13) for downstream experimentation.

**Table 12: VL CDR1-2**

| Antibody No. | VL CDR1 | VL CDR2 |
|---|---|---|
| F3 | RASQVIGNDLG (SEQ ID NO: 93) | KLLIYDVSNVQTGVPS (SEQ ID NO: 101) |
| H3 | RASQSISSWLA (SEQ ID NO: 95) | NLLMYAASSLQSGVPS (SEQ ID NO: 103) |
| A4 | QASQDIRNDLN (SEQ ID NO: 96) | KSLIYAASRLQSGVPS (SEQ ID NO: 104) |
| 15F6 | QASQDISHYLN (SEQ ID NO: 198) | KLLIHRVSSLESGVPS (SEQ ID NO: 204) |
| 15D3 | RASQGISSDLG (SEQ ID NO: 199) | KLLIYDVSNVQTGVPS (SEQ ID NO: 205) |
| 15C1 | RASETIGSWLA (SEQ ID NO: 200) | KLLIYKSTTVEGGVSS (SEQ ID NO: 206) |
| 15G4 | RASQNINRHLN (SEQ ID NO: 201) | KLLIYDVSNVQTGVPS (SEQ ID NO: 207) |
| 15A2 | QASQDIGKFLN (SEQ ID NO: 202) | KLLISATSNLQWGVPS (SEQ ID NO: 208) |
| 5A5 | RASQSISDWLA (SEQ ID NO: 203) | KLLIYAASSLQSGVPS (SEQ ID NO: 209) |

**Table 13: VH CDR1-3**

| Antibody No. | VH CDR1 | VH CDR2 | VH CDR3 |
|---|---|---|---|
| F3 | FTFSGHWMH (SEQ ID NO: 109) | WVASISDSSSYIYYADSVR GRF (SEQ ID NO: 115) | ARDYEGATRAADY (SEQ ID NO: 124) |
| H3 | FTFSDYWIS (SEQ ID NO: 111) | WIGNVYYSGNAYYNPSLE SRV (SEQ ID NO: 117) | ARNTGFETLDN (SEQ ID NO: 126) |
| A4 | FTFSGHWMH (SEQ ID NO: 109) | WVSGISGSGGSTYYADSV KGRF (SEQ ID NO: 118) | ARELTMATTTDAFDI (SEQ ID NO: 127) |
| 15F6 | FSFSTYAMH (SEQ ID NO: 210) | WVANIDGDGGVTNYAGS VKGRF (SEQ ID NO: 216) | ARDRGWLQFDY (SEQ ID NO: 222) |
| 15D3 | FTFSGHWMH (SEQ ID NO: 211) | WVSAISGSGGSTYYADSV KGRF (SEQ ID NO: 217) | AREVTMVRGVTDAFDI (SEQ ID NO: 223) |
| 15C1 | FTFTSYAMA (SEQ ID NO: 212) | WVSSISNGGSVIYYANSV KGRF (SEQ ID NO: 218) | ARDRGFLQFDY (SEQ ID NO: 224) |
| 15G4 | FTFDEYIMH (SEQ ID NO: 213) | YVSAISSNGGSTYYANSV KGRF (SEQ ID NO: 219) | ARDRGFLQFDY (SEQ ID NO: 225) |
| 15A2 | FSFSNFAMT (SEQ ID NO: 214) | WVSSISNGGSVIYYANSV KGRF (SEQ ID NO: 220) | ARDRGFLQFDY (SEQ ID NO: 226) |
| 5A5 | FPFSGHWMH (SEQ ID NO: 215) | WVSGISWNGGTTGYADSV KGRF (SEQ ID NO: 221) | ARDDYGGDSFDS (SEQ ID NO: 227) |

The amino acid sequences of the heavy chain variable region and light chain variable region of each antibody are as follows:
>15F6. VL 
DIQMTQSPSSLSASVGDRVTITCQASQDISHYLNWYQQKPGKAPKLLIHR
VSSLESGVPSRFSGSRSGTDFTLTISSLQPEDFATYYCQQ*YTTPPTFGQGTKLEI (SEQ
ID NO: 228)
>15D3. VL 
DIQMTQSPSSLSASVGDRVTITCRASQGISSDLGWYQQKPGKAPKLLIYD
VSNVQTGVPSRFSGSRSGTDFTLTISSLQPEDFATYYCQQ*YTTPPTFGQGTKLEI (SEQ
ID NO: 229)
>15C1. VL 
DIQMTQSPSSLSASVGDRVTITCRASETIGSWLAWYQQKPGKAPKLLIYK
STTVEGGVSSRFSGSRSGTDFTLTISSLQPEDFATYYCQQ*YTTPPTFGQGTKLEI (SEQ
ID NO: 230)
>15G4. VL 
DIQMTQSPSSLSASVGDRVTITCRASQNINRHLNWYQQKPGKAPKLLIYD
VSNVQTGVPSRFSGSRSGTDFTLTISSLQPEDFATYYCQQ*YTTPPTFGQGTKLEI (SEQ
ID NO: 231)
>15A2. VL 
DIQMTQSPSSLSASVGDRVTITCQASQDIGKFLNWYQQKPGKAPKLLISA
TSNLQWGVPSRFSGSRSGTDFTLTISSLQPEDFATYYCQQ*YTTPPTFGQGTKLEI (SEQ
ID NO: 232)
>5A5. VL 
DIQMTQSPSSLSASVGDRVTITCRASQSISDWLAWYQQKPGKAPKLLIYA
ASSLQSGVPSRFSGSRSGTDFTLTISSLQPEDFATYYCQQ*YTTPPTFGQGTKLEI (SEQ
ID NO: 233)
>15F6. VH 
EVQLVESGGGLVQPGGSLRLSCAASGFSFSTYAMHWVRQAPGKGLEWV
ANIDGDGGVTNYAGSVKGRFTISADTSKNTAYLQMNSLRAEDTAVYYCARDRGWLQ
FDYWGQGTLVTVSS (SEQ ID NO: 234)
>15D3. VH 
EVQLVESGGGLVQPGGSLRLSCAASGFTFSGHWMHWVRQAPGKGLEW
VSAISGSGGSTYYADSVKGRFTISADTSKNTAYLQMNSLRAEDTAVYYCAREVTMVR
GVTDAFDIWGQGTLVTVSS (SEQ ID NO: 235)
>15C1. VH 
EVQLVESGGGLVQPGGSLRLSCAASGFTFTSYAMAWVRQAPGKGLEWV
SSISNGGSVIYYANSVKGRFTISRDNSKKTLYLQMNGLRVEDTAVYYCARDRGFLQFD
YWGQGTLVTVSS (SEQ ID NO: 236)
>15G4. VH EVQLVESGGGLVQPGGSLRLSCAASGFTFDEYIMHWVRQAPGKGLEYVS 
AISSNGGSTYYANSVKGRFTISADTSKNTAYLQMNSLRAEDTAVYYCARDRGFLQFD
YWGQGTLVTVSS (SEQ ID NO: 237)
>15A2. VH 
EVQLVESGGGLVQPGGSLRLSCAASGFSFSNFAMTWVRQAPGKGLEWV
SSISNGGSVIYYANSVKGRFTISRDNSKKTLYLQMNSLRAEDTAVYYCARDRGFLQFD
YWGQGTLVTVSS (SEQ ID NO: 238)
>5A5. VH

EVQLVESGGGLVQPGGSLRLSCAASGFPFSGHWMHWVRQAPGKGLEW
VSGISWNGGTTGYADSVKGRFTISRDNAKNSVYLQMNSLRAEDTAVYYCARDDYGG
DSFDSWGQGTLVTVSS (SEQ ID NO: 239)

### 6. Inhibitory Activity of Ab-ASF Against Na_{V}1.6

To evaluate the inhibitory activity of the screened Ab-ASF against Na_{V}1.6, in the experiment, the inhibitory effects of each Ab-PEG3-ASF against Nav1.6 was first detected at concentrations of 10 nM and 100 nM. The detection results are shown in FIG. 35A. All clones exhibited a certain degree of inhibitory activity against Na_{V}1.6 at a concentration of 100 nM. Among them, 5A5-PEG3-ASF and 15G4-PEG3-ASF displayed stronger inhibitory activity, with IC₅₀ values falling within the range of 10 nM to 100 nM. In the experiment, 5A5-PEG3-ASF, which exhibited the strongest inhibitory activity, was selected for concentration-response analysis. The resulting inhibition curve is shown in FIG. 35B, and the IC₅₀ of 5A5-PEG3-ASF was 28.63 nM. By comparison, the small-molecule inhibitor ASF-PEG3-DBCO inhibited Nav1.6 with an IC₅₀ of 683.2 nM. Thus, the inhibitory activity of 5A5-PEG3-ASF was improved by more than 20-fold than that of ASF-PEG3-DBCO.

### 7. Specificity of Ab-ASF Inhibitory Activity

To verify the specificity of the inhibitory activity of 5A5-PEG3-DBCO, its inhibitory activity against Nav1.2 and Na_{V}1.7 was tested. Specifically, HEK293-Nav1.2 or HEK293-Na_{V}1.7 cells were incubated with various concentrations of Ab-ASF for 15 min at room temperature prior to the inhibitory activity assay. Data points are presented as mean ± SD, n = 2-4. The results are shown in FIG. 36. At the highest tested concentration of 500 nM in the experiment, 5A5-PEG3-ASF showed no inhibitory activity against Nav1.2 or Na_{V}1.7, indicating that 5A5-PEG3-ASF exhibits selective inhibitory activity against Na_{V}1.6.

In conclusion, through smacAb design and smacAb library screening strategies, smacAb with selective inhibitory activity against Na_{V}1.6 was obtained, further demonstrating that smacAb library screening can reverse the preference of the ASF small molecule for Na_{V}1.7 and yield selective functional antibodies targeting non-dominant targets of the small molecule. Meanwhile, these results confirm that smacAb and the smacAb library screening strategies are also applicable for screening inhibitory antibodies targeting Na_{V}1.6, which further validates the utility of this approach for functional antibody screening against complex membrane protein targets.

### Example 7: Screening and identification of smacAbs Using A2aR as a Target Protein

The Fab-ZP3 library was incubated with biotinylated target cells and negative cells. Target cells were isolated by magnetic bead sorting, AND THE bound phages were eluted by thrombin enzymatic digestion. After amplification, the Fab-ZP3 library was re-prepared for the next round of screening.

### 1. Synthesis and Activity Characterization of the ZP3 Small Molecule

The A2aR-inhibitory small molecule ZM241385 was selected as the parent core scaffold. A flexible PEG3 linker was inserted between ZM241385 and the conjugation group DBCO to synthesize the small-molecule inhibitor ZP3, whose chemical structure is shown as follows:

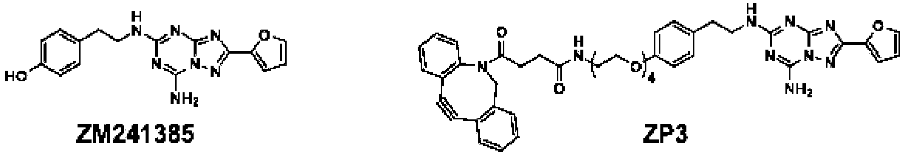

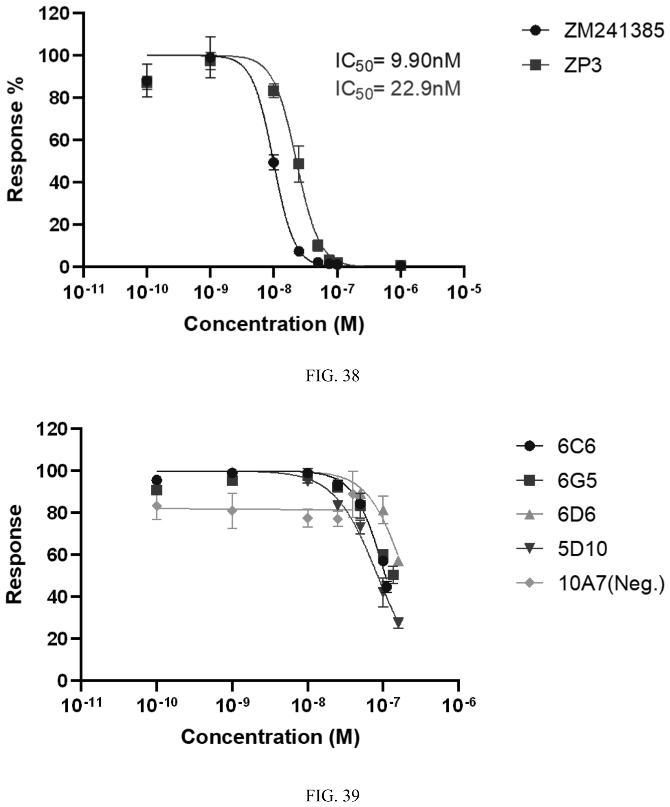

Based on this, the GloSensor^{™} cAMP assay was first employed to verify whether ZP3 retained A2aR inhibitory activity. Specifically, HEK293-A2aR cells were seeded into white 96-well plates at 15,000 cells/well and cultured overnight. Cells were transfected with the pGloSensor^{™}-22F cAMP plasmid (100 ng/well) using Lipofectamine 3000. After 24 h, the medium was replaced with assay medium (10% FBS, 88% culture medium, and 2% GloSensor^{™} cAMP Reagent) and equilibrated at room temperature for 2 h. Cells were incubated with varying concentrations of ZP3 for 30 min at room temperature, afterwhich the bioluminescence results of the samples were measured and the inhibitory activity was calculated. The inhibitory activity results of ZP3 are shown in FIG. 38. ZP3 exhibited inhibitory activity against A2aR with an IC₅₀ of 22.9 nM, slightly weaker than the parent small molecule ZM241385. Data points are presented as mean ± SD, n = 3, and curves were fitted and plotted using GraphPad.

### 2. Preparation and Screening of the Fab-ZP3 Library

In the experiment, the Fab-ZP3 library was prepared by conjugating ZP3 to the Fab-UAA library and used for A2aR-targeted screening. The screening protocol was identical to that used for A2aR-targeted selection. In the first round of screening, only A2aR-overexpressing cells were used. In subsequent rounds, HEK293 cells were added as negative control cells at a six-fold excess.

### 1) Screening of the Fab-ZP3 Library Targeting A2aR

A total of four rounds of screening were performed in this example. After screening, forty-eight clones were randomly selected from the fourth-round output for Sanger sequencing analysis. Two recurrent sequences were enriched in the fourth-round output, including 6G5, 5C9, and 6A6. In addition, clones with only light-chain repetition (5D10 and 5F9) and a clone with only heavy-chain repetition (6D6) were also selected for expression and activity testing. The VL CDR3 sequences are shown in SEQ ID NO: 5 to SEQ ID NO: 29, VL CDR1-2 sequences are listed in Table 14, and VH CDR1-3 sequences are listed in Table 15.

**Table 14: VL CDR1-2**

| Antibody No. | VL CDR1 | VL CDR2 |
|---|---|---|
| 6G5 | QASQDIRNSLN (SEQ ID NO: 247) | KLLIKYASQSISGVPS (SEQ ID NO: 251) |
| 5C9 | RASQSISRALA (SEQ ID NO: 248) | KLLIYKASSLQSGVPS (SEQ ID NO: 252) |
| 6A6 | QASQSISRYLS (SEQ ID NO: 249) | KLLIYKTSRLESGVPS (SEQ ID NO: 253) |
| 5D10 | QASQDIRNSLN (SEQ ID NO: 247) | KLLIYAASTLQSGVPS (SEQ ID NO: 254) |
| 5F9 | QASQDIRNSLN (SEQ ID NO: 247) | KLLIYAASTLQSGVPS (SEQ ID NO: 254) |
| 6D6 | RASQNIGTSLH (SEQ ID NO: 250) | KLLISRASSLQSGVPS (SEQ ID NO: 255) |
| 6C6 | RASQSISSWLA (SEQ ID NO: 30) | KLLIYKTSNLESGVPS (SEQ ID NO: 284) |

**Table 15: VH CDR1-3**

| Antibody No. | VH CDR1 | VH CDR2 | VH CDR3 |
|---|---|---|---|
| 6G5 | FTFSGHWMH (SEQ | WIGTVSYNGRTYYNPSLK | ARGASGRTYNNFFDP |
| | ID NO: 256) | SRV (SEQ ID NO: 261) | (SEQ ID NO: 267) |
| 5C9 | FTVGSNSMT (SEQ ID NO: 257) | WVSSISGTGGRTYYADSV KGRF (SEQ ID NO: 262) | ARILPVRGASGQWAR KNWFDP (SEQ ID NO: 268) |
| 6A6 | FTFSGHWMH (SEQ ID NO: 256) | WVSSITGDSNYRYYADSV KGRF (SEQ ID NO: 263) | ARDRSGSLRAFDY (SEQ ID NO: 269) |
| 5D10 | FTFGSFWMS (SEQ ID NO: 258) | WVSSISNGGSVIYYANSV KGRF (SEQ ID NO: 264) | ARGLRRASGPFDY (SEQ ID NO: 270) |
| 5F9 | FRFSSYSMN (SEQ ID NO: 259) | WVSGISGSGGSTYYADPV KGRF (SEQ ID NO: 265) | ARGGRGPTTPFDY (SEQ ID NO: 271) |
| 6D6 | FTFSNHAMH (SEQ ID NO: 260) | WVSAITGNGRSKYYADS VKGRF (SEQ ID NO: 266) | ARDRGWLQFDY (SEQ ID NO: 272) |
| 6C6 | FTFSNHAMH (SEQ ID NO: 260) | WVSAITGNGRSKYYADS VKGRF (SEQ ID NO: 266) | ARDRGWLQFDY (SEQ ID NO: 272) |

The amino acid sequences of the heavy chain variable region and light chain variable region of each antibody are as follows:
>6G5-VH 
EVQLVESGGGLVQPGGSLRLSCAASGFTFSGHWMHWVRQAPGKGLEWI
GTVSYNGRTYYNPSLKSRVTISADTSKNTAYLQMNSLRAEDTAVYYCARGASGRTYN
NFFDPWGQGTLVTVSS (SEQ ID NO: 273)
>5C9-VH 
EVQLVESGGGLVQPGGSLRLSCAASGFTVGSNSMTWVRQAPGKGLEWV
SSISGTGGRTYYADSVKGRFTISADTSKNTAYLQMNSLRAEDTAVYYCARILPVRGAS
GQWARKNWFDPWGQGTLVTVSS (SEQ ID NO: 274)
>6A6-VH 
EVQLVESGGGLVQPGGSLRLSCAASGFTFSGHWMHWVRQAPGKGLEW
VSSITGDSNYRYYADSVKGRFTISADTSKNTAYLQMNSLRAEDTAVYYCARDRSGSLR
AFDYWGQGTLVTVSS (SEQ ID NO: 275)
>5D10-VH 
EVQLVESGGGLVQPGGSLRLSCAASGFTFGSFWMSWVRQAPGKGLEWV
SSISNGGSVIYYANSVKGRFTISRDNSKKTLYLQMNSLRAEDTAVYYCARGLRRASGP
FDYWGQGTLVTVSS (SEQ ID NO: 276)
>5F9-VH 
EVQLVESGGGLVQPGGSLRLSCAASGFRFSSYSMNWVRQAPGKGLEWV
SGISGSGGSTYYADPVKGRFTISADTSKNTAYLQMNSLRAEDTAVYYCARGGRGPTTP
FDYWGQGTLVTVSS (SEQ ID NO: 277)
>6D6-VH 
EVQLVESGGGLVQPGGSLRLSCAASGFTFSNHAMHWVRQAPGKGLEWV
SAITGNGRSKYYADSVKGRFTISADTSKNTAYLQMNSLRAEDTAVYYCARDRGWLQF
DYWGQGTLVTVSS (SEQ ID NO: 278)
>6C6-VH

EVQLVESGGGLVQPGGSLRLSCAASGFTFSNHAMHWVRQAPGKGLEWV
SAITGNGRSKYYADSVKGRFTISADTSKNTAYLQMNSLRAEDTAVYYCARDRGWLQF
DYWGQGTLVTVSS (SEQ ID NO: 278)

Light chain variable region sequence:
>6G5-VL 
DIQMTQSPSSLSASVGDRVTITCQASQDIRNSLNWYQQKPGKAPKLLIKY
ASQSISGVPSRFSGSRSGTDFTLTISSLQPEDFATYYCQQ*YTTPPTFGQGTKLEI (SEQ
ID NO: 279)
>5C9-VL 
DIQMTQSPSSLSASVGDRVTITCRASQSISRALAWYQQKPGKAPKLLIYK
ASSLQSGVPSRFSGSRSGTDFTLTISSLQPEDFATYYCQQ*YTTPPTFGQGTKLEI (SEQ
ID NO: 280)
>6A6-VL 
DIQMTQSPSSLSASVGDRVTITCQASQSISRYLSWYQQKPGKAPKLLIYK
TSRLESGVPSRFSGSRSGTDFTLTISSLQPEDFATYYCQQ*YTTPPTFGQGTKLEI (SEQ
ID NO: 281)
>5D10-VL 
DIQMTQSPSSLSASVGDRVTITCQASQDIRNSLNWYQQKPGKAPKLLIYA
ASTLQSGVPSRFSGSRSGTDFTLTISSLQPEDFATYYCQQ*YTTPPTFGQGTKLEI (SEQ
ID NO: 282)
>5F9-VL 
DIQMTQSPSSLSASVGDRVTITCQASQDIRNSLNWYQQKPGKAPKLLIYA
ASTLQSGVPSRFSGSRSGTDFTLTISSLQPEDFATYYCQQ*YTTPPTFGQGTKLEI (SEQ
ID NO: 282)
>6D6-VL 
DIQMTQSPSSLSASVGDRVTITCRASQNIGTSLHWYQQKPGKAPKLLISR
ASSLQSGVPSRFSGSRSGTDFTLTISSLQPEDFATYYCQQ*YTTPPTFGQGTKLEI (SEQ
ID NO: 283)
>6C6-VL

DIQMTQSPSSLSASVGDRVTITCRASQSISSWLAWYQQKPGKAPKLLIYK
TSNLESGVPSRFSGSRSGTDFTLTISSLQPEDFATYYCQQ*YTTPPTFGQGTKLEI (SEQ
ID NO: 285)

### 2) Antibody Expression and Conjugation to ZP3

To evaluate the inhibitory activity of the screened smacAbs against A2aR, each clone was first expressed as a full-length IgG1 antibody. The Ab-UAA antibodies were then conjugated to ZP3 to prepare Ab-ZP3, which were tested for inhibitory activity and selectivity.

### 3. Inhibitory Activity of Ab-ZP3 Against A2aR

The inhibitory curves of Ab-ZP3 against A2aR are shown in FIG. 39, and the IC₅₀ values are listed in Table 14. The IC₅₀ of Ab-ZP3 for inhibiting A2aR ranged from 80 nM to 200 nM, which was slightly weaker than that of the small molecule ZP3. Data points are presented as mean ± SD; n = 3, and curves were fitted and plotted using GraphPad.

**Table 16: IC₅₀ of Ab-ZP3 for inhibition of A2aR**

| Ab-ZP3 | IC₅₀ ± SEM (nM) |
|---|---|
| 6C6-ZP3 | 107.4± 2.6 |
| 6G5-ZP3 | ND |
| 6D6-ZP3 | 196.1± 14.5 |
| 5D10-ZP3 | 84.9± 3.9 |

The experimental results demonstrate that among the clones selected from the Fab-ZP3 library screening based on the duplicate clone priority principle, three Ab-ZP3 molecules exhibited inhibitory activity against A2aR. Therefore, screening of the smacAb library enabled the identification of Ab-ZP3 molecules with inhibitory activity against A2aR, thereby validating the applicability of the smacAb design and library screening strategy for functional antibody screening targeting GPCRs.

The preferred embodiments of the present disclosure have been described in detail above. However, the present disclosure is not limited to the specific details of the above embodiments, and various simple modifications may be made within the scope of the technical concept of the present disclosure, all of which fall within the scope of protection of the present disclosure.

It should also be noted that the individual technical features described in the detailed description as described above may be combined in any suitable manner where no contradiction arises. To avoid unnecessary repetition, the present disclosure does not separately describe all possible combinations.

## Claims

1. An antibody derivative, being a ligand-anchored antibody smacAb, comprising:
an antibody or antigen-binding fragment thereof; and
a ligand, modified on the antibody or antigen-binding fragment thereof,
wherein the ligand is a small molecule compound,
wherein the antibody or antigen-binding fragment thereof is conjugated to the small molecule compound, and
wherein the small molecule compound and the antibody or antigen-binding fragment thereof jointly participate in binding to a target antigen.

2. The antibody derivative according to claim 1, wherein the small molecule compound is conjugated to a complementarity-determining region (CDR) of the antibody or antigen-binding fragment thereof;
preferably, the small molecule compound is conjugated to heavy chain CDR3 or light chain CDR3 of the antibody or antigen-binding fragment thereof; and
more preferably, the small molecule compound is conjugated to light chain CDR3 of the antibody or antigen-binding fragment thereof.

3. The antibody derivative according to claim 2, wherein the light chain CDR3 has a length of 3 to 20 amino acids.

4. The antibody derivative according to any one of claims 1 to 3, wherein the small molecule compound is conjugated to one amino acid residue in light chain CDR3 of the antibody or antigen-binding fragment thereof, preferably at position 1, 2, 3, 4, 5, 6, 7, 8, 9, 10, 11, 12, 13, 14, 15, 16, 17, 18, 19, or 20.

5. The antibody derivative according to any one of claims 2 to 4, wherein the light chain CDR3 comprises: QQ*YTTPPT (SEQ ID NO: 5), CQQ*YTTPPT (SEQ ID NO: 6), YCQQ*YTTPPT (SEQ ID NO: 7), YYCQQ*YTTPPT (SEQ ID NO: 8), TYYCQQ*YTTPPT (SEQ ID NO: 9), QQ*YTTPPTF (SEQ ID NO: 10), QQ*YTTPPTFG (SEQ ID NO: 11), QQ*YTTPPTFGQ (SEQ ID NO: 12), QQ*YTTPPTFGQG (SEQ ID NO: 13), CQQ*YTTPPTF (SEQ ID NO: 14), CQQ*YTTPPTFG (SEQ ID NO: 15), CQQ*YTTPPTFGQ (SEQ ID NO: 16), CQQ*YTTPPTFGQG (SEQ ID NO: 17), YCQQ*YTTPPTF (SEQ ID NO: 18), YCQQ*YTTPPTFG (SEQ ID NO: 19), YCQQ*YTTPPTFGQ (SEQ ID NO: 20), YCQQ*YTTPPTFGQG (SEQ ID NO: 21), YYCQQ*YTTPPTF (SEQ ID NO: 22), YYCQQ*YTTPPTFG (SEQ ID NO: 23), YYCQQ*YTTPPTFGQ (SEQ ID NO: 24), YYCQQ*YTTPPTFGQG (SEQ ID NO: 25), TYYCQQ*YTTPPTF (SEQ ID NO: 26), TYYCQQ*YTTPPTFG (SEQ ID NO: 27), TYYCQQ*YTTPPTFGQ (SEQ ID NO: 28), or TYYCQQ*YTTPPTFGQG (SEQ ID NO: 29); or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 5 to SEQ ID NO: 29, wherein * represents a natural amino acid or an unnatural amino acid and denotes a site where the small molecule compound is conjugated to the antibody or antigen-binding fragment thereof.

6. The antibody derivative according to any one of claims 1 to 5, wherein the antibody or antigen-binding fragment thereof is site-specifically conjugated to the small molecule compound, preferably by means of a Michael addition reaction or a Huisgen reaction.

7. The antibody derivative according to any one of claims 1 to 6, wherein the antibody or antigen-binding fragment thereof is conjugated to the small molecule compound via a linker group;
preferably, the linker group is a linker peptide, a PEG moiety, 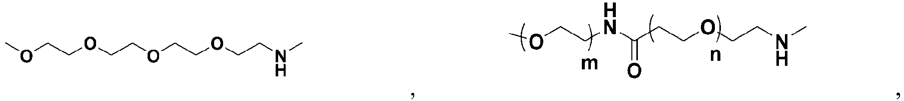 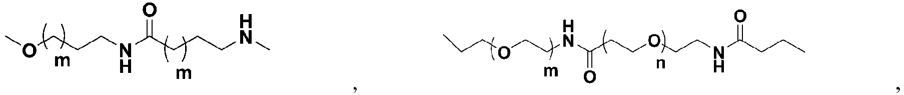 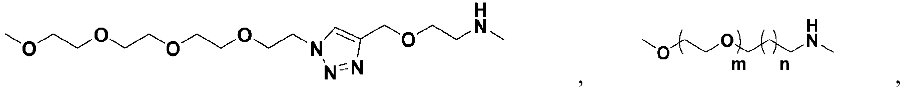 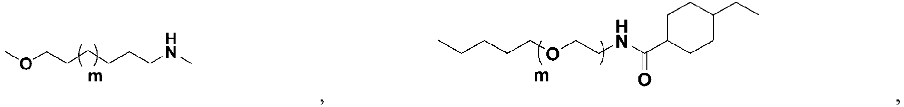 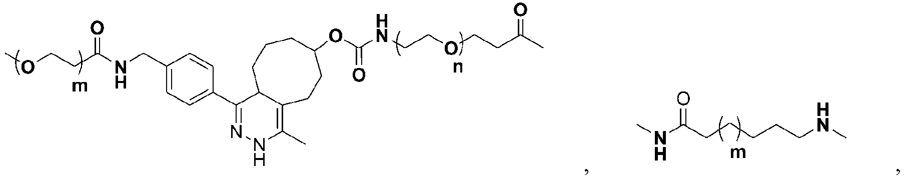 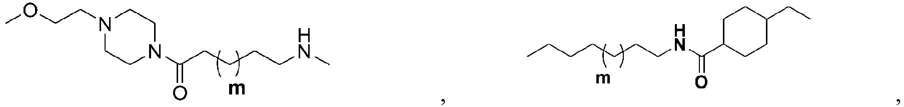 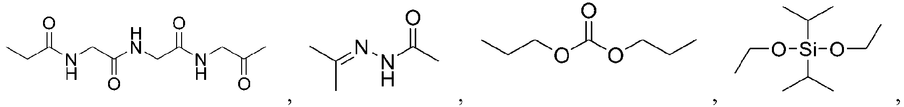 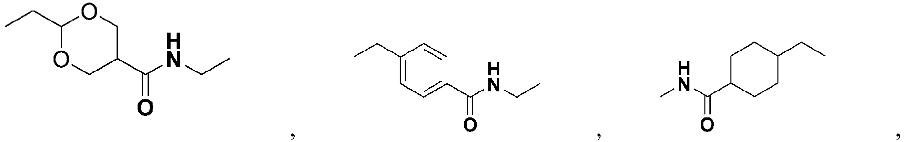 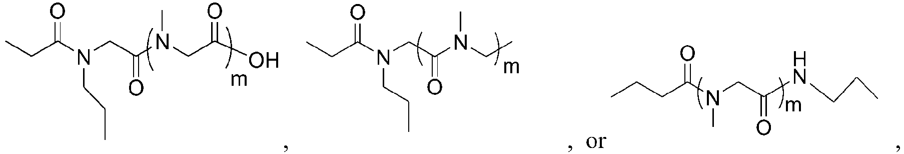 where m and n are each independently an integer selected from 1 to 20;
preferably, the linker peptide comprises: a sequence as set forth in any one of SEQ ID NO: 98, 106, 113, 121, 130, 139, 148, and 240 to 246; or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 98, 106, 113, 121, 130, 139, 148, and 240 to 246.

8. The antibody derivative according to any one of claims 1 to 7, wherein the target antigen is a target protein, peptide, lipid, polysaccharide, or a polynucleotide;
preferably, the target protein is selected from a membrane protein, a membrane transport protein, a receptor protein, a complex formed by a membrane protein and a membrane protein, or a complex formed by a membrane protein and another protein;
more preferably, the membrane protein is a peripheral membrane protein, an integral membrane protein, or a lipid-anchored protein;
more preferably, the membrane transport protein is a carrier protein or a channel protein, wherein the channel protein is preferably a sodium ion channel protein, a calcium ion channel protein, a chloride ion channel protein, a potassium ion channel protein, an Ach receptor channel, or an amino acid receptor channel; and
more preferably, the receptor protein is an intracellular receptor, a cell surface receptor, an ion channel receptor, a G-protein-coupled receptor (GPCR), or an enzyme-linked receptor.

9. The antibody derivative according to any one of claims 1 to 8, wherein the antibody or antigen-binding fragment thereof is Fab, Fv, Fd, Fab', Fab'-SH, F(ab')2, a nanobody, a single-domain antibody, or a single-chain antibody.

10. The antibody derivative according to any one of claims 1 to 9, wherein the antibody or antigen-binding fragment thereof is a CD73 antibody or antigen-binding fragment thereof, the CD73 antibody or antigen-binding fragment thereof comprising a heavy chain variable region and/or a light chain variable region, wherein the light chain variable region comprises VLCDR1, VLCDR2, and VLCDR3, and wherein the heavy chain variable region comprises VHCDR1, VHCDR2, and VHCDR3, wherein:
the VLCDR1 comprises the sequence as set forth in any one of SEQ ID NO: 30 to SEQ ID NO: 39, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 30 to SEQ ID NO: 39;
the VLCDR2 comprises the sequence as set forth in any one of SEQ ID NO: 40 to SEQ ID NO: 45, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 40 to SEQ ID NO: 45;
the VLCDR3 comprises the sequence as set forth in any one of SEQ ID NO: 5 to SEQ ID NO: 29, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 5 to SEQ ID NO: 29;
the VHCDR1 comprises the sequence as set forth in any one of SEQ ID NO: 46 to SEQ ID NO: 55, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 46 to SEQ ID NO: 55;
the VHCDR2 comprises the sequence as set forth in any one of SEQ ID NO: 56 to SEQ ID NO: 64, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 56 to SEQ ID NO: 64; and
the VHCDR3 comprises the sequence as set forth in any one of SEQ ID NO: 65 to SEQ ID NO: 72, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 65 to SEQ ID NO: 72.

11. The antibody derivative according to claim 10, wherein VLCDR1 and VLCDR2 comprise:
| Antibody No. | VL CDR1 | VL CDR2 |
|---|---|---|
| 10D8 | RASQSISSWLA (SEQ ID NO: 30) | KLLIYDVSNVQTGVPS (SEQ ID NO: 40) |
| 10A7 | RASQGISRWLA (SEQ ID NO: 31) | NLLIYGASTLQSGVPP (SEQ ID NO: 41) |
| 4C10 | RATQAISNYLA (SEQ ID NO: 32) | KLLIYDVSNVQTGVPS (SEQ ID NO: 40) |
| 5E12 | RTSQGITDPLN (SEQ ID NO: 33) | KLLIYKTSSLESGVPS (SEQ ID NO: 42) |
| 5H12 | RASQGLSSWLA (SEQ ID NO: 34) | KLLIYDASNLETGVPS (SEQ ID NO: 43) |
| 6D11 | RASQNIGTYLA (SEQ ID NO: 35) | KLLIYDASNLETGVPS (SEQ ID NO: 43) |
| ABN3-2B | RASQGINNYLA (SEQ ID NO: 36) | KLLIYDASNLETGVPS (SEQ ID NO: 43) |
| ABN3-2G | QASHDISKYLN (SEQ ID NO: 37) | KLLIYDASNLETGVPS (SEQ ID NO: 43) |
| ABN3-2A | RASETIGSWLA (SEQ ID NO: 38) | NLLIYAASTLQSGVSS (SEQ ID NO: 44) |
| 10F8 | QASQDISNYLN (SEQ ID NO: 39) | NLLIYAASTLQSGVPS (SEQ ID NO: 45) |
and
wherein VHCDR1, VHCDR2, and VHCDR3 comprise:
| Antibody No. | VH CDR1 | VH CDR2 | VH CDR3 |
|---|---|---|---|
| ABN3-2A | FTLSTYAMH (SEQ ID NO: 46) | WLARIDWDDDKYYSTS LKTRL (SEQ ID NO: 56) | ARGRDFGEPPYVRAFDI (SEQ ID NO: 65) |
| ABN3-2B | FTFSSYWMH (SEQ ID NO: 47) | WVSAVSGSGDSTYYADS VKGRF (SEQ ID NO: 57) | AREGDWGLDY (SEQ ID NO: 66) |
| ABN3-2G | ITFGSYTMS (SEQ ID NO: 48) | WIGTVHYTGKTFYSPSL KSRV (SEQ ID NO: 58) | ARGRDFGEPPYVRAFDI (SEQ ID NO: 65) |
| 4C10 | FTFSSYWMN (SEQ ID NO: 49) | WVANIKQDGSEKYYVD SVKGRF (SEQ ID NO: 59) | VTGSYRYAY (SEQ ID NO: 67) |
| 5E12 | FTFSNYAMH (SEQ ID NO: 50) | WVSAISGSGGSTYYADS VKGRF (SEQ ID NO: 60) | ARGGGNYYDDY (SEQ ID NO: 68) |
| 5H12 | FTFSHYAMH (SEQ ID NO: 51) | WIGNIYYSGSTYYNPSL KSRV (SEQ ID NO: 61) | AKGHGSGSYFPYYFDY (SEQ ID NO: 69) |
| 6D11 | FTFSGHWMH (SEQ ID NO: 52) | WIGSIYYSGSTYYNPSLK SRV (SEQ ID NO: 62) | ARDRGWLQFDY (SEQ ID NO: 70) |
| 10A7 | FAFSSYSMS (SEQ ID NO: 53) | WIGSIYYSGSTYYNPSLK SRV (SEQ ID NO: 62) | ARNSGFETLDY (SEQ ID NO: 71) |
| 10D8 | FTFSTYAIH (SEQ ID NO: 54) | WVSSISGSGGGGGTYYA DSVKGRF (SEQ ID NO: 63) | ARDRGFLQFDY (SEQ ID NO: 72) |
| 10F8 | FTFSSYGMH (SEQ ID NO: 55) | WVAAISYDGSNELYADS VKGRF (SEQ ID NO: 64) | ARDRGWLQFDY (SEQ ID NO: 70) |

12. The antibody derivative according to claim 10 or 11, wherein:
the heavy chain variable region comprises the sequence as set forth in any one of SEQ ID NO: 73 to SEQ ID NO: 82, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 73 to SEQ ID NO: 82; and
the light chain variable region comprises the sequence as set forth in any one of SEQ ID NO: 83 to SEQ ID NO: 92, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 83 to SEQ ID NO: 92.

13. The antibody derivative according to any one of claims 1 to 12, wherein the antibody or antigen-binding fragment thereof is a CD73 antibody or antigen-binding fragment thereof, and the small molecule compound is PSB12379 having a structure of:

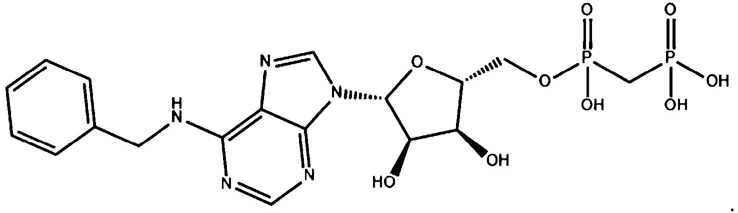

14. The antibody derivative according to any one of claims 1 to 9, wherein the antibody or antigen-binding fragment thereof is a Naᵥ1.7 antibody or antigen-binding fragment thereof, the Naᵥ1.7 antibody or antigen-binding fragment thereof comprising a heavy chain variable region and/or a light chain variable region, wherein the light chain variable region comprises VLCDR1, VLCDR2, and VLCDR3, and wherein the heavy chain variable region comprises VHCDR1, VHCDR2, and VHCDR3, wherein:
the VLCDR1 comprises the sequence as set forth in any one of SEQ ID NO: 93 to 97 and 99 to 100, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 93 to 97 and 99 to 100;
the VLCDR2 comprises the sequence as set forth in any one of SEQ ID NO: 101 to 105 and 107 to 108, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 101 to 105 and 107 to 108;
the VLCDR3 comprises the amino acid sequence as set forth in any one of SEQ ID NO: 5 to SEQ ID NO: 29, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 5 to SEQ ID NO: 29;
the VHCDR1 comprises the sequence as set forth in any one of SEQ ID NO: 109 to 112 and 114, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 109 to 112 and 114;
the VHCDR2 comprises the sequence as set forth in any one of SEQ ID NO: 115 to 120 and 122 to 123, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 115 to 120 and 122 to 123; and
the VHCDR3 comprises the sequence as set forth in any one of SEQ ID NO: 124 to 129 and 131 to 132, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 124 to 129 and 131 to 132.

15. The antibody derivative according to claim 14, wherein VLCDR1 and VLCDR2 comprise:
| Antibody No. | VL CDR1 | VL CDR2 |
|---|---|---|
| F3 | RASQVIGNDLG (SEQ ID NO: 93) | KLLIYDVSNVQTGVPS (SEQ ID NO: 101) |
| G3 | RASQYISTYLN (SEQ ID NO: 94) | KVLISKTSYLESGVPS (SEQ ID NO: 102) |
| H3 | RASQSISSWLA (SEQ ID NO: 95) | NLLMYAASSLQSGVPS (SEQ ID NO: 103) |
| A4 | QASQDIRNDLN (SEQ ID NO: 96) | KSLIYAASRLQSGVPS (SEQ ID NO: 104) |
| 15F7 | RASQGISSDLG (SEQ ID NO: 97) | KLLIYDVSNVQTGVPS (SEQ ID NO: 101) |
| 15F9 | RASQSISSWLA (SEQ ID NO: 95) | KLLIYDASSLESGVPS (SEQ ID NO: 105) |
| 15A10 | RASQSIGSSLH (SEQ ID NO: 99) | TVLIYAASSLQTGVPS (SEQ ID NO: 107) |
| 15C12 | QASQDISNSLN (SEQ ID NO: 100) | NLLIYATSNLQSGVPS (SEQ ID NO: 108) |
and
wherein VHCDR1, VHCDR2, and VHCDR3 comprise:
| Antibody No. | VH CDR1 | VH CDR2 | VH CDR3 |
|---|---|---|---|
| F3 | FTFSGHWMH (SEQ ID NO: 109) | WVASISDSSSYIYYADSV RGRF (SEQ ID NO: 115) | ARDYEGATRAADY (SEQ ID NO: 124) |
| G3 | FTFSASYMS (SEQ ID NO: 110) | WIGSIYYSGSTYYNPSLK SRV (SEQ ID NO: 116) | ARGAPYYEGAFDI (SEQ ID NO: 125) |
| H3 | FTFSDYWIS (SEQ ID NO: 111) | WIGNVYYSGNAYYNPSL ESRV (SEQ ID NO: 117) | ARNTGFETLDN (SEQ ID NO: 126) |
| A4 | FTFSGHWMH (SEQ ID NO: 109) | WVSGISGSGGSTYYADS VKGRF (SEQ ID NO: 118) | ARELTMATTTDAFDI (SEQ ID NO: 127) |
| 15F7 | FTFSGHWMH (SEQ ID NO: 109) | WVSAISGSGGSTYYADS VKGRF (SEQ ID NO: 119) | AREVTMVRGVTDAFDI (SEQ ID NO: 128) |
| 15F9 | FTFSKFWMH (SEQ ID NO: 112) | WVSSISSSSSYIYYADSV KGRF (SEQ ID NO: 120) | ARDPISTGAFDI (SEQ ID NO: 129) |
| 15A10 | FPFSGHWMH (SEQ ID NO: 114) | WVSGISGTGATSHYADS VKGRF (SEQ ID NO: 122) | AREVITAASTDAFDL (SEQ ID NO: 131) |
| 15C12 | FTFSGHWMH (SEQ ID NO: 109) | WVSAISGSGGSTYYADS VKGRF (SEQ ID NO: 123) | ARELTMASTTDAFDF (SEQ ID NO: 132) |

16. The antibody derivative according to claim 14 or 15, wherein:
the heavy chain variable region comprises the sequence as set forth in any one of SEQ ID NO: 142 to 147 and 149 to 150, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 142 to 147 and 149 to 150; and
the light chain variable region comprises the sequence as set forth in any one of SEQ ID NO: 133 to 138 and 140 to 141, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 133 to 138 and 140 to 141.

17. The antibody derivative according to any one of claims 1 to 9, wherein the antibody or antigen-binding fragment thereof is a Naᵥ1.2 antibody or antigen-binding fragment thereof, the Naᵥ1.2 antibody or antigen-binding fragment thereof comprising a heavy chain variable region and/or a light chain variable region, wherein the light chain variable region comprises VLCDR1, VLCDR2, and VLCDR3, and wherein the heavy chain variable region comprises VHCDR1, VHCDR2, and VHCDR3, wherein:
the VLCDR1 comprises the sequence as set forth in any one of SEQ ID NO: 94, 95, and 151 to 156, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 94, 95, and 151 to 156;
the VLCDR2 comprises the sequence as set forth in any one of SEQ ID NO: 102, 103, and 157 to 163, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 102, 103, and 157 to 163;
the VLCDR3 comprises the amino acid sequence as set forth in any one of SEQ ID NO: 5 to SEQ ID NO: 29, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 5 to SEQ ID NO: 29;
the VHCDR1 comprises the sequence as set forth in any one of SEQ ID NO: 110, 111, and 164 to 170, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 110, 111, and 164 to 170;
the VHCDR2 comprises the sequence as set forth in any one of SEQ ID NO: 116, 117, and 171 to 177, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 116, 117, and 171 to 177; and
the VHCDR3 comprises the sequence as set forth in any one of SEQ ID NO: 125, 126, and 178 to 183, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 125, 126, and 178 to 183.

18. The antibody derivative according to claim 17, wherein VLCDR1 and VLCDR2 comprise:
| Antibody No. | VL CDR1 | VL CDR2 |
|---|---|---|
| H3 | RASQSISSWLA (SEQ ID NO: 95) | NLLMYAASSLQSGVPS (SEQ ID NO: 103) |
| G3 | RASQYISTYLN (SEQ ID NO: 94) | KVLISKTSYLESGVPS (SEQ ID NO: 102) |
| 12B5 | QASQGISHALA (SEQ ID NO: 151) | KLLIYAASSLQSGVPS (SEQ ID NO: 157) |
| 14H3 | RASQTISDWLA (SEQ ID NO: 152) | KLLIYDASSLESGVPS (SEQ ID NO: 158) |
| 14H4 | RASQSISSDLN (SEQ ID NO: 153) | KLLIYRATFLESGVPS (SEQ ID NO: 159) |
| 12D4 | QASQDISNYLN (SEQ ID NO: 154) | KLLIYAASRLQSGVPS (SEQ ID NO: 160) |
| 14B8 | RASQSISSWLA (SEQ ID NO: 95) | NLLMYAASSLQSGVPS (SEQ ID NO: 161) |
| 14C7 | RTSQSVNNHLN (SEQ ID NO: 155) | KLLIYRASSLESGVPS (SEQ ID NO: 162) |
| 14E6 | RASQPISDWLA (SEQ ID NO: 156) | KSLIYDAYNLQSGVPP (SEQ ID NO: 163) |
and
wherein VHCDR1, VHCDR2, and VHCDR3 comprise:
| | | | |
|---|---|---|---|
| G3 | FTFSASYMS (SEQ ID NO: 110) | WIGSIYYSGSTYYNPSLKSRV (SEQ ID NO: 116) | ARGAPYYEGAFDI (SEQ ID NO: 125) |
| H3 | FTFSDYWIS (SEQ ID NO: 111) | WIGNVYYSGNAYYNPSLESRV (SEQ ID NO: 117) | ARNTGFETLDN (SEQ ID NO: 126) |
| 12B5 | FTFSTYVMH (SEQ ID NO: 164) | WLANIDTGGGVTNYADSVKGRF (SEQ ID NO: 171) | ARNSGFETLDY (SEQ ID NO: 178) |
| 12D4 | FTFSDYAMT (SEQ ID NO: 165) | VWVSRIKHDGSSTAYADSVKGRF (SEQ ID NO: 172) | AREGNYCLDY (SEQ ID NO: 179) |
| 14H3 | FTFSSYCMN (SEQ ID NO: 166) | WVSSVTGSAQRTNYADSVKGRF (SEQ ID NO: 173) | ARNTGFETLDN (SEQ ID NO: 126) |
| 14H4 | FSFSKFWMH (SEQ ID NO: 167) | WVAFMAHDESNKNYADSVKGRF (SEQ ID NO: 174) | ARDKVCNTFDI (SEQ ID NO: 180) |
| 14E6 | FTFSNFAMH (SEQ ID NO: 168) | YVSAISGGNGGGTYYADSAKGRF (SEQ ID NO: 175) | AREGNYCLDY (SEQ ID NO: 181) |
| 14C7 | FTFSSFSMS (SEQ | WVANIKEDGSDKYYVDSVTGRF | ARDWGAAAVLHH |
| | ID NO: 169) | (SEQ ID NO: 176) | (SEQ ID NO: 182) |
| 14B8 | FTFSGHWMH (SEQ ID NO: 170) | WVAVISSDGSYKSYADSVKGRF (SEQ ID NO: 177) | ASRTVAGTLDY (SEQ ID NO: 183) |

19. The antibody derivative according to claim 17 or 18, wherein:
the heavy chain variable region comprises the sequence as set forth in any one of SEQ ID NO: 143, 144, and 191 to 197, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 143, 144, and 191 to 197; and
the light chain variable region comprises the sequence as set forth in any one of SEQ ID NO: 134, 135, and 184 to 190, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 134, 135, and 184 to 190.

20. The antibody derivative according to any one of claims 1 to 9, wherein the antibody or antigen-binding fragment thereof is a Naᵥ1.6 antibody or antigen-binding fragment thereof, the Naᵥ1.6 antibody or antigen-binding fragment thereof comprising a heavy chain variable region and/or a light chain variable region, wherein the light chain variable region comprises VLCDR1, VLCDR2, and VLCDR3, and wherein the heavy chain variable region comprises VHCDR1, VHCDR2, and VHCDR3, wherein:
the VLCDR1 comprises the sequence as set forth in any one of SEQ ID NO: 93, 95, 96, and 198 to 203, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 93, 95, 96, and 198 to 203;
the VLCDR2 comprises the sequence as set forth in any one of SEQ ID NO: 101, 103, 104, and 204 to 209, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 101, 103, 104, and 204 to 209;
the VLCDR3 comprises the amino acid sequence as set forth in any one of SEQ ID NO: 5 to SEQ ID NO: 29, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 5 to SEQ ID NO: 29;
the VHCDR1 comprises the sequence as set forth in any one of SEQ ID NO: 109, 111, and 210 to 215, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 109, 111, and 210 to 215;
the VHCDR2 comprises the sequence as set forth in any one of SEQ ID NO: 115, 117, 118, and 216 to 221, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 115, 117, 118, and 216 to 221; and
the VHCDR3 comprises the sequence as set forth in any one of SEQ ID NO: 124, 126, 127, and 222 to 227, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 124, 126, 127, and 222 to 227.

21. The antibody derivative according to claim 20, wherein VLCDR1 and VLCDR2 comprise:
| Antibody No. | VL CDR1 | VL CDR2 |
|---|---|---|
| F3 | RASQVIGNDLG (SEQ ID NO: 93) | KLLIYDVSNVQTGVPS (SEQ ID NO: 101) |
| H3 | RASQSISSWLA (SEQ ID NO: 95) | NLLMYAASSLQSGVPS (SEQ ID NO: 103) |
| A4 | QASQDIRNDLN (SEQ ID NO: 96) | KSLIYAASRLQSGVPS (SEQ ID NO: 104) |
| 15F6 | QASQDISHYLN (SEQ ID NO: 198) | KLLIHRVSSLESGVPS (SEQ ID NO: 204) |
| 15D3 | RASQGISSDLG (SEQ ID NO: 199) | KLLIYDVSNVQTGVPS (SEQ ID NO: 205) |
| 15C1 | RASETIGSWLA (SEQ ID NO: 200) | KLLIYKSTTVEGGVSS (SEQ ID NO: 206) |
| 15G4 | RASQNINRHLN (SEQ ID NO: 201) | KLLIYDVSNVQTGVPS (SEQ ID NO: 207) |
| 15A2 | QASQDIGKFLN (SEQ ID NO: 202) | KLLISATSNLQWGVPS (SEQ ID NO: 208) |
| 5A5 | RASQSISDWLA (SEQ ID NO: 203) | KLLIYAASSLQSGVPS (SEQ ID NO: 209) |
and
wherein VHCDR1, VHCDR2, and VHCDR3 comprise:
| Antibody No. | VH CDR1 | VH CDR2 | VH CDR3 |
|---|---|---|---|
| F3 | FTFSGHWMH (SEQ ID NO: 109) | WVASISDSSSYIYYADSVR GRF (SEQ ID NO: 115) | ARDYEGATRAADY (SEQ ID NO: 124) |
| H3 | FTFSDYWIS (SEQ ID NO: 111) | WIGNVYYSGNAYYNPSLE SRV (SEQ ID NO: 117) | ARNTGFETLDN (SEQ ID NO: 126) |
| A4 | FTFSGHWMH (SEQ ID NO: 109) | WVSGISGSGGSTYYADSV KGRF (SEQ ID NO: 118) | ARELTMATTTDAFDI (SEQ ID NO: 127) |
| 15F6 | FSFSTYAMH (SEQ ID NO: 210) | WVANIDGDGGVTNYAGS VKGRF (SEQ ID NO: 216) | ARDRGWLQFDY (SEQ ID NO: 222) |
| 15D3 | FTFSGHWMH (SEQ ID NO: 211) | WVSAISGSGGSTYYADSV KGRF (SEQ ID NO: 217) | AREVTMVRGVTDAFDI (SEQ ID NO: 223) |
| 15C1 | FTFTSYAMA (SEQ ID NO: 212) | WVSSISNGGSVIYYANSV KGRF (SEQ ID NO: 218) | ARDRGFLQFDY (SEQ ID NO: 224) |
| 15G4 | FTFDEYIMH (SEQ ID NO: 213) | YVSAISSNGGSTYYANSV KGRF (SEQ ID NO: 219) | ARDRGFLQFDY (SEQ ID NO: 225) |
| 15A2 | FSFSNFAMT (SEQ ID NO: 214) | WVSSISNGGSVIYYANSV KGRF (SEQ ID NO: 220) | ARDRGFLQFDY (SEQ ID NO: 226) |
| 5A5 | FPFSGHWMH (SEQ ID NO: 215) | WVSGISWNGGTTGYADSV KGRF (SEQ ID NO: 221) | ARDDYGGDSFDS (SEQ ID NO: 227) |

22. The antibody derivative according to claim 20 or 21, wherein:
the heavy chain variable region comprises the sequence as set forth in any one of SEQ ID NO: 142, 144, 145, and 234 to 239, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 142, 144, 145, and 234 to 239; and
the light chain variable region comprises the sequence as set forth in any one of SEQ ID NO: 133, 135, 136, and 228 to 233, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 133, 135, 136, and 228 to 233.

23. The antibody derivative according to any one of claims 1 to 9 and 14 to 22, wherein the antibody or antigen-binding fragment thereof is a Naᵥ1.2 antibody or antigen-binding fragment thereof, a Naᵥ1.6 antibody or antigen-binding fragment thereof, or a Naᵥ1.7 antibody or antigen-binding fragment thereof, and wherein the small molecule compound is an arylsulfonamide compound, preferably 5-(2-(4-(N-(1,2,4-thiadiazol-5-sulfonamido)-2,5-difluorophenoxy)-5-chlorobenzyl)benzisoxaz ol)-3-amine, having a structure of:

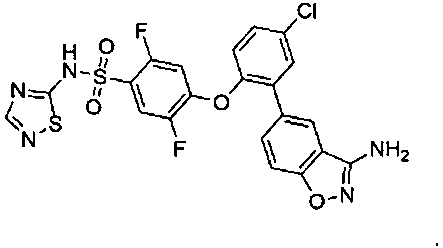

24. The antibody derivative according to any one of claims 1 to 9, wherein the antibody or antigen-binding fragment thereof is an A2aR antibody or antigen-binding fragment thereof, the A2aR antibody or antigen-binding fragment thereof comprising a heavy chain variable region and/or a light chain variable region, wherein the light chain variable region comprises VLCDR1, VLCDR2, and VLCDR3, and wherein the heavy chain variable region comprises VHCDR1, VHCDR2, and VHCDR3, wherein:
the VLCDR1 comprises the sequence as set forth in any one of SEQ ID NO: 30 and SEQ ID NO: 247 to 250, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 30 and SEQ ID NO: 247 to 250;
the VLCDR2 comprises the sequence as set forth in any one of SEQ ID NO: 251 to 255 and SEQ ID NO: 284, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 251 to 255 and SEQ ID NO: 284;
the VLCDR3 comprises the amino acid sequence as set forth in any one of SEQ ID NO: 5 to SEQ ID NO: 29, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 5 to SEQ ID NO: 29;
the VHCDR1 comprises the amino acid sequence as set forth in any one of SEQ ID NO: 256 to SEQ ID NO: 260, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 256 to SEQ ID NO: 260;
the VHCDR2 comprises the amino acid sequence as set forth in any one of SEQ ID NO: 261 to SEQ ID NO: 266, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 261 to SEQ ID NO: 266; and
the VHCDR3 comprises the amino acid sequence as set forth in any one of SEQ ID NO: 267 to SEQ ID NO: 272, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 267 to SEQ ID NO: 272.

25. The antibody derivative according to claim 24, wherein VLCDR1 and VLCDR2 comprise:
| Antibody No. | VL CDR1 | VL CDR2 |
|---|---|---|
| 6G5 | QASQDIRNSLN (SEQ ID NO: 247) | KLLIKYASQSISGVPS (SEQ ID NO: 251) |
| 5C9 | RASQSISRALA (SEQ ID NO: 248) | KLLIYKASSLQSGVPS (SEQ ID NO: 252) |
| 6A6 | QASQSISRYLS (SEQ ID NO: 249) | KLLIYKTSRLESGVPS (SEQ ID NO: 253) |
| 5D10 | QASQDIRNSLN (SEQ ID NO: 247) | KLLIYAASTLQSGVPS (SEQ ID NO: 254) |
| 5F9 | QASQDIRNSLN (SEQ ID NO: 247) | KLLIYAASTLQSGVPS (SEQ ID NO: 254) |
| 6D6 | RASQNIGTSLH (SEQ ID NO: 250) | KLLISRASSLQSGVPS (SEQ ID NO: 255) |
| 6C6 | RASQSISSWLA (SEQ ID NO: 30) | KLLIYKTSNLESGVPS (SEQ ID NO: 284) |
and
wherein VHCDR1, VHCDR2, and VHCDR3 comprise:
| Antibody No. | VH CDR1 | VH CDR2 | VH CDR3 |
|---|---|---|---|
| 6G5 | FTFSGHWMH (SEQ ID NO: 256) | WIGTVSYNGRTYYNPSLK SRV (SEQ ID NO: 261) | ARGASGRTYNNFFDP (SEQ ID NO: 267) |
| 5C9 | FTVGSNSMT (SEQ ID NO: 257) | WVSSISGTGGRTYYADSV KGRF (SEQ ID NO: 262) | ARILPVRGASGQWAR KNWFDP (SEQ ID NO: 268) |
| 6A6 | FTFSGHWMH (SEQ ID NO: 256) | WVSSITGDSNYRYYADSV KGRF (SEQ ID NO: 263) | ARDRSGSLRAFDY (SEQ ID NO: 269) |
| 5D10 | FTFGSFWMS (SEQ ID NO: 258) | WVSSISNGGSVIYYANSV KGRF (SEQ ID NO: 264) | ARGLRRASGPFDY (SEQ ID NO: 270) |
| 5F9 | FRFSSYSMN (SEQ ID NO: 259) | WVSGISGSGGSTYYADPV KGRF (SEQ ID NO: 265) | ARGGRGPTTPFDY (SEQ ID NO: 271) |
| 6D6 | FTFSNHAMH (SEQ ID NO: 260) | WVSAITGNGRSKYYADS VKGRF (SEQ ID NO: 266) | ARDRGWLQFDY (SEQ ID NO: 272) |
| 6C6 | FTFSNHAMH (SEQ ID NO: 260) | WVSAITGNGRSKYYADS VKGRF (SEQ ID NO: 266) | ARDRGWLQFDY (SEQ ID NO: 272) |

26. The antibody derivative according to claim 24 or 25, wherein:
the heavy chain variable region comprises the sequence as set forth in any one of SEQ ID NO: 273 to SEQ ID NO: 278, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 273 to SEQ ID NO: 278; and
the light chain variable region comprises the sequence as set forth in any one of SEQ ID NO: 279 to 283 and SEQ ID NO: 285, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 279 to 283 and SEQ ID NO: 285.

27. The antibody derivative according to any one of claims 1 to 9 and 24 to 26, wherein the antibody or antigen-binding fragment thereof is an A2aR antibody or antigen-binding fragment thereof, and wherein the small molecule compound is ZM241385, having a structure of:

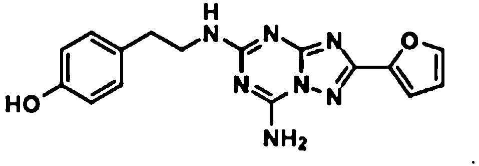

28. A CD73 antibody or antigen-binding fragment thereof, comprising a heavy chain variable region and/or a light chain variable region, wherein the light chain variable region comprises VLCDR1, VLCDR2, and VLCDR3, and wherein the heavy chain variable region comprises VHCDR1, VHCDR2, and VHCDR3, wherein:
the VLCDR1 comprises the amino acid sequence as set forth in any one of SEQ ID NO: 30 to SEQ ID NO: 39, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 30 to SEQ ID NO: 39;
the VLCDR2 comprises the sequence as set forth in any one of SEQ ID NO: 40 to SEQ ID NO: 45, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 40 to SEQ ID NO: 45;
the VLCDR3 comprises the sequence as set forth in any one of SEQ ID NO: 5 to SEQ ID NO: 29, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 5 to SEQ ID NO: 29, wherein * represents a natural amino acid or an unnatural amino acid;
the VHCDR1 comprises the amino acid sequence as set forth in any one of SEQ ID NO: 46 to SEQ ID NO: 55, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 46 to SEQ ID NO: 55;
the VHCDR2 comprises the amino acid sequence as set forth in any one of SEQ ID NO: 56 to SEQ ID NO: 64, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 56 to SEQ ID NO: 64; and
the VHCDR3 comprises the amino acid sequence as set forth in any one of SEQ ID NO: 65 to SEQ ID NO: 72, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 65 to SEQ ID NO: 72.

29. The CD73 antibody or antigen-binding fragment thereof according to claim 28, wherein VLCDR1 and VLCDR2 comprise:
| Antibody No. | VL CDR1 | VL CDR2 |
|---|---|---|
| 10D8 | RASQSISSWLA (SEQ ID NO: 30) | KLLIYDVSNVQTGVPS (SEQ ID NO: 40) |
| 10A7 | RASQGISRWLA (SEQ ID NO: 31) | NLLIYGASTLQSGVPP (SEQ ID NO: 41) |
| 4C10 | RATQAISNYLA (SEQ ID NO: 32) | KLLIYDVSNVQTGVPS (SEQ ID NO: 40) |
| 5E12 | RTSQGITDPLN (SEQ ID NO: 33) | KLLIYKTSSLESGVPS (SEQ ID NO: 42) |
| 5H12 | RASQGLSSWLA (SEQ ID NO: 34) | KLLIYDASNLETGVPS (SEQ ID NO: 43) |
| 6D11 | RASQNIGTYLA (SEQ ID NO: 35) | KLLIYDASNLETGVPS (SEQ ID NO: 43) |
| ABN3-2B | RASQGINNYLA (SEQ ID NO: 36) | KLLIYDASNLETGVPS (SEQ ID NO: 43) |
| ABN3-2G | QASHDISKYLN (SEQ ID NO: 37) | KLLIYDASNLETGVPS (SEQ ID NO: 43) |
| ABN3-2A | RASETIGSWLA (SEQ ID NO: 38) | NLLIYAASTLQSGVSS (SEQ ID NO: 44) |
| 10F8 | QASQDISNYLN (SEQ ID NO: 39) | NLLIYAASTLQSGVPS (SEQ ID NO: 45) |
and
wherein VHCDR1, VHCDR2, and VHCDR3 comprise:
| Antibody No. | VH CDR1 | VH CDR2 | VH CDR3 |
|---|---|---|---|
| ABN3-2A | FTLSTYAMH (SEQ ID NO: 46) | WLARIDWDDDKYYSTSLKTRL (SEQ ID NO: 56) | ARGRDFGEPPYVRAF DI (SEQ ID NO: 65) |
| ABN3-2B | FTFSSYWMH (SEQ ID NO: 47) | WVSAVSGSGDSTYYADSVKGRF (SEQ ID NO: 57) | AREGDWGLDY (SEQ ID NO: 66) |
| ABN3-2G | ITFGSYTMS (SEQ ID NO: 48) | WIGTVHYTGKTFYSPSLKSRV (SEQ ID NO: 58) | ARGRDFGEPPYVRAF DI (SEQ ID NO: 65) |
| 4C10 | FTFSSYWMN (SEQ ID NO: 49) | WVANIKQDGSEKYYVDSVKGRF (SEQ ID NO: 59) | VTGSYRYAY (SEQ ID NO: 67) |
| 5E12 | FTFSNYAMH (SEQ ID NO: 50) | WVSAISGSGGSTYYADSVKGRF (SEQ ID NO: 60) | ARGGGNYYDDY (SEQ ID NO: 68) |
| 5H12 | FTFSHYAMH (SEQ ID NO: 51) | WIGNIYYSGSTYYNPSLKSRV (SEQ ID NO: 61) | AKGHGSGSYFPYYFD Y (SEQ ID NO: 69) |
| 6D11 | FTFSGHWMH (SEQ ID NO: 52) | WIGSIYYSGSTYYNPSLKSRV (SEQ ID NO: 62) | ARDRGWLQFDY (SEQ ID NO: 70) |
| 10A7 | FAFSSYSMS (SEQ ID NO: 53) | WIGSIYYSGSTYYNPSLKSRV (SEQ ID NO: 62) | ARNSGFETLDY (SEQ ID NO: 71) |
| 10D8 | FTFSTYAIH (SEQ ID NO: 54) | WVSSISGSGGGGGTYYADSVKG RF (SEQ ID NO: 63) | ARDRGFLQFDY (SEQ ID NO: 72) |
| 10F8 | FTFSSYGMH (SEQ ID NO: 55) | WVAAISYDGSNELYADSVKGRF (SEQ ID NO: 64) | ARDRGWLQFDY (SEQ ID NO: 70) |

30. The CD73 antibody or antigen-binding fragment thereof according to claim 28 or 29, wherein:
the heavy chain variable region comprises the sequence as set forth in any one of SEQ ID NO: 73 to SEQ ID NO: 82, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 73 to SEQ ID NO: 82; and
the light chain variable region comprises the sequence as set forth in any one of SEQ ID NO: 83 to SEQ ID NO: 92, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 83 to SEQ ID NO: 92.

31. A Naᵥ1.2 antibody or antigen-binding fragment thereof, comprising a heavy chain variable region and/or a light chain variable region, wherein the light chain variable region comprises VLCDR1, VLCDR2, and VLCDR3, and wherein the heavy chain variable region comprises VHCDR1, VHCDR2, and VHCDR3, wherein:
the VLCDR1 comprises the sequence as set forth in any one of SEQ ID NO: 94, 95, and 151 to 156, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 94, 95, and 151 to 156;
the VLCDR2 comprises the sequence as set forth in any one of SEQ ID NO: 102, 103, and 157 to 163, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 102, 103, and 157 to 163;
the VLCDR3 comprises the sequence as set forth in any one of SEQ ID NO: 5 to SEQ ID NO: 29, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 5 to SEQ ID NO: 29, wherein * represents a natural amino acid or an unnatural amino acid;
the VHCDR1 comprises the sequence as set forth in any one of SEQ ID NO: 110, 111, and 164 to 170, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 110, 111, and 164 to 170;
the VHCDR2 comprises the sequence as set forth in any one of SEQ ID NO: 116, 117, and 171 to 177, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 116, 117, and 171 to 177; and
the VHCDR3 comprises the sequence as set forth in any one of SEQ ID NO: 125, 126, and 178 to 183, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 125, 126, and 178 to 183.

32. The Naᵥ1.2 antibody or antigen-binding fragment thereof according to claim 31, wherein VLCDR1 and VLCDR2 comprise:
| Antibody No. | VL CDR1 | VL CDR2 |
|---|---|---|
| H3 | RASQSISSWLA (SEQ ID NO: 95) | NLLMYAASSLQSGVPS (SEQ ID NO: 103) |
| G3 | RASQYISTYLN (SEQ ID NO: 94) | KVLISKTSYLESGVPS (SEQ ID NO: 102) |
| 12B5 | QASQGISHALA (SEQ ID NO: 151) | KLLIYAASSLQSGVPS (SEQ ID NO: 157) |
| 14H3 | RASQTISDWLA (SEQ ID NO: 152) | KLLIYDASSLESGVPS (SEQ ID NO: 158) |
| 14H4 | RASQSISSDLN (SEQ ID NO: 153) | KLLIYRATFLESGVPS (SEQ ID NO: 159) |
| 12D4 | QASQDISNYLN (SEQ ID NO: 154) | KLLIYAASRLQSGVPS (SEQ ID NO: 160) |
| 14B8 | RASQSISSWLA (SEQ ID NO: 95) | NLLMYAASSLQSGVPS (SEQ ID NO: 161) |
| 14C7 | RTSQSVNNHLN (SEQ ID NO: 155) | KLLIYRASSLESGVPS (SEQ ID NO: 162) |
| 14E6 | RASQPISDWLA (SEQ ID NO: 156) | KSLIYDAYNLQSGVPP (SEQ ID NO: 163) |
and
wherein VHCDR1, VHCDR2, and VHCDR3 comprise:
| | | | |
|---|---|---|---|
| G3 | FTFSASYMS (SEQ ID NO: 110) | WIGSIYYSGSTYYNPSLKSRV (SEQ ID NO: 116) | ARGAPYYEGAFDI (SEQ ID NO: 125) |
| H3 | FTFSDYWIS (SEQ ID NO: 111) | WIGNVYYSGNAYYNPSLESRV (SEQ ID NO: 117) | ARNTGFETLDN (SEQ ID NO: 126) |
| 12B5 | FTFSTYVMH (SEQ ID NO: 164) | WLANIDTGGGVTNYADSVKGR F (SEQ ID NO: 171) | ARNSGFETLDY (SEQ ID NO: 178) |
| 12D4 | FTFSDYAMT (SEQ ID NO: 165) | VWVSRIKHDGSSTAYADSVKG RF (SEQ ID NO: 172) | AREGNYCLDY (SEQ ID NO: 179) |
| 14H3 | FTFSSYCMN (SEQ ID NO: 166) | WVSSVTGSAQRTNYADSVKGR F (SEQ ID NO: 173) | ARNTGFETLDN (SEQ ID NO: 126) |
| 14H4 | FSFSKFWMH (SEQ ID NO: 167) | WVAFMAHDESNKNYADSVKG RF (SEQ ID NO: 174) | ARDKVCNTFDI (SEQ ID NO: 180) |
| 14E6 | FTFSNFAMH (SEQ ID NO: 168) | YVSAISGGNGGGTYYADSAKG RF (SEQ ID NO: 175) | AREGNYCLDY (SEQ ID NO: 181) |
| 14C7 | FTFSSFSMS (SEQ ID NO: 169) | WVANIKEDGSDKYYVDSVTGR F (SEQ ID NO: 176) | ARDWGAAAVLHH (SEQ ID NO: 182) |
| 14B8 | FTFSGHWMH (SEQ ID NO: 170) | WVAVISSDGSYKSYADSVKGRF (SEQ ID NO: 177) | ASRTVAGTLDY (SEQ ID NO: 183) |

33. The Naᵥ1.2 antibody or antigen-binding fragment thereof according to claim 31 or 32, wherein:
the heavy chain variable region comprises the sequence as set forth in any one of SEQ ID NO: 143, 144, and 191 to 197, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 143, 144, and 191 to 197; and
the light chain variable region comprises the sequence as set forth in any one of SEQ ID NO: 134, 135, and 184 to 190, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 134, 135, and 184 to 190.

34. A Naᵥ1.6 antibody or antigen-binding fragment thereof, comprising a heavy chain variable region and/or a light chain variable region, wherein the light chain variable region comprises VLCDR1, VLCDR2, and VLCDR3, and wherein the heavy chain variable region comprises VHCDR1, VHCDR2, and VHCDR3, wherein:
the VLCDR1 comprises the sequence as set forth in any one of SEQ ID NO: 93, 95, 96, and 198 to 203, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 93, 95, 96, and 198 to 203;
the VLCDR2 comprises the sequence as set forth in any one of SEQ ID NO: 101, 103, 104, and 204 to 209, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 101, 103, 104, and 204 to 209;
the VLCDR3 comprises the sequence as set forth in any one of SEQ ID NO: 5 to SEQ ID NO: 29, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 5 to SEQ ID NO: 29, wherein * represents a natural amino acid or an unnatural amino acid;
the VHCDR1 comprises the sequence as set forth in any one of SEQ ID NO: 109, 111, and 210 to 215, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 109, 111, and 210 to 215;
the VHCDR2 comprises the sequence as set forth in any one of SEQ ID NO: 115, 117, 118, and 216 to 221, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 115, 117, 118, and 216 to 221; and
the VHCDR3 comprises the sequence as set forth in any one of SEQ ID NO: 124, 126, 127, and 222 to 227, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 124, 126, 127, and 222 to 227.

35. The Naᵥ1.6 antibody or antigen-binding fragment thereof according to claim 34, wherein VLCDR1 and VLCDR2 comprise:
| Antibody No. | VL CDR1 | VL CDR2 |
|---|---|---|
| F3 | RASQVIGNDLG (SEQ ID NO: 93) | KLLIYDVSNVQTGVPS (SEQ ID NO: 101) |
| H3 | RASQSISSWLA (SEQ ID NO: 95) | NLLMYAASSLQSGVPS (SEQ ID NO: 103) |
| A4 | QASQDIRNDLN (SEQ ID NO: 96) | KSLIYAASRLQSGVPS (SEQ ID NO: 104) |
| 15F6 | QASQDISHYLN (SEQ ID NO: 198) | KLLIHRVSSLESGVPS (SEQ ID NO: 204) |
| 15D3 | RASQGISSDLG (SEQ ID NO: 199) | KLLIYDVSNVQTGVPS (SEQ ID NO: 205) |
| 15C1 | RASETIGSWLA (SEQ ID NO: 200) | KLLIYKSTTVEGGVSS (SEQ ID NO: 206) |
| 15G4 | RASQNINRHLN (SEQ ID NO: 201) | KLLIYDVSNVQTGVPS (SEQ ID NO: 207) |
| 15A2 | QASQDIGKFLN (SEQ ID NO: 202) | KLLISATSNLQWGVPS (SEQ ID NO: 208) |
| 5A5 | RASQSISDWLA (SEQ ID NO: 203) | KLLIYAASSLQSGVPS (SEQ ID NO: 209) |
and
wherein VHCDR1, VHCDR2, and VHCDR3 comprise:
| Antibody No. | VH CDR1 | VH CDR2 | VH CDR3 |
|---|---|---|---|
| F3 | FTFSGHWMH (SEQ ID NO: 109) | WVASISDSSSYIYYADSVRGR F (SEQ ID NO: 115) | ARDYEGATRAADY (SEQ ID NO: 124) |
| H3 | FTFSDYWIS (SEQ ID NO: 111) | WIGNVYYSGNAYYNPSLESR V (SEQ ID NO: 117) | ARNTGFETLDN (SEQ ID NO: 126) |
| A4 | FTFSGHWMH (SEQ ID NO: 109) | WVSGISGSGGSTYYADSVKG RF (SEQ ID NO: 118) | ARELTMATTTDAFDI (SEQ ID NO: 127) |
| 15F6 | FSFSTYAMH (SEQ ID NO: 210) | WVANIDGDGGVTNYAGSVK GRF (SEQ ID NO: 216) | ARDRGWLQFDY (SEQ ID NO: 222) |
| 15D3 | FTFSGHWMH (SEQ ID NO: 211) | WVSAISGSGGSTYYADSVKG RF (SEQ ID NO: 217) | AREVTMVRGVTDAFDI (SEQ ID NO: 223) |
| 15C1 | FTFTSYAMA (SEQ ID NO: 212) | WVSSISNGGSVIYYANSVKGR F (SEQ ID NO: 218) | ARDRGFLQFDY (SEQ ID NO: 224) |
| 15G4 | FTFDEYIMH | YVSAISSNGGSTYYANSVKG | ARDRGFLQFDY (SEQ ID |
| | (SEQ ID NO: 213) | RF (SEQ ID NO: 219) | NO: 225) |
| 15A2 | FSFSNFAMT (SEQ ID NO: 214) | WVSSISNGGSVIYYANSVKGR F (SEQ ID NO: 220) | ARDRGFLQFDY (SEQ ID NO: 226) |
| 5A5 | FPFSGHWMH (SEQ ID NO: 215) | WVSGISWNGGTTGYADSVK GRF (SEQ ID NO: 221) | ARDDYGGDSFDS (SEQ ID NO: 227) |

36. The Naᵥ1.6 antibody or antigen-binding fragment thereof according to claim 34 or 35, wherein:
the heavy chain variable region comprises the sequence as set forth in any one of SEQ ID NO: 142, 144, 145, and 234 to 239, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 142, 144, 145, and 234 to 239; and
the light chain variable region comprises the sequence as set forth in any one of SEQ ID NO: 133, 135, 136, and 228 to 233, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 133, 135, 136, and 228 to 233.

37. A Naᵥ1.7 antibody or antigen-binding fragment thereof, comprising a heavy chain variable region and/or a light chain variable region, wherein the light chain variable region comprises VLCDR1, VLCDR2, and VLCDR3, and wherein the heavy chain variable region comprises VHCDR1, VHCDR2, and VHCDR3, wherein:
the VLCDR1 comprises the sequence as set forth in any one of SEQ ID NO: 93 to 97 and 99 to 100, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 93 to 97 and 99 to 100;
the VLCDR2 comprises the sequence as set forth in any one of SEQ ID NO: 101 to 105 and 107 to 108, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 101 to 105 and 107 to 108;
the VLCDR3 comprises the sequence as set forth in any one of SEQ ID NO: 5 to SEQ ID NO: 29, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 5 to SEQ ID NO: 29, wherein * represents a natural amino acid or an unnatural amino acid;
the VHCDR1 comprises the sequence as set forth in any one of SEQ ID NO: 109 to 112 and 114, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 109 to 112 and 114;
the VHCDR2 comprises the sequence as set forth in any one of SEQ ID NO: 115 to 120 and 122 to 123, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 115 to 120 and 122 to 123; and
the VHCDR3 comprises the sequence as set forth in any one of SEQ ID NO: 124 to 129 and 131 to 132, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 124 to 129 and 131 to 132.

38. The Naᵥ1.7 antibody or antigen-binding fragment thereof according to claim 37, wherein VLCDR1 and VLCDR2 comprise:
| Antibody No. | VL CDR1 | VL CDR2 |
|---|---|---|
| F3 | RASQVIGNDLG (SEQ ID NO: 93) | KLLIYDVSNVQTGVPS (SEQ ID NO: 101) |
| G3 | RASQYISTYLN (SEQ ID NO: 94) | KVLISKTSYLESGVPS (SEQ ID NO: 102) |
| H3 | RASQSISSWLA (SEQ ID NO: 95) | NLLMYAASSLQSGVPS (SEQ ID NO: 103) |
| A4 | QASQDIRNDLN (SEQ ID NO: 96) | KSLIYAASRLQSGVPS (SEQ ID NO: 104) |
| 15F7 | RASQGISSDLG (SEQ ID NO: 97) | KLLIYDVSNVQTGVPS (SEQ ID NO: 101) |
| 15F9 | RASQSISSWLA (SEQ ID NO: 95) | KLLIYDASSLESGVPS (SEQ ID NO: 105) |
| 15A10 | RASQSIGSSLH (SEQ ID NO: 99) | TVLIYAASSLQTGVPS (SEQ ID NO: 107) |
| 15C12 | QASQDISNSLN (SEQ ID NO: 100) | NLLIYATSNLQSGVPS (SEQ ID NO: 108) |
and
wherein VHCDR1, VHCDR2, and VHCDR3 comprise:
| Antibody No. | VH CDR1 | VH CDR2 | VH CDR3 |
|---|---|---|---|
| F3 | FTFSGHWMH (SEQ ID NO: 109) | WVASISDSSSYIYYADSV RGRF (SEQ ID NO: 115) | ARDYEGATRAADY (SEQ ID NO: 124) |
| G3 | FTFSASYMS (SEQ ID NO: 110) | WIGSIYYSGSTYYNPSLK SRV (SEQ ID NO: 116) | ARGAPYYEGAFDI (SEQ ID NO: 125) |
| H3 | FTFSDYWIS (SEQ ID NO: 111) | WIGNVYYSGNAYYNPSL ESRV (SEQ ID NO: 117) | ARNTGFETLDN (SEQ ID NO: 126) |
| A4 | FTFSGHWMH (SEQ ID NO: 109) | WVSGISGSGGSTYYADS VKGRF (SEQ ID NO: 118) | ARELTMATTTDAFDI (SEQ ID NO: 127) |
| 15F7 | FTFSGHWMH (SEQ ID NO: 109) | WVSAISGSGGSTYYADS VKGRF (SEQ ID NO: 119) | AREVTMVRGVTDAFDI (SEQ ID NO: 128) |
| 15F9 | FTFSKFWMH (SEQ ID NO: 112) | WVSSISSSSSYIYYADSV KGRF (SEQ ID NO: 120) | ARDPISTGAFDI (SEQ ID NO: 129) |
| 15A10 | FPFSGHWMH (SEQ ID NO: 114) | WVSGISGTGATSHYADS VKGRF (SEQ ID NO: 122) | AREVITAASTDAFDL (SEQ ID NO: 131) |
| 15C12 | FTFSGHWMH (SEQ ID NO: 109) | WVSAISGSGGSTYYADS VKGRF (SEQ ID NO: 123) | ARELTMASTTDAFDF (SEQ ID NO: 132) |

39. The Naᵥ1.7 antibody or antigen-binding fragment thereof according to claim 37 or 38, wherein:
the heavy chain variable region comprises the sequence as set forth in any one of SEQ ID NO: 142 to 147 and 149 to 150, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 142 to 147 and 149 to 150; and
the light chain variable region comprises the sequence as set forth in any one of SEQ ID NO: 133 to 138 and 140 to 141, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 133 to 138 and 140 to 141.

40. An A2aR antibody or antigen-binding fragment thereof, comprising a heavy chain variable region and/or a light chain variable region, wherein the light chain variable region comprises VLCDR1, VLCDR2, and VLCDR3, and wherein the heavy chain variable region comprises VHCDR1, VHCDR2, and VHCDR3, wherein:
the VLCDR1 comprises the sequence as set forth in any one of SEQ ID NO: 30 and SEQ ID NO: 247 to 250, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 30 and SEQ ID NO: 247 to 250;
the VLCDR2 comprises the sequence as set forth in any one of SEQ ID NO: 251 to 255 and SEQ ID NO: 284, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 251 to 255 and SEQ ID NO: 284;
the VLCDR3 comprises the amino acid sequence as set forth in any one of SEQ ID NO: 5 to SEQ ID NO: 29, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 5 to SEQ ID NO: 29;
the VHCDR1 comprises the amino acid sequence as set forth in any one of SEQ ID NO: 256 to SEQ ID NO: 260, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 256 to SEQ ID NO: 260;
the VHCDR2 comprises the amino acid sequence as set forth in any one of SEQ ID NO: 261 to SEQ ID NO: 266, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 261 to SEQ ID NO: 266; and
the VHCDR3 comprises the amino acid sequence as set forth in any one of SEQ ID NO: 267 to SEQ ID NO: 272, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 267 to SEQ ID NO: 272.

41. The A2aR antibody or antigen-binding fragment thereof according to claim 40, wherein VLCDR1 and VLCDR2 comprise:
| Antibody No. | VL CDR1 | VL CDR2 |
|---|---|---|
| 6G5 | QASQDIRNSLN (SEQ ID NO: 247) | KLLIKYASQSISGVPS (SEQ ID NO: 251) |
| 5C9 | RASQSISRALA (SEQ ID NO: 248) | KLLIYKASSLQSGVPS (SEQ ID NO: 252) |
| 6A6 | QASQSISRYLS (SEQ ID NO: 249) | KLLIYKTSRLESGVPS (SEQ ID NO: 253) |
| 5D10 | QASQDIRNSLN (SEQ ID NO: 247) | KLLIYAASTLQSGVPS (SEQ ID NO: 254) |
| 5F9 | QASQDIRNSLN (SEQ ID NO: 247) | KLLIYAASTLQSGVPS (SEQ ID NO: 254) |
| 6D6 | RASQNIGTSLH (SEQ ID NO: 250) | KLLISRASSLQSGVPS (SEQ ID NO: 255) |
| 6C6 | RASQSISSWLA (SEQ ID NO: 30) | KLLIYKTSNLESGVPS (SEQ ID NO: 284) |
and
wherein VHCDR1, VHCDR2, and VHCDR3 comprise:
| Antibody No. | VH CDR1 | VH CDR2 | VH CDR3 |
|---|---|---|---|
| 6G5 | FTFSGHWMH (SEQ ID NO: 256) | WIGTVSYNGRTYYNPSLK SRV (SEQ ID NO: 261) | ARGASGRTYNNFFDP (SEQ ID NO: 267) |
| 5C9 | FTVGSNSMT (SEQ ID NO: 257) | WVSSISGTGGRTYYADSV KGRF (SEQ ID NO: 262) | ARILPVRGASGQWARKN WFDP (SEQ ID NO: 268) |
| 6A6 | FTFSGHWMH (SEQ ID NO: 256) | WVSSITGDSNYRYYADSV KGRF (SEQ ID NO: 263) | ARDRSGSLRAFDY (SEQ ID NO: 269) |
| 5D10 | FTFGSFWMS (SEQ ID NO: 258) | WVSSISNGGSVIYYANSV KGRF (SEQ ID NO: 264) | ARGLRRASGPFDY (SEQ ID NO: 270) |
| 5F9 | FRFSSYSMN (SEQ ID NO: 259) | WVSGISGSGGSTYYADPV KGRF (SEQ ID NO: 265) | ARGGRGPTTPFDY (SEQ ID NO: 271) |
| 6D6 | FTFSNHAMH (SEQ ID NO: 260) | WVSAITGNGRSKYYADS VKGRF (SEQ ID NO: 266) | ARDRGWLQFDY (SEQ ID NO: 272) |
| 6C6 | FTFSNHAMH (SEQ ID NO: 260) | WVSAITGNGRSKYYADS VKGRF (SEQ ID NO: 266) | ARDRGWLQFDY (SEQ ID NO: 272) |

42. The A2aR antibody or antigen-binding fragment thereof according to claim 40 or 41, wherein:
the heavy chain variable region comprises the sequence as set forth in any one of SEQ ID NO: 273 to SEQ ID NO: 278, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 273 to SEQ ID NO: 278; and
the light chain variable region comprises the sequence as set forth in any one of SEQ ID NO: 279 to 283 and SEQ ID NO: 285, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 279 to 283 and SEQ ID NO: 285.

43. The antibody or antigen-binding fragment thereof according to any one of claims 28 to 42, wherein the antibody or the antigen-binding fragment is Fab, Fv, Fd, Fab', Fab'-SH, F(ab')2, a nanobody, a single-domain antibody, or a single-chain antibody.

44. A nucleic acid molecule, encoding the CD73 antibody or antigen-binding fragment thereof according to any one of claims 28 to 30 and 43, the Naᵥ1.2 antibody or antigen-binding fragment thereof according to any one of claims 31 to 33 and 43, the Naᵥ1.6 antibody or antigen-binding fragment thereof according to any one of claims 34 to 36 and 43, the Naᵥ1.7 antibody or antigen-binding fragment thereof according to any one of claims 37 to 39 and 43, or the A2aR antibody or antigen-binding fragment thereof according to any one of claims 40 to 43.

45. A vector, expressing the antibody or antigen-binding fragment thereof according to any one of claims 28 to 43, or comprising the nucleic acid molecule according to claim 44.

46. A host cell or immune cell, comprising the antibody or antigen-binding fragment thereof according to any one of claims 28 to 43, or the nucleic acid molecule according to claim 44, or the vector according to claim 45.

47. Use of a small molecule compound in:
A) the construction of a smacAb library,
preferably, the smacAb library being constructed by conjugating the small molecule compound to the antibody or antigen-binding fragment thereof in an antibody or an antigen-binding fragment library;
B) the screening of smacAb,
preferably, a smacAb library being constructed by conjugating the small molecule compound to the antibody or antigen-binding fragment thereof in an antibody or an antigen-binding fragment library, and screening the smacAb using a target antigen; or
C) the improvement of binding strength or affinity of the antibody to a target antigen,
preferably, the small molecule compound being conjugated to the antibody or antigen-binding fragment thereof, wherein the small molecule compound and the antibody jointly participate in binding to the target antigen.

48. The use according to claim 47, wherein the small molecule compound is selected from PSB12379, 5-(2-(4-(N-(1,2,4-thiadiazol-5-sulfonamido)-2,5-difluorophenoxy)-5-chlorophenyl)benzisoxa zol-3-amine, or ZM241385.

49. The use according to claim 47, wherein the target antigen is a target protein, peptide, lipid, polysaccharide, or polynucleotide;
preferably, the target protein is selected from a membrane protein, a membrane transport protein, a receptor protein, a complex formed by a membrane protein and a membrane protein, a complex formed by a membrane protein and another protein;
more preferably, the membrane protein is a peripheral membrane protein, an integral membrane protein, or a lipid-anchored protein;
more preferably, the membrane transport protein is a carrier protein or a channel protein, wherein the channel protein is preferably a sodium ion channel protein, a calcium ion channel protein, a chloride ion channel protein, a potassium ion channel protein, an Ach receptor channel, or an amino acid receptor channel; and
more preferably, the receptor protein is an intracellular receptor, a cell surface receptor, an ion channel receptor, a G-protein-coupled receptor (GPCR), or an enzyme-linked receptor.

50. The use according to claim 47, wherein the antibody or antigen-binding fragment thereof is selected from the antibody or the antigen-binding fragment according to any one of claims 28 to 43.

51. A smacAb library, comprising a small molecule compound conjugated to an antibody or antigen-binding fragment thereof in an antibody or antigen-binding fragment thereof library.

52. The smacAb library according to claim 51, wherein the small molecule compound is conjugated to a complementarity-determining region (CDR) of the antibody or antigen-binding fragment thereof;
preferably, the small molecule compound is conjugated to heavy chain CDR3 or light chain CDR3 of the antibody or antigen-binding fragment thereof; and
more preferably, the small molecule compound is conjugated to light chain CDR3 of the antibody or antigen-binding fragment thereof.

53. The smacAb library according to claim 52, wherein the light chain CDR3 has a length of 3 to 20 amino acids.

54. The smacAb library according to any one of claims 51 to 53, wherein the small molecule compound is conjugated to one amino acid residue in light chain CDR3 of the antibody or antigen-binding fragment thereof, preferably at position 1, 2, 3, 4, 5, 6, 7, 8, 9, 10, 11, 12, 13, 14, 15, 16, 17, 18, 19, or 20.

55. The smacAb library according to any one of claims 52 to 54, wherein the light chain CDR3 comprises: QQ*YTTPPT (SEQ ID NO: 5), CQQ*YTTPPT (SEQ ID NO: 6), YCQQ*YTTPPT (SEQ ID NO: 7), YYCQQ*YTTPPT (SEQ ID NO: 8), TYYCQQ*YTTPPT (SEQ ID NO: 9), QQ*YTTPPTF (SEQ **ID** NO: 10), QQ*YTTPPTFG (SEQ **ID** NO: 11), QQ*YTTPPTFGQ (SEQ ID NO: 12), QQ*YTTPPTFGQG (SEQ ID NO: 13), CQQ*YTTPPTF (SEQ **ID** NO: 14), CQQ*YTTPPTFG (SEQ **ID** NO: 15), CQQ*YTTPPTFGQ (SEQ ID NO: 16), CQQ*YTTPPTFGQG (SEQ ID NO: 17), YCQQ*YTTPPTF (SEQ **ID** NO: 18), YCQQ*YTTPPTFG (SEQ **ID** NO: 19), YCQQ*YTTPPTFGQ (SEQ ID NO: 20), YCQQ*YTTPPTFGQG (SEQ ID NO: 21), YYCQQ*YTTPPTF (SEQ ID NO: 22), YYCQQ*YTTPPTFG (SEQ ID NO: 23), YYCQQ*YTTPPTFGQ (SEQ ID NO: 24), YYCQQ*YTTPPTFGQG (SEQ **ID** NO: 25), TYYCQQ*YTTPPTF (SEQ ID NO: 26), TYYCQQ*YTTPPTFG (SEQ ID NO: 27), TYYCQQ*YTTPPTFGQ (SEQ ID NO: 28), or TYYCQQ*YTTPPTFGQG (SEQ ID NO: 29); or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 5 to SEQ ID NO: 29, wherein * represents a natural amino acid or an unnatural amino acid and denotes a site where the small molecule compound is conjugated to the antibody or antigen-binding fragment thereof.

56. The smacAb library according to any one of claims 51 to 55, wherein the antibody or antigen-binding fragment thereof is site-specifically conjugated to the small molecule compound, preferably by means of a Michael addition reaction or a Huisgen reaction.

57. The smacAb library according to any one of claims 51 to 56, wherein the antibody or antigen-binding fragment thereof is conjugated to the small molecule compound via a linker group;
preferably, the linker group is a linker peptide, a PEG moiety, 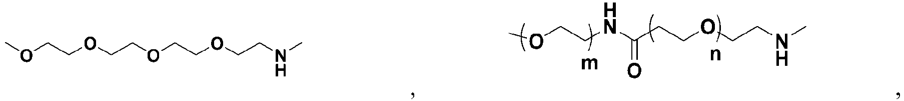 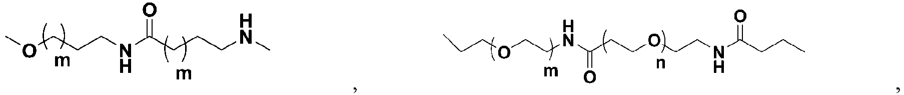 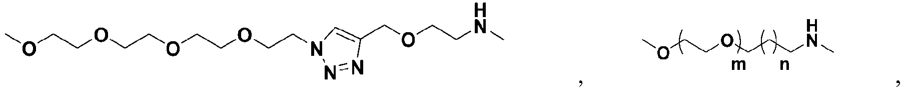 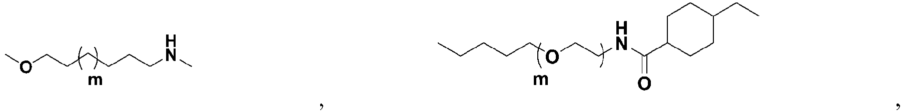 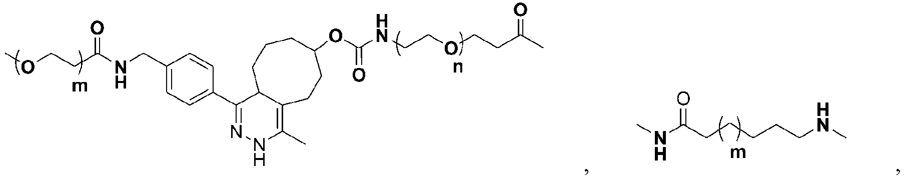 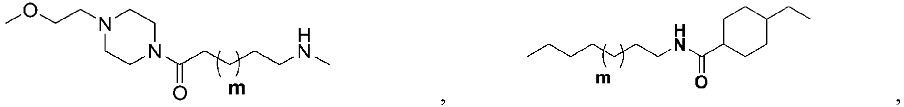 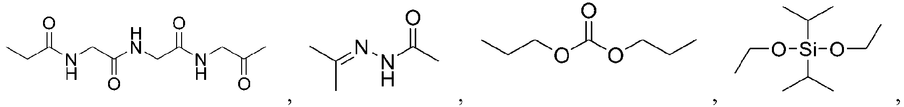 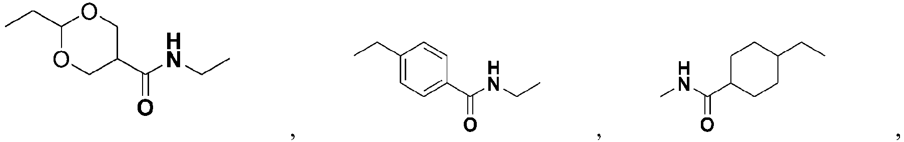 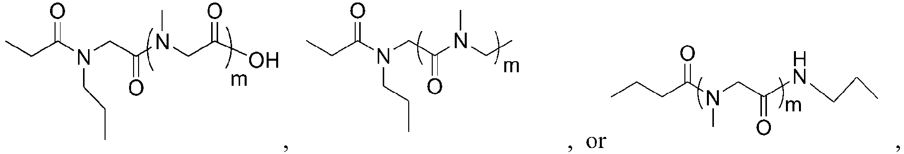 where m and n are each independently an integer selected from 1 to 20;
preferably, the linker peptide comprises a sequence as set forth in any one of SEQ ID NO: 98, 106, 113, 121, 130, 139, 148, and 240 to 246, or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 98, 106, 113, 121, 130, 139, 148, and 240 to 246.

58. The smacAb library according to any one of claims 51 to 57, wherein the small molecule compound is selected from PSB12379, 5-(2-(4-(N-(1,2,4-thiadiazol-5-sulfonamido)-2,5-difluorophenoxy)-5-chlorophenyl)benzisoxa zol-3-amine, or ZM241385.

59. The smacAb library according to any one of claims 51 to 58, wherein the antibody or antigen-binding fragment thereof is selected from the antibody or the antigen-binding fragment according to any one of claims 28 to 43.

60. A method for constructing a smacAb library, the method comprising conjugating a displayed antibody or antigen-binding fragment thereof with a small molecule compound.

61. The method according to claim 60, comprising:
A) acquiring CDR library fragments and assembling the CDR library fragments at corresponding positions of a framework antibody to obtain library phagemids;
B) introducing the library phagemids into *Escherichia coli* and displaying, under the action of helper phage, the antibody or antigen-binding fragment thereof on phage surface; and
C) site-specifically conjugating the displayed antibody or antigen-binding fragment thereof with a small molecule compound to obtain the smacAb library.

62. The method according to claim 61, wherein the framework antibody is from Fab, Fv, Fd, Fab', Fab'-SH, F(ab')2, a nanobody, a single-domain antibody, a single-chain antibody, IgG, IgA, IgM, IgD, or IgE.

63. The method according to claim 60 or 61, wherein the antibody or antigen-binding fragment thereof is Fab, Fv, Fd, Fab', Fab'-SH, F(ab')2, a nanobody, a single-domain antibody, or a single-chain antibody.

64. The method according to any one of claims 60 to 63, wherein the antibody or antigen-binding fragment thereof comprises light chain CDR3, wherein the light chain CDR3 comprises: QQ*YTTPPT (SEQ ID NO: 5), CQQ*YTTPPT (SEQ ID NO: 6), YCQQ*YTTPPT (SEQ ID NO: 7), YYCQQ*YTTPPT (SEQ ID NO: 8), TYYCQQ*YTTPPT (SEQ ID NO: 9), QQ*YTTPPTF (SEQ ID NO: 10), QQ*YTTPPTFG (SEQ ID NO: 11), QQ*YTTPPTFGQ (SEQ ID NO: 12), QQ*YTTPPTFGQG (SEQ ID NO: 13), CQQ*YTTPPTF (SEQ ID NO: 14), CQQ*YTTPPTFG (SEQ ID NO: 15), CQQ*YTTPPTFGQ (SEQ ID NO: 16), CQQ*YTTPPTFGQG (SEQ ID NO: 17), YCQQ*YTTPPTF (SEQ ID NO: 18), YCQQ*YTTPPTFG (SEQ ID NO: 19), YCQQ*YTTPPTFGQ (SEQ ID NO: 20), YCQQ*YTTPPTFGQG (SEQ ID NO: 21), YYCQQ*YTTPPTF (SEQ ID NO: 22), YYCQQ*YTTPPTFG (SEQ ID NO: 23), YYCQQ*YTTPPTFGQ (SEQ ID NO: 24), YYCQQ*YTTPPTFGQG (SEQ ID NO: 25), TYYCQQ*YTTPPTF (SEQ ID NO: 26), TYYCQQ*YTTPPTFG (SEQ ID NO: 27), TYYCQQ*YTTPPTFGQ (SEQ ID NO: 28), or TYYCQQ*YTTPPTFGQG (SEQ ID NO: 29); or a sequence having at least 70%, 75%, 80%, 85%, 90%, 91%, 92%, 93%, 94%, 95%, 96%, 97%, 98%, 99%, 99.5%, or 99.9% identity to any one of SEQ ID NO: 5 to SEQ ID NO: 29, wherein * represents a natural amino acid or an unnatural amino acid and denotes a site where the small molecule compound is conjugated to the antibody or antigen-binding fragment thereof.

65. The method according to any one of claims 60 to 64, wherein the small molecule compound is selected from PSB12379, 5-(2-(4-(N-(1,2,4-thiadiazol-5-sulfonamido)-2,5-difluorophenoxy)-5-chlorophenyl)benzisoxa zol-3-amine, or ZM241385.

66. Use of a smacAb library in the screening an antibody or smacAb, the smacAb library being constructed with the method according to any one of claims 60 to 65 or being the smacAb library according to any one of claims 51 to 59.

67. A method for screening an antibody or antigen-binding fragment thereof or smacAb, the method comprising:
adding a target antigen to a smacAb library constructed by the method according to any one of claims 60 to 65 or to the smacAb library according to any one of claims 51 to 59, and
collecting the antibody or antigen-binding fragment thereof or smacAb that binds to the target antigen.

68. Use of the antibody derivative according to any one of claims 1 to 27, the antibody or antigen-binding fragment thereof according to any one of claims 28 to 43, the nucleic acid molecule according to claim 44, the vector according to claim 45, the host cell or immune cell according to claim 46, or the smacAb library according to any one of claims 51 to 59, in the preparation of a product for diagnosing and/or treating a disease.

69. The use according to claim 68, wherein the disease is selected from a tumor, inflammation, or an infectious disease.

70. A medicament or a diagnostic kit, comprising the antibody derivative according to any one of claims 1 to 27, the antibody or antigen-binding fragment thereof according to any one of claims 28 to 43, the nucleic acid molecule according to claim 44, the vector according to claim 45, or the host cell or immune cell according to claim 46.

71. A method for treating a disease, the method comprising:
administering the antibody derivative according to any one of claims 1 to 27, the antibody or antigen-binding fragment thereof according to any one of claims 28 to 43, the nucleic acid molecule according to claim 44, the vector according to claim 45, or the host cell or immune cell according to claim 46.

72. The method according to claim 71, wherein the disease is selected from a tumor, an infectious disease, or inflammation.
